(19) Europäisches Patentamt
European Patent Office
Office européen des brevets

(11) EP 4 275 606 B1

(12) **EUROPEAN PATENT SPECIFICATION**

(45) Date of publication and mention of the grant of the patent:
**03.06.2026 Bulletin 2026/23**

(21) Application number: **21917443.0**

(22) Date of filing: **06.01.2021**

(51) International Patent Classification (IPC):
***A61B 5/346*** $^{(2021.01)}$ ***A61B 5/35*** $^{(2021.01)}$

(52) Cooperative Patent Classification (CPC):
**A61B 5/35; A61B 5/7275**

(86) International application number:
**PCT/JP2021/000209**

(87) International publication number:
**WO 2022/149215 (14.07.2022 Gazette 2022/28)**

(54) **SIGNAL ANALYSIS DEVICE, SIGNAL ANALYSIS METHOD, AND PROGRAM**
SIGNALANALYSEVORRICHTUNG, SIGNALANALYSEVERFAHREN UND PROGRAMM
DISPOSITIF D’ANALYSE DE SIGNAL, PROCÉDÉ D’ANALYSE DE SIGNAL ET PROGRAMME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**15.11.2023 Bulletin 2023/46**

(73) Proprietor: **NTT, Inc.**
**Tokyo 100-8116 (JP)**

(72) Inventor: **TSUKADA, Shingo**
**Musashino-shi, Tokyo 180-8585 (JP)**

(74) Representative: **Santarelli**
**Tour Trinity**
**1 bis Place de la Défense**
**92400 Courbevoie (FR)**

(56) References cited:
**JP-A- 2018 504 199**

- **KHEIRATI ROONIZI EBADOLLAH: "A New Algorithm for Fitting a Gaussian Function Riding on the Polynomial Background", IEEE SIGNAL PROCESSING LETTERS, IEEE, USA, vol. 20, no. 11, 1 November 2013 (2013-11-01), pages 1062 - 1065, XP011526432, ISSN: 1070-9908, [retrieved on 20130911], DOI: 10.1109/LSP.2013.2280577**
- **MAHSA AKHBARI ET AL: "ECG denoising and fiducial point extraction using an extended Kalman filtering framework with linear and nonlinear phase observations", PHYSIOLOGICAL MEASUREMENT, INSTITUTE OF PHYSICS PUBLISHING, BRISTOL, GB, vol. 37, no. 2, 15 January 2016 (2016-01-15), pages 203 - 226, XP020297441, ISSN: 0967-3334, [retrieved on 20160115], DOI: 10.1088/0967-3334/37/2/203**
- **MOHAMMAD SAAD BILLAH ET AL: "A novel method to model ECG beats using Gaussian functions", BIOMEDICAL ENGINEERING AND INFORMATICS (BMEI), 2011 4TH INTERNATIONAL CONFERENCE ON, IEEE, 15 October 2011 (2011-10-15), pages 612 - 616, XP032071565, ISBN: 978-1-4244-9351-7, DOI: 10.1109/BMEI.2011.6098409**
- **TANAKA HIROSHI: "On the Inverse Solution of Electrocardiology", IYO DENSHI TO SEITAI KOGAKU. JAPANESE JOURNAL OF MEDICAL ELECTRONICS AND BIOLOGICAL ENGINEERING, vol. 23, no. 3, 30 June 1985 (1985-06-30), JP , pages 147 - 158, XP009538878, ISSN: 0021-3292, DOI: 10.11239/jsmbe1963.23.147**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- TANAKA, YOSHIFUMI: "The ECG Recognition and Treatment of Antiarrhythmic Drugs", THE JOURNAL OF JAPAN SOCIETY FOR CLINICAL ANESTHESIA, vol. 30, no. 4, 28 October 2010 (2010-10-28), JP , pages 506 - 516, XP009538877, ISSN: 0285-4945, DOI: 10.2199/jjsca.30.506

## Description

### Technical Field

The present invention relates to a signal analyzing apparatus, a signal analyzing method and a program.

### Background Art

An electrocardiogram is useful information for allowing heart conditions to be recognized, and for example, it is possible to determine whether or not a subject has a high risk of heart failure by using an electrocardiogram (Non Patent Literature 1).

### Citation List

### Non Patent Literature

Non Patent Literature 1: Hiroshi Tanaka, "Methodology in Electrocardiographic Inverse Problems", Medical Electronics and Biotechnology, 1985, Vol. 23, No. 3p.147-158

Non Patent Literature 2: Wataru Shimizu, "The role of M cells in the origin of T waves", JPN. J. ELECTROCARDIOLOGY, Vol.21, No.2, 2001, p.101 to 108

Non Patent Literature 3: Yoshifumi Tanaka, Shinji Nodo, Yasuo Yamazaki, Arisa Nakasone, "Program for constructing body surface electrocardiogram from cardiac action potential waveform", Anaesthesia/intensive care and technology 2011 (1): p. 91 to 95, 2011.

Non Patent Literature 4: KHEIRATI ROONIZI EBADOLLAH: "A New Algorithm for Fitting a Gaussian Function Riding on the Polynomial Background", IEEE SIGNAL PROCESSING LETTERS, IEEE, USA, vol. 20, no. 11, 1 November 2013 (2013-11-01), pages 1062-1065, ISSN: 1070-9908, DOI: 10.1109/LSP.2013.2280577.

Non Patent Literature 5: MAHSA AKHBARI ET AL: "ECG denoising and fiducial point extraction using an extended Kalman filtering framework with linear and nonlinear phase observations", PHYSIOLOGICAL MEASUREMENT, INSTITUTE OF PHYSICS PUBLISHING, BRISTOL, GB, vol. 37, no. 2, 15 January 2016 (2016-01-15), pages 203-226, ISSN: 0967-3334, DOi: 10.1088/0967-3334/37/2/203.


### Summary of Invention

### Technical Problem

However, the information obtained from the waveforms of an electrocardiogram may not always be sufficient for allowing the state of the heart to be recognized. For example, there may be cases in which the manner of development of heart-related diseases differs even though the waveforms of electrocardiograms are similar. In this manner, it may be difficult to acquire other information depending on the disease since the information obtained from the waveforms of an electrocardiogram may not always be sufficient to allow the state of the heart to be recognized. In a case of preventing heart failure, for example, it is possible to prevent development of the disease by observing a heart state from a waveform of the electrocardiogram in everyday life. Although acquisition of other information using other techniques such as blood collection is conceivable in order to further enhance accuracy of preventing onset, doing this in everyday life is not practical. Therefore, depending on the disease, it may be necessary to ascertain the state of the heart substantially based only on the waveform of an electrocardiogram.

Also, such circumstances are not limited to a case where a state of a heart is recognized on the basis of a waveform of an electrocardiogram. Such circumstances are also the same in a case where a state of the heart is recognized on the basis only of time-series biological information regarding pulsation of the heart. Note that the time-series biological information regarding pulsation of the heart is, for example, a waveform indicating a change in cardiac potential, a waveform indicating change in image of the heart, a waveform indicating change in pressure of the heart, a waveform indicating a change in amount of blood flow, and a waveform indicating a change in heart sound. Note that a waveform of an electrocardiogram is also an example of the time-series biological information regarding pulsation of the heart. Note that the image of the heart is, for example, a magnetic resonance image (MRI) of the heart or a computed tomography (CT) image of the heart.

In view of the above circumstances, an object of the present invention is to provide a technique for increasing information obtained from time-series biological information regarding pulsation of a heart.

Solution to Problem

An aspect of the present invention is a signal analysis device according to claim 1 or to claim 4.

Advantageous Effects of Invention

According to the present invention, it is possible to provide a technique for increasing information obtained from time-series biological information regarding pulsation of a heart.

Brief Description of Drawings

Fig. 1 is a diagram illustrating a hardware configuration of a signal analysis device 1 according to an embodiment.
Fig. 2 is a diagram illustrating an example of a result of fitting a waveform of an electrocardiogram of a target heart with four channel cumulative distribution functions according to the embodiment.
Fig. 3 is an explanatory diagram for explaining that it is possible to fit a difference in two channel cumulative distribution functions to a waveform that is substantially the same as a rising waveform of an R wave according to the embodiment.
Fig. 4 is a diagram illustrating an example of a functional configuration of a control unit 11 according to the embodiment.
Fig. 5 is a flowchart illustrating an example of a flow of processing that a signal analysis device 1 executes according to the embodiment.
Fig. 6 is a first diagram illustrating an example of an analysis result of the signal analysis device 1 according to the embodiment.
Fig. 7 is a second diagram illustrating an example of an analysis result of the signal analysis device 1 according to the embodiment.
Fig. 8 is a third diagram illustrating an example of an analysis result of the signal analysis device 1 according to the embodiment.
Fig. 9 is a fourth diagram illustrating an example of an analysis result of the signal analysis device 1 according to the embodiment.
Fig. 10 is a first explanatory diagram of an example in which the signal analysis device 1 analyzes an electrocardiogram of premature ventricular contraction according to the embodiment.
Fig. 11 is a second explanatory diagram of an example in which the signal analysis device 1 analyzes an electrocardiogram of premature ventricular contraction according to the embodiment.
Fig. 12 is a third explanatory diagram of an example in which the signal analysis device 1 analyzes an electrocardiogram of premature ventricular contraction according to the embodiment.
Fig. 13 is a first explanatory diagram in which the signal analysis device 1 analyzes an electrocardiogram of a target heart in a depolarization period of the Brugada syndrome type 1 according to the embodiment.
Fig. 14 is a second explanatory diagram in which the signal analysis device 1 analyzes an electrocardiogram of a target heart in a depolarization period of the Brugada syndrome type 1 according to the embodiment.
Fig. 15 is a third explanatory diagram in which the signal analysis device 1 analyzes an electrocardiogram of a target heart in a depolarization period of the Brugada syndrome type 1 according to the embodiment.
Fig. 16 is a diagram illustrating a first example in which the signal analysis device 1 analyzes an electrocardiogram according to the embodiment.
Fig. 17 is a diagram illustrating a second example in which the signal analysis device 1 analyzes an electrocardiogram according to the embodiment.
Fig. 18 is a diagram illustrating a third example in which the signal analysis device 1 analyzes an electrocardiogram according to the embodiment.
Fig. 19 is a diagram illustrating a fourth example in which the signal analysis device 1 analyzes an electrocardiogram according to the embodiment.
Fig. 20 is a diagram illustrating a fifth example in which the signal analysis device 1 analyzes an electrocardiogram according to the embodiment.
Fig. 21 is a diagram illustrating a sixth example in which the signal analysis device 1 analyzes an electrocardiogram according to the embodiment.
Fig. 22 is a diagram illustrating a seventh example in which the signal analysis device 1 analyzes an electrocardiogram according to the embodiment.
Fig. 23 is a diagram illustrating an eighth example in which the signal analysis device 1 analyzes an electrocardiogram according to the embodiment.

Fig. 24 is a diagram illustrating a ninth example in which the signal analysis device 1 analyzes an electrocardiogram according to the embodiment.

Fig. 25 is a diagram illustrating a tenth example in which the signal analysis device 1 analyzes an electrocardiogram according to the embodiment.

Fig. 26 is a diagram illustrating an eleventh example in which the signal analysis device 1 analyzes an electrocardiogram according to the embodiment.

Fig. 27 is a diagram illustrating a twelfth example in which the signal analysis device 1 analyzes an electrocardiogram according to the embodiment.

Fig. 28 is a diagram illustrating a thirteenth example in which the signal analysis device 1 analyzes an electrocardiogram according to the embodiment.

Fig. 29 is a diagram illustrating a fourteenth example in which the signal analysis device 1 analyzes an electrocardiogram according to the embodiment.

Fig. 30 is a diagram illustrating a fifteenth example in which the signal analysis device 1 analyzes an electrocardiogram according to the embodiment.

Fig. 31 is a diagram illustrating a sixteenth example in which the signal analysis device 1 analyzes an electrocardiogram according to the embodiment.

Fig. 32 is a diagram illustrating a seventeenth example in which the signal analysis device 1 analyzes an electrocardiogram according to the embodiment.

Fig. 33 is a diagram illustrating an eighteenth example in which the signal analysis device 1 analyzes an electrocardiogram according to the embodiment.

Fig. 34 is a diagram illustrating a nineteenth example in which the signal analysis device 1 analyzes an electrocardiogram according to the embodiment.

Fig. 35 is a diagram illustrating a twentieth example in which the signal analysis device 1 analyzes an electrocardiogram according to the embodiment.

Fig. 36 is a diagram illustrating a twenty first example in which the signal analysis device 1 analyzes an electrocardiogram according to the embodiment.

Fig. 37 is a diagram illustrating a twenty second example in which the signal analysis device 1 analyzes an electrocardiogram according to the embodiment.

Fig. 38 is a diagram illustrating a twenty third example in which the signal analysis device 1 analyzes an electrocardiogram according to the embodiment.

Fig. 39 is a diagram illustrating a twenty fourth example in which the signal analysis device 1 analyzes an electrocardiogram according to the embodiment.

Fig. 40 is a diagram illustrating a twenty fifth example in which the signal analysis device 1 analyzes an electrocardiogram according to the embodiment.

Fig. 41 is a diagram illustrating a twenty sixth example in which the signal analysis device 1 analyzes an electrocardiogram according to the embodiment.

Fig. 42 is a diagram illustrating a twenty seventh example in which the signal analysis device 1 analyzes an electrocardiogram according to the embodiment.

Fig. 43 is a diagram illustrating a twenty eighth example in which the signal analysis device 1 analyzes an electrocardiogram according to the embodiment.

Fig. 44 is a diagram illustrating a twenty ninth example in which the signal analysis device 1 analyzes an electrocardiogram according to the embodiment.

Fig. 45 is a diagram illustrating a thirtieth example in which the signal analysis device 1 analyzes an electrocardiogram according to the embodiment.

Fig. 46 is a diagram illustrating a thirty first example in which the signal analysis device 1 analyzes an electrocardiogram according to the embodiment.

Fig. 47 is a diagram illustrating a thirty second example in which the signal analysis device 1 analyzes an electrocardiogram according to the embodiment.

Fig. 48 is a diagram illustrating a thirty third example in which the signal analysis device 1 analyzes an electrocardiogram according to the embodiment.

Fig. 49 is a diagram illustrating a thirty fourth example in which the signal analysis device 1 analyzes an electrocardiogram according to the embodiment.

Fig. 50 is a diagram illustrating a thirty fifth example in which the signal analysis device 1 analyzes an electrocardiogram according to the embodiment.

Fig. 51 is a diagram illustrating a thirty sixth example in which the signal analysis device 1 analyzes an electrocardiogram according to the embodiment.

Fig. 52 is a diagram illustrating a thirty seventh example in which the signal analysis device 1 analyzes an electrocardiogram according to the embodiment.

Fig. 53 is a diagram illustrating a thirty eighth example in which the signal analysis device 1 analyzes an electrocardiogram according to the embodiment.

Fig. 54 is a diagram illustrating a thirty ninth example in which the signal analysis device 1 analyzes an electrocardiogram according to the embodiment.

Description of Embodiments

(First Embodiment)

Fig. 1 is a diagram illustrating an example of the hardware configuration of a signal analysis device 1 according to an embodiment. Hereinafter, the signal analysis device 1 will be described by exemplifying a case where analysis is performed at least on the basis of a waveform of an electrocardiogram for simplicity of explanation. However, the signal analysis device 1 can perform similar analysis on the basis not only of the electrocardiogram but also of time-series biological information regarding pulsation of a heart. Note that the time-series biological information regarding pulsation of the heart is, for example, a waveform indicating a change in cardiac potential, a waveform indicating a change in image of the heart, a waveform indicating a change in pressure of the heart, a waveform indicating a change in amount of blood flow, and a waveform indicating a change in heart sound. Therefore, the signal analysis device 1 may use a waveform indicating a cardiac cycle instead of the waveform of the electrocardiogram. The signal analysis device 1 may use a waveform indicating a change in image of the heart instead of the waveform of the electrocardiogram.

The signal analysis device 1 may use a waveform indicating a change in pressure of the heart instead of the waveform of the electrocardiogram. The signal analysis device 1 may use a waveform indicating a change in amount of blood flow instead of the waveform of the electrocardiogram. The signal analysis device 1 may use a waveform indicating a change in heart sound instead of the waveform of the electrocardiogram. Note that the electrocardiogram is also an example of the time-series biological information regarding pulsation of the heart. Note that the time-series biological information regarding pulsation of the heart may be time-series biological information regarding cyclic pulsation of the heart. Note that the image of the heart is, for example, a magnetic resonance image (MRI) of the heart or a computed tomography (CT) image of the heart.

The signal analysis device 1 acquires a waveform of an electrocardiogram of a heart that is an analysis target (hereinafter, referred to as a "target heart"). The signal analysis device 1 acquires information (hereinafter, referred to as "ion channel activity information") indicating a state of an activity of an ion channel of a myocardium in an outer layer and a myocardium in an inner layer of myocardia of the target heart on the basis of the acquired waveform of the electrocardiogram.

Here, a relationship between the ion channel and the waveform of the electrocardiogram will be described. In the medical field, a model that explains a relationship between motion of myocardium and an electrocardiogram called a cardiac electromotive force dipole model (Reference Literature 1) is known. According to the electromotive force dipole model, the myocardia are modeled by two layers, namely an outer layer (hereinafter, referred to as "outer myocardial layer") and an inner layer (hereinafter referred to as "inner myocardial layer").

Reference Literature 1: Yoshifumi Tanaka, "Electrocardiogram waveform understood from the constitution, Reading action potentials of myocardium", Gakken Medical Shujunsha Co., Ltd. (2012)

In the electromotive force dipole model, the outer myocardial layer and the inner myocardial layer are modeled as different electromotive force generation sources. According to the electromotive force dipole model, a composite wave of the epicardial myocardial activity potential and the endocardial myocardial activity potential substantially coincides with a temporal change in potential of a body surface observed on the body surface. A graph representing the temporal change in potential on the body surface is a waveform of the electrocardiogram. The epicardial myocardial activity potential is a result obtained by directly measuring a change in electromotive force generated through pulsation of the outer myocardial layer by inserting a catheter. The endocardial myocardial activity potential is a result obtained by directly measuring a change in electromotive force generated through pulsation of the inner myocardial layer by inserting a catheter. An overview description of the electromotive force dipole model has been provided hitherto.

Incidentally, the outer myocardial layer in the electromotive force dipole model is a group of cells. Therefore, timings of pulsation of the cells in the outer myocardial layer in one-time pulsation of the outer myocardial layer are not always the same for all the cells, and there is a probability that the pulsation timings will have a distribution. The same applies to the inner myocardial layer. Therefore, timings of pulsation of the cells in the inner myocardial layer in one-time pulsation of the inner myocardial layer are not always the same for all the cells, and there is a probability that the pulsation timings have distribution. However, such a probability that cell pulsation timings may have distribution is not assumed in the electromotive force dipole model.

Also, for each cell in one-time myocardial pulsation, a plurality of kinds of ion channels carry out activities at timings corresponding to a timing in accordance with a timing of pulsation of each cell. The timings of activities of the ion channels are timings in accordance with the types of the ion channels and are not necessarily the same. For example, activity timings are different for a sodium channel and a potassium channel.
Therefore, if there is distribution of timings of pulsation of the cells, timings of activities of the ion channels in one-type myocardial pulsation also have distribution in accordance with the distribution of the timings of pulsation of the cells.

Also, since the timings of the activities of the ion channels are timings in accordance with the types of the ion channels, channel activity timing distribution appears at different timings for ion channels that carry out activities at different timings if the timings of pulsation of cells have distribution. The channel activity timing distribution is distribution of timings of ion channel activities.

The channel activity timing distribution is, for example, a Gaussian distribution. The channel activity timing distribution may satisfy a condition that the cumulative distribution function of the probability density function representing the channel activity timing distribution is a function representing a sigmoid curve. The function representing a sigmoid curve may be, for example, a sigmoid function, a cumulative normal distribution function, a Gompertz function, or a Gudermann function. The channel activity timing distribution may be, for example, a logistic distribution.

The signal analysis device 1 acquires ion channel activity information on the basis of the acquired waveform of the electrocardiogram of the target heart using information (hereinafter, referred to as "distribution candidate information") indicating channel activity timing distribution candidates. The distribution candidate information is expressed by a cumulative distribution function (hereinafter, referred to as a "channel cumulative distribution function") using a function representing candidates for channel activity timing distribution as a probability density function, for example. The function representing candidates for channel activity timing distribution is, specifically, a function in which values representing the shape of the channel activity timing distribution such as variance and an average value of the channel activity timing distribution are expressed by parameters. Hereinafter, a parameter representing the shape of the channel activity timing distribution will be referred to as a shape parameter. Therefore, the cumulative distribution function representing distribution candidate information is a functional having one or a plurality of shape parameters.

Hereinafter, processing for acquiring the ion channel activity information on the basis of the acquired waveform of the electrocardiogram of the target heart using the distribution candidate information will be referred to as ion channel activity information acquisition processing. The acquired ion channel activity information is an example of a result obtained by the signal analysis device 1 analyzing the waveform of the electrocardiogram of the target heart.

Hereinafter, the signal analysis device 1 will be described by exemplifying a case where distribution candidate information is a channel cumulative distribution function for simplicity of explanation. The ion channel activity information acquisition processing in this case is specifically processing of determining the values of shape parameters that the channel cumulative distribution function has by performing fitting to the waveform of the electrocardiogram of the target heart using one or a plurality of channel cumulative distribution functions.

The channel cumulative distribution function is represented by Expression (1) or Expression (2) below, for example.

[Math. 1]

$$\frac{1}{\sqrt{2\pi\sigma^2}} \exp\left(-\frac{(x-\mu)^2}{2\sigma^2}\right) \quad \cdots (1)$$

[Math. 2]

$$\frac{1}{2}\left(1 + \mathrm{erf}\,\frac{x-\mu}{\sqrt{2\sigma^2}}\right) \quad \cdots (2)$$

Expression (1) is normal distribution (Gaussian distribution). Expression (2) is a cumulative distribution function of Expression (1). In Expression (2), "erf" is an error function (sigmoid function).

If averages are expressed as $\mu_a$ and $\mu_b$ and variances are expressed as $\sigma^2{}_a$ and $\sigma^2{}_b$ for two cumulative distribution functions, at least either averages or variance are different, a difference between the two cumulative distribution functions is expressed by Expression (3) below.

[Math. 3]

$$\frac{1}{2}\left(1 + \mathrm{erf}\,\frac{x-\mu_a}{\sqrt{2\sigma^2{}_a}}\right) - \frac{1}{2}\left(1 + \mathrm{erf}\,\frac{x-\mu_b}{\sqrt{2\sigma^2{}_b}}\right) \quad \cdots (3)$$

For example, the first function described in the image G1 in Fig. 3, which will be described later, is represented by Expression (4) below.
[Math. 4]

$$\frac{1}{2}\left(1+\operatorname{erf}\frac{x-\mu_a}{\sqrt{2\sigma^2{}_a}}\right) \quad \cdots (4)$$

For example, the second function in Fig. 3, which will be described below, is represented by Expression (5) below.
[Math. 5]

$$\frac{1}{2}\left(1+\operatorname{erf}\frac{x-\mu_b}{\sqrt{2\sigma^2{}_b}}\right) \quad \cdots (5)$$

For example, the third function in Fig. 3, which will be described below, is represented by Expression (6) below.
[Math. 6]

$$\frac{1}{2}\left(1+\operatorname{erf}\frac{x-\mu_a}{\sqrt{2\sigma^2{}_a}}\right)-\frac{1}{2}\left(1+\operatorname{erf}\frac{x-\mu_b}{\sqrt{2\sigma^2{}_b}}\right) \quad \cdots (6)$$

An average and a variance of a cumulative distribution function of the first function and the second function are obtained by approximating the third function to the R wave or the T wave of the cardiac potential. The processing of approximating the third function to the R wave or the T wave of the cardiac potential is processing of minimizing a difference between the cardiac potential and the third function by a least square method, for example.
The domain of definition of the channel cumulative distribution function used for fitting may not necessarily the same as the domain of definition of the function representing the waveform of the electrocardiogram that is the fitting target. Also, each domain of definition of the channel cumulative distribution function used for fitting may not necessarily the same. The term "not necessarily the same" means that at least one may be different or all may be the same.
The fitting in the ion channel activity information acquisition processing is specifically processing of performing optimization for minimizing a difference between the channel cumulative distribution function and the waveform of the electrocardiogram within the domain of definition of the channel cumulative distribution function using the shape parameters as variables.

(Concerning meaning of a result of fitting using channel cumulative distribution function)

Here, meaning of a result of fitting using the channel cumulative distribution function will be described. In a case where the shape parameter values are fixed, the channel cumulative distribution function is a result of integral of probability density function and has a one-to-one correspondence with the probability density function. Also, in a case where the shape parameter values are fixed, the probability density function is a function representing channel activity timing distribution. Therefore, in a case where the shape parameter values are fixed, the channel cumulative distribution function is a function representing channel activity timing distribution. Therefore, in a case where the shape parameter values are not fixed, the channel cumulative distribution function is a function representing candidates for channel activity timing distribution corresponding to the number of combinations of the shape parameter values.
Incidentally, fitting is typically processing of performing optimization using, as variables, parameter values representing the shape of the function. Therefore, execution of the fitting using the channel cumulative distribution function for the fitting is processing of determining channel activity timing distribution that minimizes a difference from the fitting target from the candidates for the channel activity timing distribution representing the channel cumulative distribution function. Therefore, execution of the fitting using the channel cumulative distribution function for the fitting corresponds to acquisition of information indicating how the ion channel activity is related to the fitting target. Therefore, in a case where the fitting target is the waveform of the electrocardiogram, information indicating how the ion channel activity is related to the waveform of the electrocardiogram is acquired by the fitting.
The relation between the ion channel activity and the waveform of the electrocardiogram is indicated by the shape parameter values of the fitting result, for example. Therefore, the shape parameter values as results of the fitting are examples of the ion channel activity information.

Although the case where one channel cumulative distribution function is used in the fitting has been described hitherto, a plurality of channel cumulative distribution functions may be used for the fitting. Each of the plurality of channel cumulative distribution functions used for the fitting in such a case represents channel activity timing distribution of a mutually different type. In such a case, information indicating how the activities of a plurality of types of ion channels are related to the waveform of the electrocardiogram is acquired. Note that the fact that the types of channel activity timing distribution are different means that any one of or both the types of ion channels and a difference in opening and closing operations of the ion channels are different.

The domain of definition of the channel cumulative distribution function used for fitting may not necessarily the same as the domain of definition of the function representing the waveform of the electrocardiogram that is the fitting target. Also, in a case where the plurality of channel cumulative distribution functions are used for fitting, each domain of definition of each channel cumulative distribution function may not necessarily be the same. The term "not necessarily the same" means that at least one may be different or all may be the same.

Fig. 2 is a diagram illustrating an example of a result of fitting the waveform of the electrocardiogram of the target heart by four channel cumulative distribution functions according to the embodiment. The horizontal axis represents a clock time, and the vertical axis represents a potential in Fig. 2. Units of both the horizontal axis and the vertical axis are arbitrary units.

The four channel cumulative distribution functions in Fig. 2 are specifically a first cumulative distribution function, a second cumulative distribution function, a third cumulative distribution function, and a fourth cumulative distribution function. At least one of domains of definition of the first cumulative distribution function, the second cumulative distribution function, the third cumulative distribution function, and the fourth cumulative distribution function in Fig. 2 differs. Specifically, the domain of definition of the first cumulative distribution function and the domain of definition of the second cumulative distribution function are the same and are the clock time 0 to the clock time T1. The domain of definition of the third cumulative distribution function is the clock time T3 to the clock time T5. The domain of definition of the fourth cumulative distribution function is the clock time T2 to the clock time T4. Note that the domain of definition of the waveform of the electrocardiogram that is the fitting target includes at least a period from the clock time 0 to the clock time T5.

The first cumulative distribution function represents candidates for channel activity timing distribution of a sodium channel that is present in the inner myocardial layer which are candidates for channel activity timing distribution at timings at which excitement of the myocardium starts.

The second cumulative distribution function represents candidates for channel activity timing distribution of a potassium channel that is present in the outer myocardial layer which are candidates for channel activity timing distribution of timings at which excitement of myocardium starts.

The third cumulative distribution function represents candidates for channel activity timing distribution of a sodium channel that is present in the inner myocardial layer which are candidates for channel activity timing distribution of timings at which prevention of excitement of myocardium starts.

The fourth cumulative distribution function represents candidates for channel activity timing distribution of a potassium channel that is present in the outer myocardial layer which are candidates for channel activity timing distribution of timings at which prevention of excitement of myocardium starts.

In Fig. 2, the "first fitting result" indicates a result of fitting to the waveform of the electrocardiogram using the first cumulative distribution function. Hereinafter, fitting to the waveform of the electrocardiogram using the first cumulative distribution function will be referred to as first fitting. In Fig. 2, the "second fitting result" indicates a result of fitting to the waveform of the electrocardiogram using the second cumulative distribution function. Hereinafter, fitting to the waveform of the electrocardiogram using the second cumulative distribution function will be referred to as second fitting. In Fig. 2, the "third fitting result" indicates a result of fitting to the waveform of the electrocardiogram using the third cumulative distribution function. Hereinafter, fitting to the waveform of the electrocardiogram using the third cumulative distribution function will be referred to as third fitting.

In Fig. 2, the "fourth fitting result" indicates a result of fitting to the waveform of the electrocardiogram using the fourth cumulative distribution function. Hereinafter, fitting to the waveform of the electrocardiogram using the fourth cumulative distribution function will be referred to as fourth fitting. In Fig. 2, the "potential on the body surface" represents the waveform of the electrocardiogram of the fitting target. In Fig. 2, all the first cumulative distribution function, the second cumulative distribution function, the third cumulative distribution function, and the fourth cumulative distribution function are specifically cumulative Gaussian distributions.

The "first fitting result" and the "second fitting result" are results of fitting to the R wave. The "third fitting result" and the "fourth fitting result" are results of fitting to the T wave.

Note that the period from the clock time T1 to the clock time T2 does not belong to any of domains of definition of the channel cumulative distribution functions from the first cumulative distribution function to the fourth cumulative distribution function. Therefore, fitting is not performed for the waveforms in the period from the clock time T1 to the clock time T2 among the waveforms of the electrocardiogram illustrated in Fig. 2. In Fig. 2, the period during which fitting is not performed is expressed by a constant function. The period during which fitting is not performed may be expressed by not only the constant function but also any function such as a linear function as long as the function is determined in advance.

Among the fitting results illustrated in Fig. 2, the result obtained by subtracting the "second fitting result" from the "first fitting result" shows substantially the same waveform as the waveform in the rising period of the R wave. Among the fitting results illustrated in Fig. 2, the result obtained by subtracting the "fourth fitting result" from the "third fitting result" shows substantially the same waveform as the waveform in the rising period of the T wave.

Note that the fitting result with respect to the R wave in Fig. 2 may be a result of performing fitting under a condition that the result obtained by subtracting the "second fitting result" from the "first fitting result" shows the waveform that is substantially the same as the waveform in the rising period of the R wave. Note that the fitting result with respect to the T wave in Fig. 2 may be a result of performing fitting under a condition that the result obtained by subtracting the "fourth fitting result" from the "third fitting result" shows the waveform that is substantially the same as the waveform in the rising period of the T wave.

For such two channel cumulative distribution functions having overlapping domains of definition, a difference between the functions may be fitted to the waveform of the electrocardiogram in regard to the overlapping domains of definition. In other words, for the two channel cumulative distribution functions having overlapping domains of definition, fitting that minimizes the difference between the functions and a difference from the waveform of the electrocardiogram may be performed.

The fact that the difference between the two channel cumulative distribution functions is substantially the same as the rising waveform of the R wave will be described using Fig. 3.

Fig. 3 is an explanatory diagram for explaining that a difference in two channel cumulative distribution functions can fit to a waveform that is substantially the same as a rising waveform of an R wave according to the embodiment. Fig. 3 illustrates four images, namely an image G1, an image G2, an image G3, and an image G4. Each of the images G1 to G4 represents a graph having the horizontal axis representing a clock time and the vertical axis representing a potential. Both units of the horizontal axis and the vertical axis in each of the images G1 to G4 in Fig. 3 are arbitrary units.

The "first function" in Fig. 3 is an example of the cumulative distribution function. The "second function" in Fig. 3 is an example of the cumulative distribution function and is a cumulative distribution function that is different from the "second function". The "third function" in Fig. 3 represents a function obtained by subtracting the "second function" from the "first function". The "third function" in Fig. 3 indicates that the shape is substantially the same as the shape of the R wave.

The "fourth function" in Fig. 3 is an example of the cumulative distribution function. The "fifth function" in Fig. 3 is an example of the cumulative distribution function and is a cumulative distribution function that is different from the "fourth function". The "sixth function" in Fig. 3 represents a function obtained by subtracting the "fifth function" from the "fourth function". The "sixth function" in Fig. 3 indicates that the shape is substantially the same as the shape of the R wave.

The "seventh function" in Fig. 3 is an example of the cumulative distribution function. The "eighth function" in Fig. 3 is an example of the cumulative distribution function and is a cumulative distribution function that is different from the "seventh function". The "ninth function" in Fig. 3 represents a function obtained by subtracting the "seventh function" from the "eighth function". The "ninth function" in Fig. 3 indicates that the shape is substantially the same as the shape of the R wave.

The "tenth function" in Fig. 3 is an example of the cumulative distribution function. The "eleventh function" in Fig. 3 is an example of the cumulative distribution function and is a cumulative distribution function that is different from the "tenth function". The "twelfth function" in Fig. 3 represents a function obtained by subtracting the "eleventh function" from the "tenth function". The "twelfth function" in Fig. 3 indicates that the shape is substantially the same as the shape of the R wave.

In this manner, a function of a difference between two cumulative distribution functions can express the shape that is substantially the same as the shape of the R wave. The same applies to the shape of the T wave. In other words, the function of the difference between the two cumulative distribution functions can express the shape that is substantially the same as the shape of the T wave. The function of the difference between the two cumulative distribution function can express the wave in which the width in the vertical axis direction and the width in the horizontal axis direction are different, such as the "third function", the "sixth function", and the "ninth function". Also, the function of the difference between the two cumulative distribution functions can express a negative wave, such as the "twelfth function".

In this manner, the signal analysis device 1 fits the waveform of the electrocardiogram of the target heart by the channel cumulative distribution function. Also, the signal analysis device 1 acquires ion channel activity information on the basis of the fitting result.

The description returns to Fig. 1. The signal analysis device 1 includes a control unit 11 including a processor 91 such as a CPU and a memory 92 which are connected by a bus and executes a program. The signal analysis device 1 functions as a device including a control unit 11, an input unit 12, a communication unit 13, a storage unit 14, and an output unit 15 by executing the program.

More specifically, the processor 91 reads the program stored in the storage unit 14 and stores the read program in the memory 92. The signal analysis device 1 functions as the device including the control unit 11, the input unit 12, the communication unit 13, the storage unit 14, and the output unit 15 by the processor 91 executing the program stored in the memory 92.

The control unit 11 controls operations of various functional units included in the signal analysis device 1. The control unit 11 executes, for example, ion channel activity information acquisition processing. The control unit 11 controls operations of the output unit 15 and causes the output unit 15 to output an acquisition result of the ion channel activity information acquisition processing, for example. The control unit 11 records various kinds of information generated through execution of the ion channel activity information acquisition processing, for example, in the storage unit 14.

The input unit 12 is configured to include an input device such as a mouse, a keyboard, and a touch panel. The input unit 12 may be configured as an interface that connects these input devices to the signal analysis device 1. The input unit 12 receives inputs of various kinds of information to the signal analysis device 1.

Information (hereinafter, referred to as "number designation information") indicating the number of channel cumulative distribution functions used for the fitting, for example, is input to the input unit 12. Information (hereinafter, referred to as "distribution shape designation information") indicating the shape of distribution represented by each channel cumulative distribution function is input to the input unit 12 in regard to each channel cumulative distribution function used for the fitting, for example. Information (hereinafter, referred to as "definition domain designation information") indicating a domain of definition is input to the input unit 12 in regard to each channel cumulative distribution function used for the fitting, for example.

Note that a part of the number designation information, the distribution shape designation information, or the definition domain designation information may be stored in advance in the storage unit 14. In such a case, it is not necessary for the part of the number designation information, the distribution shape designation information, or the definition domain designation information which have already been stored in the storage unit 14 to be input from the input unit 12. Hereinafter, the signal analysis device 1 will be described by exemplifying the case where the storage unit 14 stores the number designation information, the distribution shape designation information, or the definition domain designation information in advance for simplicity of explanation.

The communication unit 13 is configured to include a communication interface for connecting the signal analysis device 1 to an external device. The communication unit 13 communicates with the external device in a wired or wireless manner. The external device is, for example, a device that is a transmission source of the waveform of the electrocardiogram of the target heart. The device that is the transmission source of the waveform of the electrocardiogram of the target heart is, for example, an electrocardiogram measurement device. In a case where the external device is an electrocardiogram measurement device, for example, the communication unit 13 acquires the waveform of the electrocardiogram from the electrocardiogram measurement device through communication. Note that the waveform of the electrocardiogram may be input to the input unit 12.

The storage unit 14 is configured using a non-transitory computer-readable storage medium device such as a magnetic hard disk device or a semiconductor storage device. The storage unit 14 stores various kinds of information regarding the signal analysis device 1. The storage unit 14 stores, for example, information input via the input unit 12 or the communication unit 13. The storage unit 14 stores, for example, the electrocardiogram input via the input unit 12 or the communication unit 13. The storage unit 14 stores, for example, various kinds of information generated through execution of the ion channel activity information acquisition processing.

The output unit 15 outputs various kinds of information. The output unit 15 is configured to include, for example, a display device such as a cathode ray tube (CRT) display, a liquid crystal display, or an organic electro-luminescence (EL) display. The output unit 15 may be configured as an interface that connects these display devices to the signal analysis device 1. The output unit 15 outputs, for example, information input to the input unit 12. The output unit 15 may display the electrocardiogram input to the input unit 12 or the communication unit 13, for example. The output unit 15 may display an execution result of the ion channel activity information acquisition processing, for example.

Fig. 4 is a diagram illustrating an example of a functional configuration of the control unit 11 according to the embodiment. The control unit 11 includes an electrocardiogram acquisition unit 110, a fitting information acquisition unit 120, an analysis unit 130, and a recording unit 140.

The electrocardiogram acquisition unit 110 acquires the waveform of the electrocardiogram of the target heart input to the input unit 12 or the communication unit 13. The fitting information acquisition unit 120 acquires the number designation information, the distribution shape designation information, and the definition domain designation information. In a case where the number designation information, the distribution shape designation information, and the definition domain designation information are stored in the storage unit 14, the fitting information acquisition unit 120 reads, from the storage unit 14, the number designation information, the distribution shape designation information, and the definition domain designation information.

The analysis unit 130 includes a fitting unit 131 and an ion channel activity information acquisition unit 132.

The fitting unit 131 performs fitting to the waveform of the electrocardiogram acquired by the electrocardiogram acquisition unit 110 using the channel cumulative distribution function indicated by the number designation information, the distribution shape designation information, and the definition domain designation information.

The ion channel activity information acquisition unit 132 acquires ion channel activity information on the basis of a result of the fitting performed by the fitting unit 131. The ion channel activity information acquisition unit 132 acquires a shape parameter value for each channel cumulative distribution function, for example. The shape parameter value acquired by the ion channel activity information acquisition unit 132 is an example of ion channel activity information.

In this manner, the analysis unit 130 acquires the ion channel activity information on the basis of the waveform of the electrocardiogram of the target heart and the distribution candidate information.

The storage unit 14 records, in the storage unit 14, various kinds of information generated through processing executed by the control unit 11.

Fig. 5 is a flowchart illustrating an example of a flow of processing executed by the signal analysis device 1 according to the embodiment. The electrocardiogram acquisition unit 110 acquires the electrocardiogram of the target heart via the input unit 12 or the communication unit 13 (Step S101). Next, the fitting information acquisition unit 120 acquires the number definition information, the distribution shape definition information, and the definition domain designation information (Step S102). Next, the fitting unit 131 performs fitting to the waveform of the electrocardiogram acquired in Step S101 using the channel cumulative distribution function indicated by the number designation information, the distribution shape designation information, and the definition domain designation information (Step S103). Next, the ion channel activity information acquisition unit 132 acquires ion channel activity information on the basis of the fitting result (Step S104). The acquired ion channel activity information is output to the output unit 15 (Step S105).

In Step S105, a graph of the fitting result of each channel cumulative distribution function may be displayed. Also, it is only necessary for the processing in Step S102 to be executed before execution of the processing in Step S103, and the processing in Step S102 may be executed before execution of Step S101. The processing in Step S103 and Step S104 is an example of processing executed by the analysis unit 130.

Fig. 6 is a first diagram illustrating an example of an analysis result of the signal analysis device 1 according to the embodiment. More specifically, Fig. 6 is an example of a result obtained by the signal analysis device 1 performing analysis on a waveform of an electrocardiogram of the target heart that operates normally. The horizontal axis represents a clock time, and the vertical axis represents a potential in Fig. 6. The unit of the vertical axis is an arbitrary unit.

Fig. 6 illustrates results of performing first fitting and second fitting on an R waveform of depolarization of the electrocardiogram of the target heart that operates normally. Also, Fig. 6 illustrates an example of results of performing fourth fitting and third fitting on a T waveform of a repolarization phase of the electrocardiogram of the target heart that operates normally.

Fig. 6 illustrates that in the depolarization of the normal heart, an ion channel activity starts earlier and more rapidly advances in the inner myocardial layer than in the outer myocardial layer. Fig. 6 illustrates that the outer myocardial layer starts its activity with a slight delay from a timing at which the activity of the ion channel in the inner myocardial layer starts. Fig. 6 illustrates that a difference between the timing at which the activity of the ion channel in the inner myocardial layer starts and the timing at which the outer myocardial layer starts its activity shows a positive sharp R waveform. An average value and variance of an inner-layer-side cumulative distribution function which is the first fitting result and an outer-layer-side cumulative distribution function which is the second fitting result represent a timing of the activity and a progress of the activity, respectively.

Fig. 6 illustrates that inactivation of the activity of the ion channel starts earlier in the outer myocardial layer than in the inner myocardial layer in the repolarization phase of the normal heart. Fig. 6 illustrates that inactivation in the inner myocardial layer has a delay and inactivation in the both advances slowly. The difference shows a positive and gentle T waveform. An average value and variance of an inner-layer-side cumulative distribution function which is the fourth fitting result and an outer-layer-side cumulative distribution function which is the third fitting result represent a timing of inactivation and a progress of the inactivation, respectively. As described above, the average and the variance of the inner-layer-side cumulative distribution function and the outer-layer-side cumulative distribution function obtained through the first to fourth fitting represent a collective activation of the ion channel of depolarization and a timing and a progress of collective inactivation of the ion channel in the repolarization phase, respectively.

The shapes of the inner-layer-side cumulative distribution function and the outer-layer-side cumulative distribution function in Fig. 6 substantially coincide with results of directly measuring an electromotive force by inserting a catheter into the myocardium of the target heart that operates normally. Therefore, Fig. 6 illustrates that the signal analysis device 1 acquires the ion channel activity information only from the electrocardiogram without inserting the catheter.

Fig. 7 is a second diagram illustrating an example of an analysis result obtained by the signal analysis device 1 according to the embodiment. More specifically, Fig. 7 is an example of a result obtained by the signal analysis device 1 performing analysis on a waveform of an electrocardiogram of the target heart that operates normally.

Fig. 7 illustrates three results of a graph G5, a graph G6, and a result G7. In Fig. 7, the "inner-layer-side cumulative distribution function" indicates a fitting result of a function representing collective channel activity timing distribution candidates for an ion channel that is present in the inner myocardial layer. In Fig. 7, the "outer-layer-side cumulative distribution function" indicates a fitting result of a function representing collective channel activity timing distribution candidates for an ion channel that is present in the outer myocardial layer. In Fig. 7, the "potential on the body surface" is a function representing a temporal change in potential on the body surface and is a waveform of the electrocardiogram. The horizontal axis represents a clock time, and the vertical axis represents a potential in Fig. 7. Units of both the horizontal axis and the vertical axis are arbitrary units. Note that the time lengths represented by one-scale interval on the horizontal axes in Figs. 7 to 9 are the same. Also, 1 on all the vertical axes in Figs. 7 to 9 represents the maximum value of cumulative Gaussian distributions.

The graph G5 represents all the waveforms of the electrocardiogram generated in one-time pulsation. The graph G6 illustrates an enlarged view of a region of a T wave which is a part of the graph G5. The region of the T wave is a region indicated as a region A1 in Fig. 7. The result G7 indicates the amounts of statistics of two Gaussian distributions. Each value in the result G7 represents the amount of statistics of the two Gaussian distributions. Specifically, the amounts of statistics of two Gaussian distributions are average values and variances of Gaussian distributions of the inner-layer-side cumulative distribution function and the outer-layer-side cumulative distribution function.

Fig. 8 is a third diagram illustrating an example of an analysis result obtained by the signal analysis device 1 according to the embodiment. More specifically, Fig. 8 is an example of a result obtained by the signal analysis device 1 performing analysis on the waveform of the electrocardiogram of the target heart whose operation is of a T extension type III.

Fig. 8 illustrates three results of a graph G8, a graph G9, and a result G10. In Fig. 8, the "inner-layer-side cumulative distribution function" indicates a fitting result of a function representing collective channel activity timing distribution candidates for a channel that is present in the inner myocardial layer. In Fig. 8, the "outer-layer-side cumulative distribution function" indicates a fitting result of a function representing collective channel activity timing distribution candidates for a channel that is present in the outer myocardial layer. In Fig. 8, the "potential on the body surface" is a function representing a temporal change in potential on the body surface and is a waveform of the electrocardiogram. The horizontal axis represents a clock time, and the vertical axis represents a potential in Fig. 8. Units of both the horizontal axis and the vertical axis are arbitrary units.

The graph G8 represents all the waveforms of the electrocardiogram generated in one-time pulsation. The graph G9 illustrates an enlarged view of a region of a T wave which is a part of the graph G8. The region of the T wave is a region indicated as a region A2 in Fig. 8. The result G10 indicates the amounts of statistics of two Gaussian distributions. Each value of the result G10 represents the amounts of statistics of two Gaussian distributions, that is, average values and variances of the Gaussian distributions of the inner-layer-side cumulative distribution function and the outer-layer-side cumulative distribution function.

The shapes of the inner-layer-side cumulative distribution function and the outer-layer-side cumulative distribution function in Fig. 8 substantially coincide with results of directly measuring changes in electromotive force generated through pulsation of the outer myocardial layer of the target heart whose operation is of a T extension type III by inserting a catheter. Therefore, Fig. 8 illustrates that the signal analysis device 1 acquires the ion channel activity information only from the electrocardiogram without inserting the catheter.

Fig. 9 is a fourth diagram illustrating an example of an analysis result of the signal analysis device 1 according to the embodiment. More specifically, Fig. 9 is an example of a result obtained by the signal analysis device 1 performing analysis on the waveform of the electrocardiogram of the target heart whose operation is of a QT extension type I.

Fig. 9 illustrates three results of a graph G11, a graph G12, and a result G13. In Fig. 9, the "inner-layer-side cumulative distribution function" indicates a fitting result of a function representing collective channel activity timing distribution candidates for a channel that is present in the inner myocardial layer. In Fig. 9, the "outer-layer-side cumulative distribution function" indicates a fitting result of a function representing collective channel activity timing distribution candidates for a channel that is present in the outer myocardial layer. In Fig. 9, the "potential on the body surface" is a function representing a temporal change in potential on the body surface and is a waveform of the electrocardiogram. The horizontal axis represents a clock time, and the vertical axis represents a potential in Fig. 9. Units of both the horizontal axis and the vertical axis are arbitrary units.

The graph G11 represents all the waveforms of the electrocardiogram generated in one-time pulsation. The graph G12 illustrates an enlarged view of a region of a T wave which is a part of the graph G11. The region of the T wave is a region indicated as a region A3 in Fig. 8. The result G13 indicates the amounts of statistics of two Gaussian distributions. Each value in the result G13 represents the amount of statistics of the two Gaussian distributions. Specifically, the amounts of statistics of two Gaussian distributions are average values and variances of Gaussian distributions of the inner-layer-side cumulative distribution function and the outer-layer-side cumulative distribution function.

The shapes of the inner-layer-side cumulative distribution function and the outer-layer-side cumulative distribution function in Fig. 9 substantially coincide with results of directly measuring changes in electromotive force generated through pulsation of the outer myocardial layer of the target heart whose operation is of a QT extension type III by inserting a catheter. Therefore, Fig. 9 illustrates that the signal analysis device 1 acquires the ion channel activity information only from the electrocardiogram without inserting the catheter. It illustrates that the signal analysis device 1 acquires the ion channel activity information only from the electrocardiogram.

Note that Fig. 9 is also an example of a result of estimating channel current properties related to sudden death obtained by the signal analysis device 1.

The fact that the signal analysis device 1 acquires ion channel activity information only from an electrocardiogram in a case of the electrocardiogram of premature ventricular contraction as well will be illustrated using Figs. 10 to 12. The horizontal axis represents a clock time (second), and the vertical axis represents a potential (mV) in Figs. 10 to 12.

Fig. 10 is a first explanatory diagram of an example in which the signal analysis device 1 analyzes an electrocardiogram of premature ventricular contraction according to the embodiment. Fig. 11 is a second explanatory diagram of an example in which the signal analysis device 1 analyzes an electrocardiogram of premature ventricular contraction according to the embodiment. Fig. 12 is a third explanatory diagram of an example in which the signal analysis device 1 analyzes an electrocardiogram of premature ventricular contraction according to the embodiment.

More specifically, Fig. 10 illustrates a cardiac potential on the body surface. In other words, Fig. 10 illustrates one normal heartbeat recorded in an electrocardiogram and premature ventricular contraction occurring twice in straight. More specifically, Fig. 11 illustrates an inner-layer-side cumulative distribution function and an outer-layer-side cumulative distribution function of depolarization and an inner-layer-side cumulative distribution function in the repolarization phase of the premature ventricular contraction analyzed by the signal analysis device 1. More specifically, Fig. 12 illustrates an example of a waveform of the premature ventricular contraction in the electrocardiogram actually measured.

Fig. 11 illustrates that the inner-layer-side cumulative distribution function of depolarization precedes the outer-layer-side cumulative distribution function, variance of the both is greater than that of normal heartbeats, and spread of excitement is gentle. This analysis results coincide with features of a waveform of a wide-ranging R wave.

Fig. 11 illustrates that the inner-layer-side cumulative distribution function starts inactivation earlier than the outer-layer-side cumulative distribution function in the repolarization phase, and it is shown as an average value of the cumulative distribution functions. These coincide with features of a large negative T wave in the repolarization phase, and the waveform obtained from the difference in cumulative distribution functions substantially coincide with the waveform of the premature ventricular contraction of the electrocardiogram actually measured as illustrated in Fig. 12.

Note that the results in Figs. 10 to 12 show that the signal analysis device 1 is compatible with examples in which a huge wave or a negative potential occurs due to transmission of a change in excitement of the myocardium, early repolarization, or a delay of repolarization. Note that in Figs. 10 to 12, an average $\mu$ of the inner-layer-side cumulative distribution function in the depolarization phase is 0.46 and variance $\sigma^2$ is 1.43. Also, an average $\mu$ of the outer-layer-side cumulative distribution function in depolarization is -1, and variance $\sigma^2$ is 0.99 in Figs. 10 to 12. Note that in Figs. 10 to 12, an average $\mu$ of the inner-layer-side cumulative distribution function in the repolarization phase is 0.46 and variance $\sigma^2$ is 1.43. Also, an average $\mu$ of the outer-layer-side cumulative distribution function in the repolarization phase is -1, and variance $\sigma^2$ is 0.99 in Figs. 10 to 12. Variance $1 = \sigma^2$ corresponds to 0.087 seconds.

The fact that the signal analysis device 1 acquires ion channel activity information only from an electrocardiogram in a case of the electrocardiogram of the target heart in the depolarization period of the Brugada syndrome type 1 will be described using Figs. 13 to 15. The vertical axis in Figs. 13 to 15 represents a potential in units of millivolts.

Fig. 13 is a first explanatory diagram in which the signal analysis device 1 analyzes an electrocardiogram of the target heart in the depolarization period of the Brugada syndrome type 1 according to the embodiment. Fig. 14 is a second explanatory diagram in which the signal analysis device 1 analyzes an electrocardiogram of the target heart in the depolarization period of the Brugada syndrome type 1 according to the embodiment. Fig. 15 is a third explanatory diagram in which the signal analysis device 1 analyzes an electrocardiogram of the target heart in the depolarization period of the Brugada syndrome type 1 according to the embodiment.

More specifically, Fig. 13 illustrates an electrocardiogram of precordial lead II of the Brugada syndrome. In Fig. 13, the inner frame W1 and the inner frame W2 indicate distinction of the depolarization phase and the repolarization phase, respectively. The same applies to Figs. 14 and 15. In other words, the inner frame W1 represents the depolarization phase, and the inner frame W2 represents the repolarization phase in Figs. 14 and 15 as well.

More specifically, Fig. 14 illustrates the inner-layer-side cumulative distribution function and the outer-layer-side cumulative distribution function in the depolarization and repolarization phases analyzed by the signal analysis device 1. In the example illustrated in Fig. 14, the repolarization phase of the inner-layer-side cumulative distribution function starts to follow the depolarization phase and shows features of early repolarization. On the other hand, in regard to a potential amplitude of the outer-layer-side cumulative distribution function in the example in Fig. 14, a difference is observed between the depolarization phase and the repolarization phase. Also, Fig. 14 illustrates a gap and anisotropy of the depolarization phase and the repolarization phase of the outer-layer-side cumulative distribution function. In this manner, the signal analysis device 1 can represent early repolarization and anisotropy of depolarization and repolarization which are features of the waveform of the electrocardiogram of the target heart of the Brugada syndrome using an average, variance, and a ratio in the depolarization phase and the repolarization phase of the inner-layer-side cumulative distribution function and the outer-layer-side cumulative distribution function.

Fig. 15 is comparison between an analysis result and actually measured values. More specifically, Fig. 15 illustrates a difference between the inner-layer-side cumulative distribution function and the outer-layer-side cumulative distribution function in the depolarization and repolarization phases in Fig. 14. Additionally, Fig. 15 also illustrates an actually measured values of the electrocardiogram. The analysis result and the actually measured values substantially coincide with each other except for the end part. The end part means the potential in late time.

Note that in Figs. 13 to 15, an average $\mu$ of the inner-layer-side cumulative distribution function in the depolarization phase is 15 and variance $\sigma^2$ is 0.15. Also, an average $\mu$ of the outer-layer-side cumulative distribution function in depolarization is 14, and variance $\sigma^2$ is 0.25 in Figs. 13 to 15. In addition, the ratio of the inner and outer layers in depolarization is 0.45 in Figs. 13 to 15. Also, an average $\mu$ of the inner-layer-side cumulative distribution function in the repolarization phase is 25, and variance $\sigma^2$ is 0.25 in Figs. 13 to 15. Also, an average $\mu$ of the outer-layer-side cumulative distribution function in the repolarization phase is 20, and variance $\sigma^2$ is 0.5 in Figs. 13 to 15. In addition, the ratio of the inner and outer layers in depolarization is 0.75 in Figs. 13 to 15. Additionally, the ratio of polarization and repolarization is 1.4 in Figs. 13 to 15.

Fig. 16 to 54 illustrates a result of performing analysis by the signal analysis device 1 using a published electrocardiogram data library https://physionet.org/about/database/. Figs. 16 to 54 illustrate inner-layer-side cumulative distribution functions and outer-layer-side cumulative distribution functions and fitting results obtained by the signal analysis device 1 executing analysis on cardiac potentials.

Each of Figs. 16 to 54 is a diagram illustrating an example in which the signal analysis device 1 analyzes an electrocardiogram according to the embodiment. Determined points illustrated in each drawing represent a point Q, a point R, a point S, a T start point, and a T end point, respectively, of the cardiac potential in the order from the left side in the drawing. The determined points are determined by inflection point detection and peak detection algorithms. Each of Figs. 16 to 54 illustrates an inner-layer-side cumulative distribution function and an outer-layer-side cumulative distribution function in each section obtained for a section of the depolarization phase (QRS wave) and a section of the repolarization phase (T wave). Figs. 16 to 54 illustrate that the signal analysis device 1 can show substantially the same shape by adjusting an average and variance of the inner-layer-side cumulative distribution function and the outer-layer-side cumulative distribution function for various QRS waves and T waves. The lower diagrams in Figs. 16 to 54 represent original waveforms of electrocardiograms and fitting results. Note that each of the results in Figs. 16 to 54 is a result of sampling at 300 Hz. Therefore, the origin of the horizontal axis represents 0 seconds, and the value 1 represents 3.33 milliseconds in each of Figs. 16 to 54.

The signal analysis device 1 configured in this manner fits the waveform of the electrocardiogram of the target heart using one or a plurality of channel cumulative distribution functions and acquires ion channel activity information on the basis of the shape of each channel cumulative distribution function in the fitting result. Each channel cumulative distribution function used for fitting is information indicating candidates for channel activity timing distribution. The channel activity timing distribution represents distribution of timings of the activity of the ion channel, and each of mutually different channel cumulative distribution functions represents a different kind of channel activity timing distribution. Therefore, each shape of each channel cumulative distribution function in the fitting result indicates information of channel activity timing distribution represented by each channel cumulative distribution function. Such information cannot be obtained through conventional analysis of the waveform of the electrocardiogram. Therefore, the signal analysis device 1 can increase information obtained from the waveform of the electrocardiogram.

(Modification Example)

Note that the number of channel cumulative distribution functions with overlapping domains of definition may not necessarily be two and may be three or more. In such a case, fitting to minimize a difference between the waveform of the electrocardiogram and a linear sum of a plurality of channel cumulative distribution functions which have overlapping domains of definition and satisfy a predetermined condition regarding weights may be performed. The predetermined conditions regarding weights may be any conditions as long as they include at least a condition that at least one of the weights has a sign that is different from that of the other weights.

Note that the electrocardiogram is preferably an electrocardiogram of lead that is close to an electromotive force vector. The electrocardiogram of lead that is close to the electromotive force vector is preferably an electrocardiogram capable of acquiring 3D information of the cardiac potential, such as lead II, lead V4, or lead V5, for example. Since the amount of information increases as the number of channels in the electrocardiogram increases, it is more desirable that the electrocardiogram has a larger number of channels.

Note that the signal analysis device 1 may be implemented using a plurality of information processing devices that are communicably connected via a network. In this case, each functional unit included in the signal analysis device 1 may be implemented in a distributed manner by the plurality of information processing devices.

Note that the electrocardiogram acquisition unit 110 is an example of a biological information acquisition unit.

Note that all or some of the functions of the signal analysis device 1 may be realized using hardware such as an application specific integrated circuit (ASIC), a programmable logic device (PLD), or a field programmable gate array (FPGA). The program may be recorded in a computer-readable recording medium. The computer-readable recording medium is a storage device such as a portable medium such as a flexible disk, a magneto-optical disc, a ROM, or a CD-ROM or a hard disk incorporated in a computer system. The program may be transmitted via an electric communication line.

Although the embodiments of the present invention have been described in detail with reference to the drawings, specific configurations are not limited to the embodiments but rather are defined by the claims.

Reference Signs List

| | |
|---|---|
| 1 | Signal analysis device |
| 11 | Control unit |
| 12 | Input unit |
| 13 | Communication unit |
| 14 | Storage unit |
| 15 | Output unit |
| 110 | Electrocardiogram acquisition unit |
| 120 | Fitting information acquisition unit |
| 130 | Analysis unit |
| 131 | Fitting unit |
| 132 | Ion channel activity information acquisition unit |
| 140 | Recording unit |
| 91 | Processor |
| 92 | Memory |

## Claims

**1.** A signal analysis device (1) comprising:

a biological information acquisition unit (11, 110) that acquires time-series biological information regarding pulsation of a heart that is an analysis target; and
an analysis unit (11, 130) that acquires information indicating a state of an activity of an ion channel in the heart on the basis of the biological information and distribution candidate information using, as the distribution candidate information, information indicating candidates for channel activity timing distribution which is distribution of timings of the activity of the ion channel, wherein the distribution candidate information is expressed by a cumulative distribution function using a sigmoid function representing candidates for channel activity timing distribution as a probability density function, wherein the analysis unit (11, 130) acquires information indicating a state of the activity of the ion channel on the basis of a result of fitting one or a plurality of the cumulative distribution functions to the biological information, **characterized in that** the fitting is fitting that minimizes a difference between the biological information and a linear sum of the plurality of cumulative distribution sigmoid functions which includes overlapping domains of definition and satisfies predetermined conditions regarding weights including at least a condition that at least one of the weights has a sign that is different from that of other weights, said sigmoid functions being independent of each other.

**2.** A signal analysis device (1) according to claim 1, wherein each cumulative distribution function is a functional having one or a plurality of shape parameters.

**3.** A signal analysis method comprising:

a biological information acquisition step (S101) of acquiring time-series biological information regarding pulsation of a heart that is an analysis target; and
an analysis step (S104) of acquiring information indicating a state of an activity of an ion channel on the basis of the biological information and distribution candidate information using, as the distribution candidate information, information indicating candidates for channel activity timing distribution which is distribution of timings of the activity of the ion channel in the heart,

wherein the distribution candidate information is expressed by a cumulative distribution function using a sigmoid function representing candidates for channel activity timing distribution as a probability density function, wherein the analysis unit acquires information indicating a state of the activity of the ion channel on the basis of a result of fitting one or a plurality of the cumulative distribution functions to the biological information, wherein the fitting is fitting that minimizes a difference between the biological information and a linear sum of the plurality of cumulative distribution sigmoid functions which includes overlapping domains of definition and satisfies predetermined conditions regarding weights including at least a condition that at least one of the weights has a sign that is different from that of other weights, said sigmoid functions being independent of each other.

**4.** A program that causes a computer to function as the signal analysis device according to claim 1.

**5.** A signal analysis device (1) comprising:

a biological information acquisition unit (11, 110) that acquires time-series biological information regarding pulsation of a heart that is an analysis target; and
an analysis unit (11, 130) that acquires information indicating a state of an activity of an ion channel in the heart on the basis of the biological information and distribution candidate information using, as the distribution candidate information, information indicating candidates for channel activity timing distribution which is distribution of timings of the activity of the ion channel,

**characterized in that** the distribution candidate information is expressed by a cumulative distribution function using a function representing candidates for channel activity timing distribution as a probability density function, wherein the cumulative distribution function is a Gompertz function or a Gudermann function.

**6.** The signal analysis device according to claim 5, wherein the analysis unit (11, 130) acquires information indicating a state of the activity of the ion channel on the basis of a result of fitting one or a plurality of the cumulative distribution functions to the biological information.

**7.** The signal analysis device according to claim 6, wherein the fitting is fitting that minimizes a difference between the biological information and a linear sum of the plurality of cumulative distribution functions which includes overlapping domains of definition and satisfies predetermined conditions regarding weights including at least a condition that at least one of the weights has a sign that is different from that of other weights.

**8.** A signal analysis method comprising:

a biological information acquisition step (S101) of acquiring time-series biological information regarding pulsation of a heart that is an analysis target; and
an analysis step (S104) of acquiring information indicating a state of an activity of an ion channel on the basis of the biological information and distribution candidate information using, as the distribution candidate information, information indicating candidates for channel activity timing distribution which is distribution of timings of the activity of the ion channel in the heart,

**characterized in that** the distribution candidate information is expressed by a cumulative distribution function using a function representing candidates for channel activity timing distribution as a probability density function, wherein the cumulative distribution function is a Gompertz function or a Gudermann function.

**9.** A program that causes a computer to function as the signal analysis device according to any one of claims 5 to 7.

## Patentansprüche

**1.** Signalanalysevorrichtung (1), umfassend:

eine biologische Informationserwerbungseinheit (11, 110), die zeitserielle biologische Informationen in Bezug auf die Pulsation eines Herzens erwirbt, das ein Analyseziel ist; und
eine Analyseeinheit (11, 130), die Informationen erwirbt, die einen Zustand einer Aktivität eines Ionenkanals in dem Herzen auf der Grundlage der biologischen Informationen und der Verteilungskandidateninformationen anzeigen, indem sie als Verteilungskandidateninformationen Informationen verwendet, die Kandidaten für die Kanalaktivitätszeitverteilung anzeigen, was die Verteilung von Zeiten der Aktivität des Ionenkanals ist, wobei die Verteilungskandidateninformationen durch eine kumulative Verteilungsfunktion unter Verwendung einer Sigmoidfunktion ausgedrückt werden, die Kandidaten für die Kanalaktivitätszeitverteilung als Wahrscheinlichkeitsdichtefunktion darstellt, wobei die Analyseeinheit (11, 130) Informationen erwirbt, die einen Zustand der Aktivität des Ionenkanals auf der Grundlage eines Ergebnisses des Anpassens einer oder einer Vielzahl der kumulativen Verteilungsfunktionen an die biologischen Informationen angeben,
**dadurch gekennzeichnet, dass** das Anpassen ein Anpassen ist, das eine Differenz zwischen den biologischen Informationen und einer linearen Summe der Vielzahl von kumulativen Verteilungssigmoidfunktionen minimiert, die überlappende Definitionsbereiche einschließt und vorbestimmte Bedingungen in Bezug auf Gewichte erfüllt, einschließlich mindestens einer Bedingung, dass mindestens eines der Gewichte ein Vorzeichen aufweist, das sich von dem anderer Gewichte unterscheidet, wobei die Sigmoidfunktionen voneinander unabhängig sind.

**2.** Signalanalysevorrichtung (1) nach Anspruch 1, wobei jede kumulative Verteilungsfunktion eine funktionale Funktion ist, die einen oder eine Vielzahl von Formparametern aufweist.

**3.** Signalanalyseverfahren, umfassend:

einen biologischen Informationserwerbungsschritt (S101) zum Erwerben von zeitseriellen biologischen Informationen in Bezug auf die Pulsation eines Herzens, das ein Analyseziel ist; und
einen Analyseschritt (S104) zum Erwerben von Informationen, die einen Zustand einer Aktivität eines Ionenkanals auf der Grundlage der biologischen Informationen und der Verteilungskandidateninformationen anzeigen, wobei als Verteilungskandidateninformationen Informationen verwendet werden, die Kandidaten für die Kanalaktivitätszeitverteilung anzeigen, die die Verteilung von Zeiten der Aktivität des Ionenkanals in dem Herzen ist,
wobei die Verteilungskandidateninformationen durch eine kumulative Verteilungsfunktion unter Verwendung einer Sigmoidfunktion ausgedrückt werden, die Kandidaten für die Kanalaktivitätszeitverteilung als Wahrscheinlichkeitsdichtefunktion darstellt, wobei die Analyseeinheit Informationen erwirbt, die einen Zustand der Aktivität des Ionenkanals auf der Grundlage eines Ergebnisses des Anpassens einer oder einer Vielzahl der kumulativen Verteilungsfunktionen an die biologischen Informationen angeben, wobei das Anpassen ein Anpassen ist, das eine Differenz zwischen den biologischen Informationen und einer linearen Summe der Vielzahl von kumulativen Verteilungssigmoidfunktionen minimiert, die überlappende Definitionsbereiche einschließt und vorbestimmte Bedingungen in Bezug auf Gewichte erfüllt, einschließlich mindestens einer Bedingung, dass mindestens eines der Gewichte ein Vorzeichen aufweist, das sich von dem anderer Gewichte unterscheidet, wobei die Sigmoidfunktionen voneinander unabhängig sind.

**4.** Programm, das einen Computer dazu veranlasst, als Signalanalysevorrichtung nach Anspruch 1 zu funktionieren.

**5.** Signalanalysevorrichtung (1), umfassend:

eine biologische Informationserwerbungseinheit (11, 110), die zeitserielle biologische Informationen in Bezug auf die Pulsation eines Herzens erwirbt, das ein Analyseziel ist; und
eine Analyseeinheit (11, 130), die Informationen erwirbt, die einen Zustand einer Aktivität eines Ionenkanals in dem Herzen auf der Grundlage der biologischen Informationen und der Verteilungskandidateninformationen anzeigen, indem sie als Verteilungskandidateninformationen Informationen verwendet, die Kandidaten für die Kanalaktivitätszeitverteilung anzeigen, was die Verteilung von Zeiten der Aktivität des Ionenkanals ist,
**dadurch gekennzeichnet, dass** die Verteilungskandidateninformationen durch eine kumulative Verteilungsfunktion unter Verwendung einer Funktion ausgedrückt werden, die Kandidaten für die Kanalaktivitätszeitverteilung als Wahrscheinlichkeitsdichtefunktion darstellt, wobei die kumulative Verteilungsfunktion eine Gompertz-Funktion oder eine Gudermann-Funktion ist.

**6.** Signalanalysevorrichtung nach Anspruch 5, wobei die Analyseeinheit (11, 130) Informationen erwirbt, die einen Zustand der Aktivität des Ionenkanals auf der Grundlage eines Ergebnisses des Anpassens einer oder einer Vielzahl der kumulativen Verteilungsfunktionen an die biologischen Informationen anzeigen.

**7.** Signalanalysevorrichtung nach Anspruch 6, wobei das Anpassen ein Anpassen ist, das eine Differenz zwischen den biologischen Informationen und einer linearen Summe der Vielzahl von kumulativen Verteilungsfunktionen minimiert, die überlappende Definitionsbereiche einschließt und vorgegebene Bedingungen hinsichtlich Gewichten erfüllt, einschließlich mindestens einer Bedingung, dass mindestens eines der Gewichte ein Vorzeichen hat, das sich von dem der anderen Gewichte unterscheidet.

**8.** Signalanalyseverfahren, umfassend:

einen biologischen Informationserwerbungsschritt (S101) zum Erwerben von zeitseriellen biologischen Informationen in Bezug auf die Pulsation eines Herzens, das ein Analyseziel ist; und
einen Analyseschritt (S104) zum Erwerben von Informationen, die einen Zustand einer Aktivität eines Ionenkanals auf der Grundlage der biologischen Informationen und der Verteilungskandidateninformationen anzeigen, wobei als Verteilungskandidateninformationen Informationen verwendet werden, die Kandidaten für die Kanalaktivitätszeitverteilung anzeigen, die die Verteilung von Zeiten der Aktivität des Ionenkanals in dem Herzen ist,
**dadurch gekennzeichnet, dass** die Verteilungskandidateninformationen durch eine kumulative Verteilungsfunktion unter Verwendung einer Funktion ausgedrückt werden, die Kandidaten für die Kanalaktivitätszeitverteilung als Wahrscheinlichkeitsdichtefunktion darstellt, wobei die kumulative Verteilungsfunktion eine Gompertz-Funktion oder eine Gudermann-Funktion ist.

**9.** Programm, das einen Computer dazu veranlasst, als Signalanalysevorrichtung nach einem der Ansprüche 5 bis 7 zu funktionieren.

## Revendications

**1.** Dispositif d'analyse de signal (1) comportant :

une unité d'acquisition d'informations biologiques (11, 110) qui acquiert des informations biologiques chronologiques concernant la pulsation d'un cœur qui est une cible d'analyse ; et
une unité d'analyse (11, 130) qui acquiert des informations indiquant un état d'une activité d'un canal ionique dans le cœur sur la base des informations biologiques et des informations de candidats de distribution en utilisant, en tant qu'informations de candidats de distribution, des informations indiquant des candidats pour la distribution temporelle d'activité de canal qui est la distribution de synchronisations de l'activité du canal ionique, dans lequel les informations de candidats de distribution sont exprimées par une fonction de distribution cumulative utilisant une fonction sigmoïde représentant des candidats pour la distribution temporelle d'activité de canal en tant que fonction de densité de probabilité, dans lequel l'unité d'analyse (11, 130) acquiert des informations indiquant un état de l'activité du canal ionique sur la base d'un résultat d'ajustement d'une ou d'une pluralité des fonctions de distribution cumulative aux informations biologiques,
**caractérisé en ce que** l'ajustement est un ajustement qui minimise une différence entre les informations biologiques et une somme linéaire de la pluralité de fonctions sigmoïdes de distribution cumulative qui inclut des domaines de définition se chevauchant et satisfait à des conditions prédéterminées concernant des poids incluant au moins une condition selon laquelle au moins l'un des poids présente un signe qui est différent de celui des autres poids, lesdites fonctions sigmoïdes étant indépendantes les unes des autres.

**2.** Dispositif d'analyse de signal (1) selon la revendication 1, dans lequel chaque fonction de distribution cumulative est une fonctionnelle ayant un ou une pluralité de paramètres de forme.

**3.** Procédé d'analyse de signal comportant :

une étape d'acquisition d'informations biologiques (S101) consistant à acquérir des informations biologiques chronologiques concernant la pulsation d'un cœur qui est une cible d'analyse ; et
une étape d'analyse (S104) consistant à acquérir des informations indiquant un état d'une activité d'un canal ionique sur la base des informations biologiques et d'informations de candidats de distribution en utilisant, en tant qu'informations de candidats de distribution, des informations indiquant des candidats pour la distribution temporelle d'activité de canal qui est la distribution de synchronisations de l'activité du canal ionique dans le cœur, dans lequel les informations de candidats de distribution sont exprimées par une fonction de distribution cumulative utilisant une fonction sigmoïde représentant des candidats pour la distribution temporelle d'activité de canal en tant que fonction de densité de probabilité, dans lequel l'unité d'analyse acquiert des informations indiquant un état de l'activité du canal ionique sur la base d'un résultat d'ajustement d'une ou d'une pluralité des fonctions de distribution cumulative aux informations biologiques, dans lequel l'ajustement est un ajustement qui minimise une différence entre les informations biologiques et une somme linéaire de la pluralité de fonctions sigmoïdes de distribution cumulative qui inclut des domaines de définition se chevauchant et satisfait à des conditions prédéterminées concernant des poids incluant au moins une condition selon laquelle au moins l'un des poids présente un signe qui est différent de celui des autres poids, lesdites fonctions sigmoïdes étant indépendantes les unes des autres.

**4.** Programme qui amène un ordinateur à fonctionner en tant que dispositif d'analyse de signal selon la revendication 1.

**5.** Dispositif d'analyse de signal (1) comportant :

une unité d'acquisition d'informations biologiques (11, 110) qui acquiert des informations biologiques chronologiques concernant la pulsation d'un cœur qui est une cible d'analyse ; et

une unité d'analyse (11, 130) qui acquiert des informations indiquant un état d'une activité d'un canal ionique dans le cœur sur la base des informations biologiques et d'informations de candidats de distribution en utilisant, en tant qu'informations de candidats de distribution, des informations indiquant des candidats pour la distribution temporelle d'activité de canal qui est la distribution de synchronisations de l'activité du canal ionique,

**caractérisé en ce que** les informations de candidats de distribution sont exprimées par une fonction de distribution cumulative utilisant une fonction représentant des candidats pour la distribution temporelle d'activité de canal en tant que fonction de densité de probabilité, dans lequel la fonction de distribution cumulative est une fonction de Gompertz ou une fonction de Gudermann.

**6.** Dispositif d'analyse de signal selon la revendication 5, dans lequel l'unité d'analyse (11, 130) acquiert des informations indiquant un état de l'activité du canal ionique sur la base d'un résultat d'ajustement d'une ou d'une pluralité des fonctions de distribution cumulative aux informations biologiques.

**7.** Dispositif d'analyse de signal selon la revendication 6, dans lequel l'ajustement est un ajustement qui minimise une différence entre les informations biologiques et une somme linéaire de la pluralité de fonctions de distribution cumulative qui inclut des domaines de définition se chevauchant et satisfait à des conditions prédéterminées concernant des poids incluant au moins une condition selon laquelle au moins l'un des poids présente un signe qui est différent de celui d'autres poids.

**8.** Procédé d'analyse de signal comportant :

une étape d'acquisition d'informations biologiques (S101) consistant à acquérir des informations biologiques chronologiques concernant la pulsation d'un cœur qui est une cible d'analyse ; et

une étape d'analyse (S104) consistant à acquérir des informations indiquant un état d'une activité d'un canal ionique sur la base des informations biologiques et d'informations de candidats de distribution en utilisant, en tant qu'informations de candidats de distribution, des informations indiquant des candidats pour la distribution temporelle d'activité de canal qui est la distribution de synchronisations de l'activité du canal ionique dans le cœur,

**caractérisé en ce que** les informations de candidats de distribution sont exprimées par une fonction de distribution cumulative utilisant une fonction représentant des candidats pour la distribution temporelle d'activité de canal en tant que fonction de densité de probabilité, dans lequel la fonction de distribution cumulative est une fonction de Gompertz ou une fonction de Gudermann.

**9.** Programme qui amène un ordinateur à fonctionner en tant que dispositif d'analyse de signal selon l'une quelconque des revendications 5 à 7.

FIG. 1

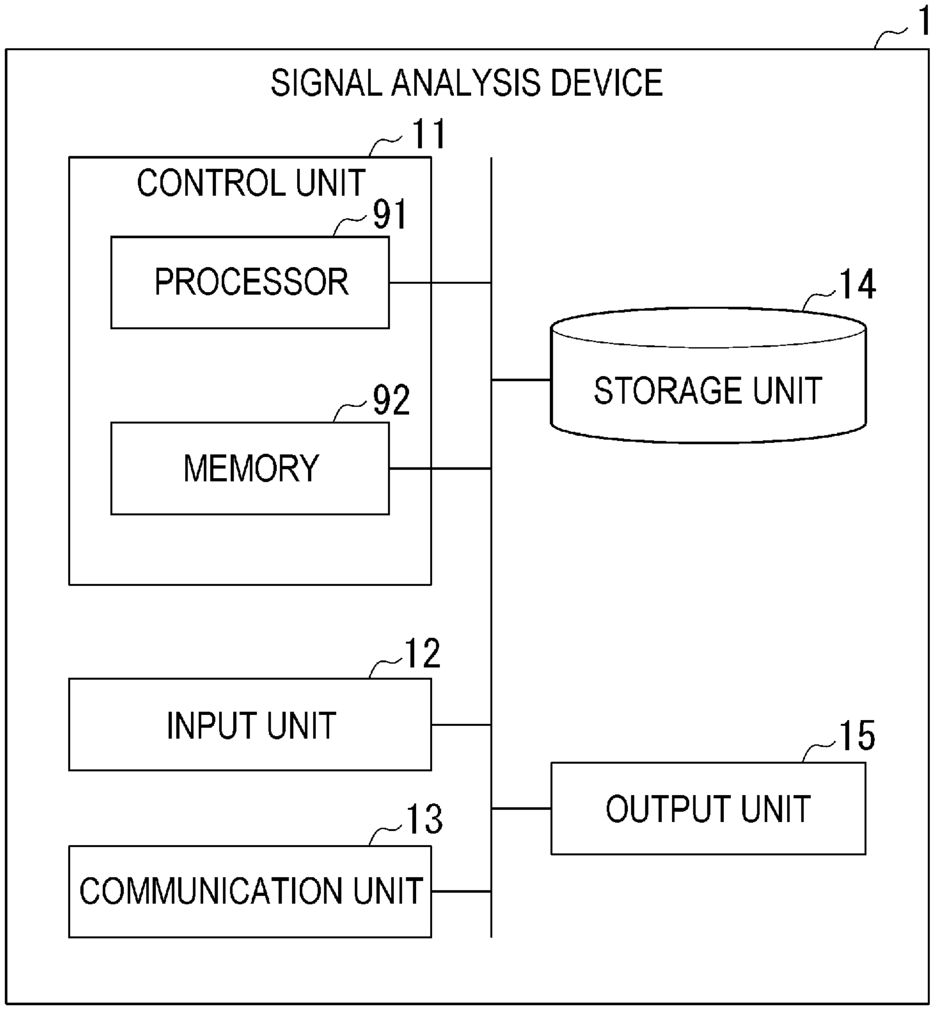
1
SIGNAL ANALYSIS DEVICE
11
CONTROL UNIT
91
PROCESSOR
92
MEMORY
14
STORAGE UNIT
12
INPUT UNIT
15
OUTPUT UNIT
13
COMMUNICATION UNIT

FIG. 2

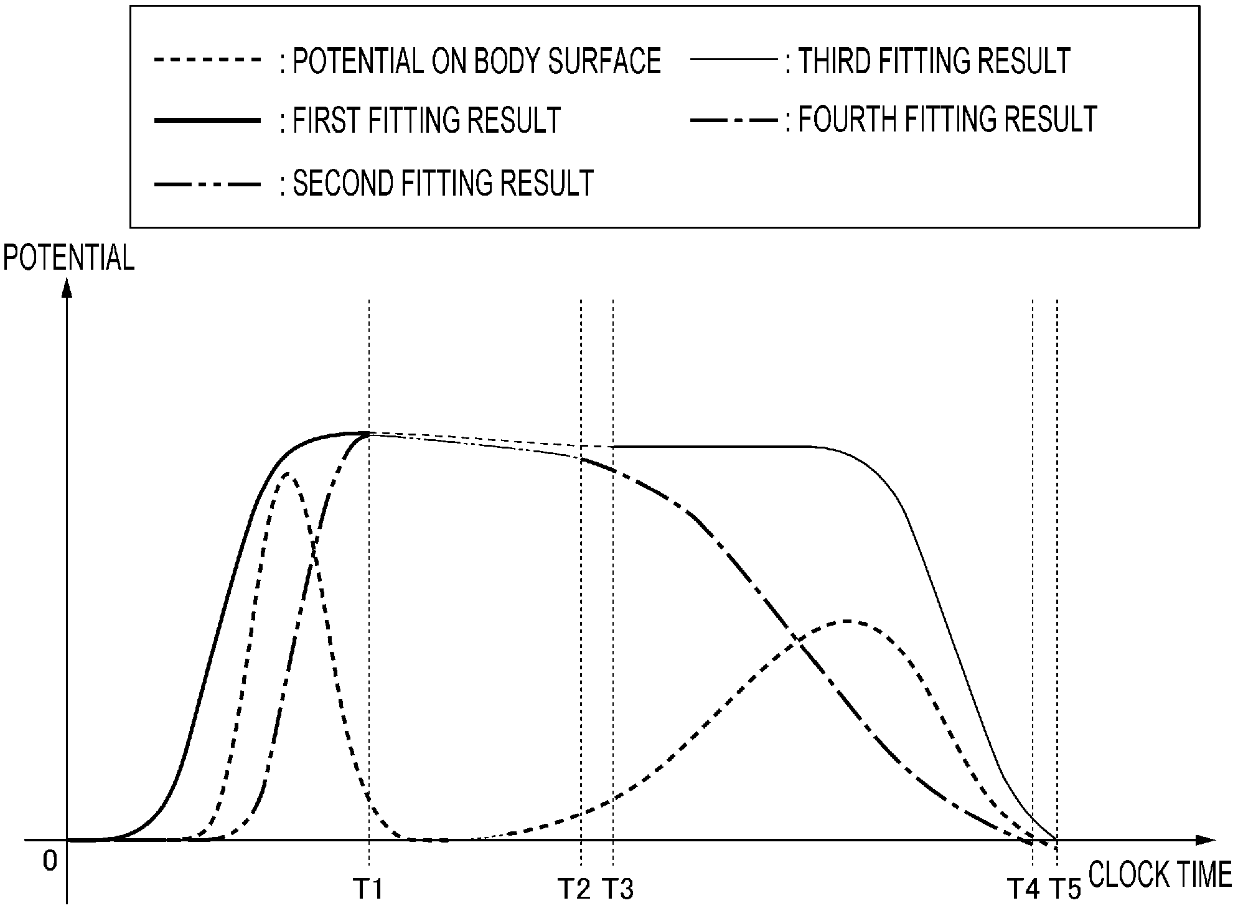
: POTENTIAL ON BODY SURFACE
: FIRST FITTING RESULT
: SECOND FITTING RESULT
: THIRD FITTING RESULT
: FOURTH FITTING RESULT
POTENTIAL
0
T1
T2
T3
T4
T5
CLOCK TIME

FIG. 3

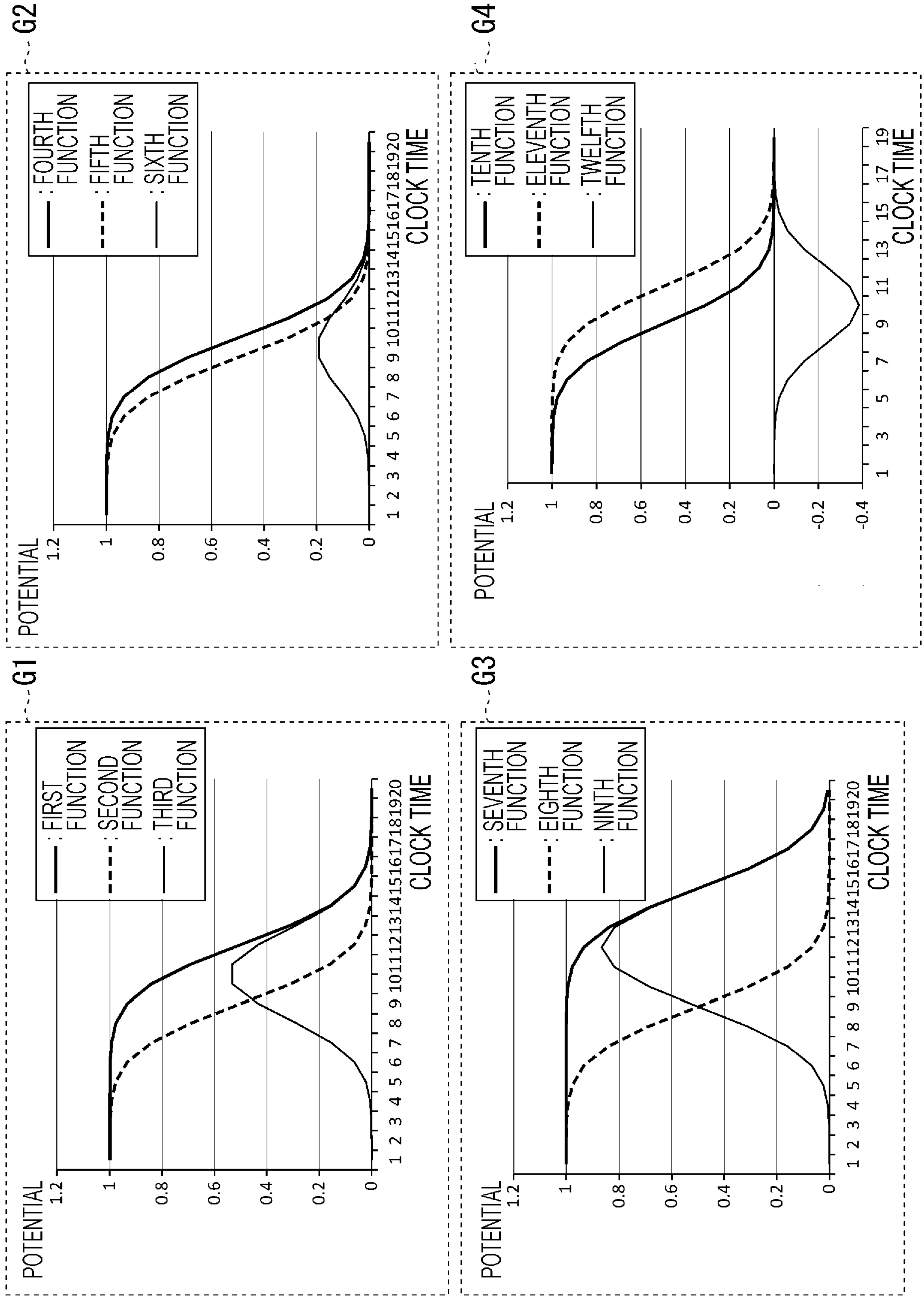
G1
POTENTIAL
CLOCK TIME
: FIRST FUNCTION
: SECOND FUNCTION
: THIRD FUNCTION
G2
: FOURTH FUNCTION
: FIFTH FUNCTION
: SIXTH FUNCTION
G3
: SEVENTH FUNCTION
: EIGHTH FUNCTION
: NINTH FUNCTION
G4
: TENTH FUNCTION
: ELEVENTH FUNCTION
: TWELFTH FUNCTION

FIG. 4

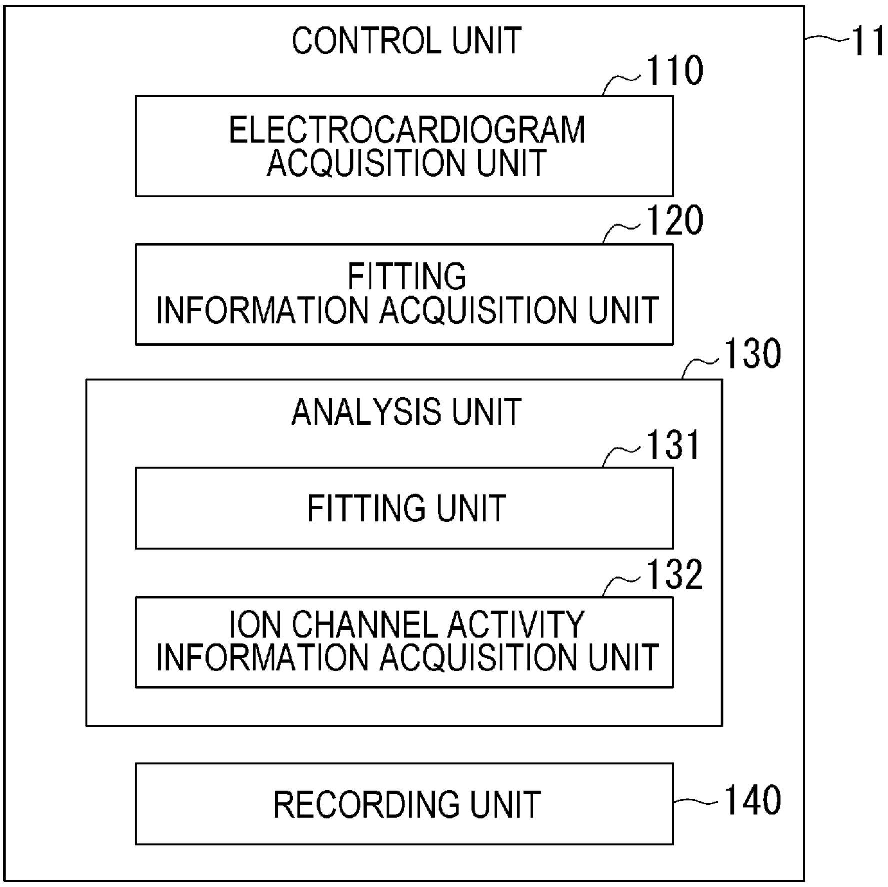
CONTROL UNIT
11
110
ELECTROCARDIOGRAM ACQUISITION UNIT
120
FITTING INFORMATION ACQUISITION UNIT
130
ANALYSIS UNIT
131
FITTING UNIT
132
ION CHANNEL ACTIVITY INFORMATION ACQUISITION UNIT
RECORDING UNIT
140

FIG. 5

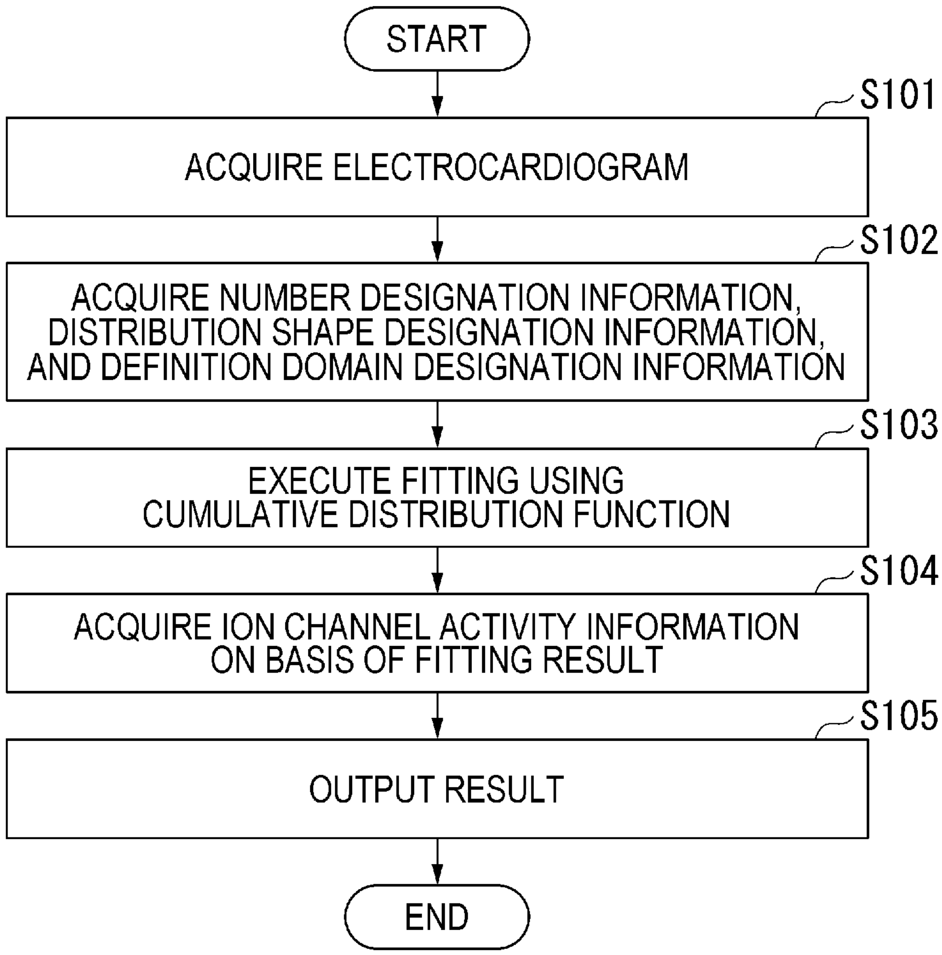
START
S101
ACQUIRE ELECTROCARDIOGRAM
S102
ACQUIRE NUMBER DESIGNATION INFORMATION, DISTRIBUTION SHAPE DESIGNATION INFORMATION, AND DEFINITION DOMAIN DESIGNATION INFORMATION
S103
EXECUTE FITTING USING CUMULATIVE DISTRIBUTION FUNCTION
S104
ACQUIRE ION CHANNEL ACTIVITY INFORMATION ON BASIS OF FITTING RESULT
S105
OUTPUT RESULT
END

FIG. 6

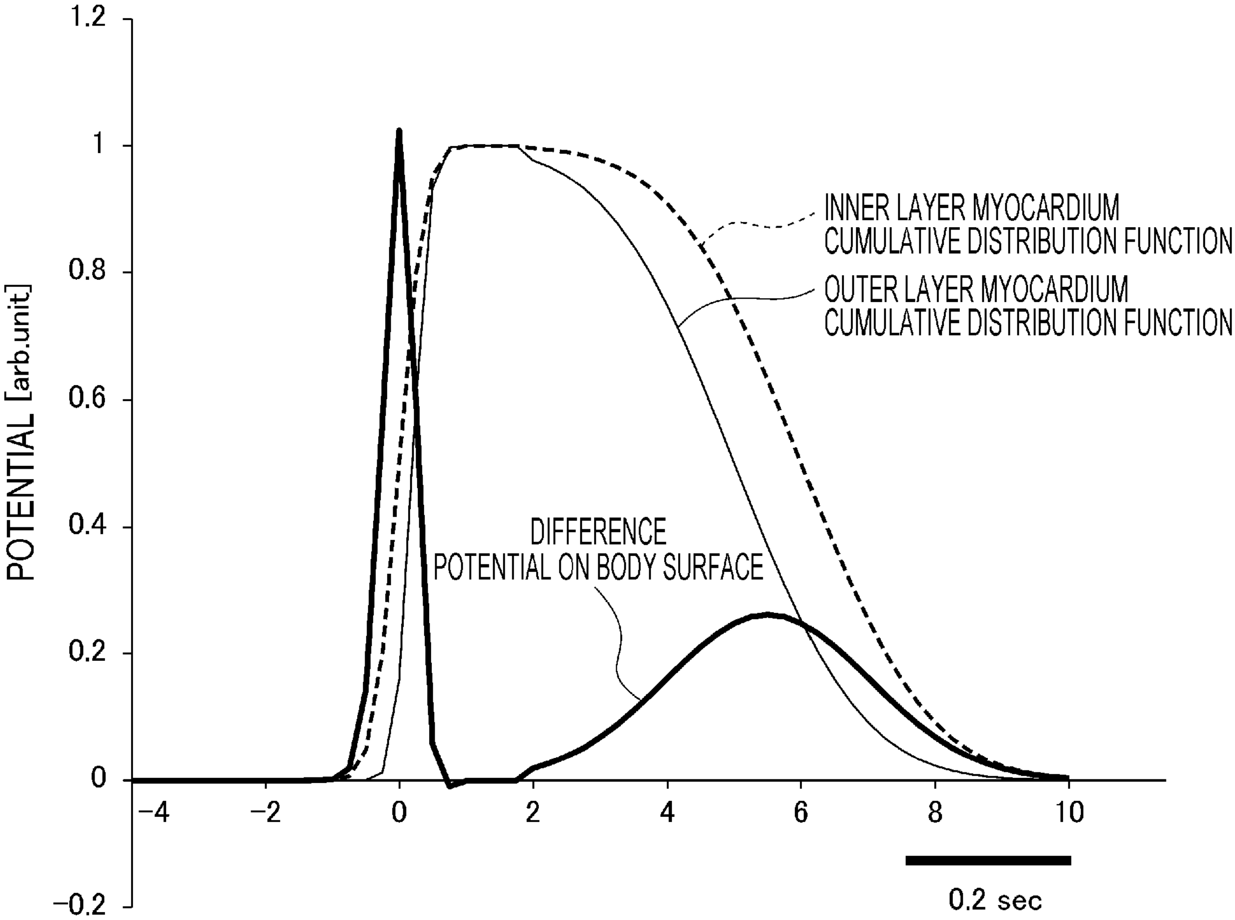
1.2
1
0.8
0.6
0.4
0.2
0
−0.2
POTENTIAL [arb.unit]
−4
−2
0
2
4
6
8
10
INNER LAYER MYOCARDIUM
CUMULATIVE DISTRIBUTION FUNCTION
OUTER LAYER MYOCARDIUM
CUMULATIVE DISTRIBUTION FUNCTION
DIFFERENCE
POTENTIAL ON BODY SURFACE
0.2 sec

FIG. 7

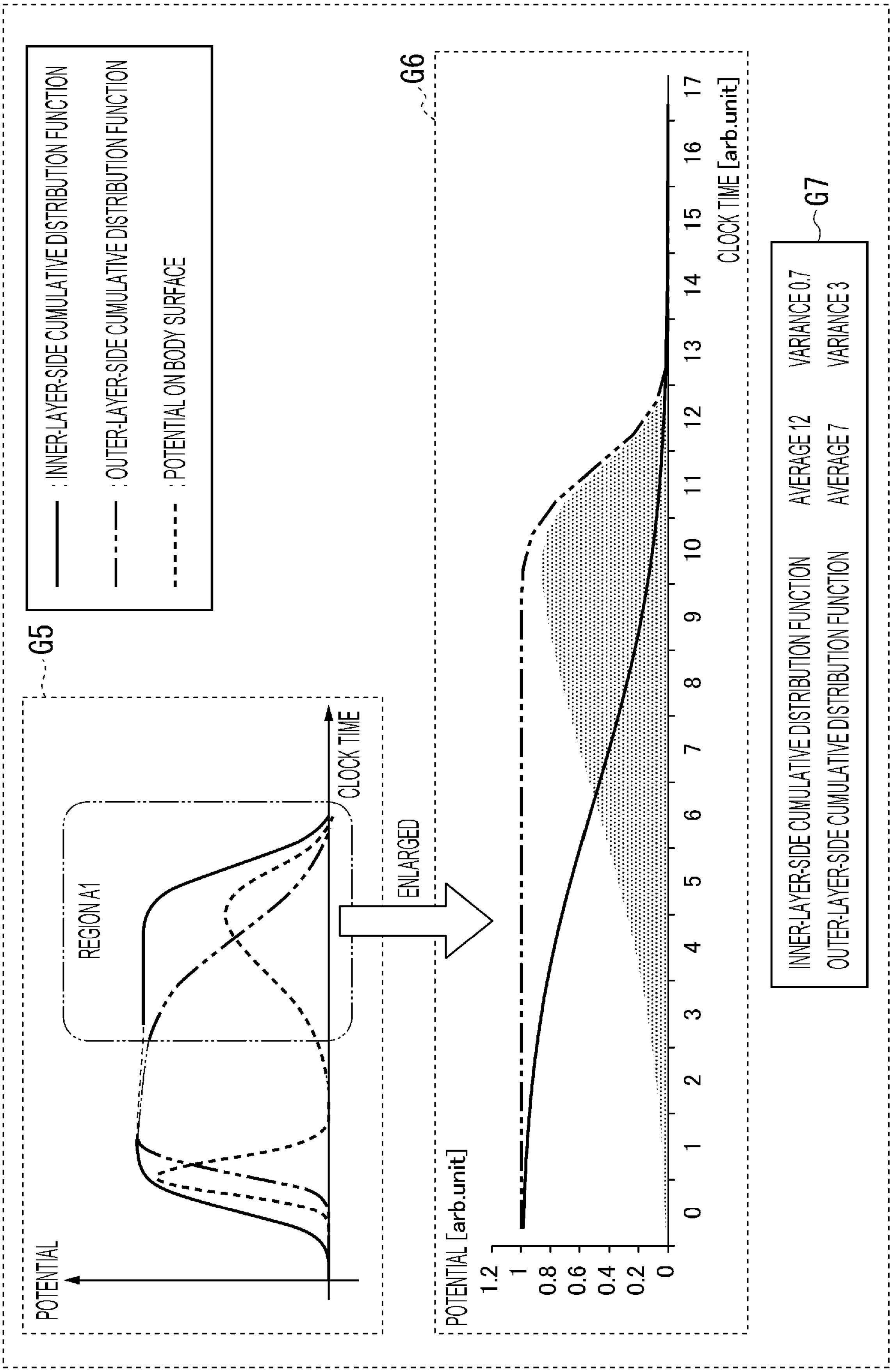
: INNER-LAYER-SIDE CUMULATIVE DISTRIBUTION FUNCTION
: OUTER-LAYER-SIDE CUMULATIVE DISTRIBUTION FUNCTION
: POTENTIAL ON BODY SURFACE
G5
POTENTIAL
CLOCK TIME
REGION A1
ENLARGED
G6
POTENTIAL [arb.unit]
1.2
1
0.8
0.6
0.4
0.2
0
0 1 2 3 4 5 6 7 8 9 10 11 12 13 14 15 16 17
CLOCK TIME [arb.unit]
G7
INNER-LAYER-SIDE CUMULATIVE DISTRIBUTION FUNCTION AVERAGE 12 VARIANCE 0.7
OUTER-LAYER-SIDE CUMULATIVE DISTRIBUTION FUNCTION AVERAGE 7 VARIANCE 3

FIG. 8

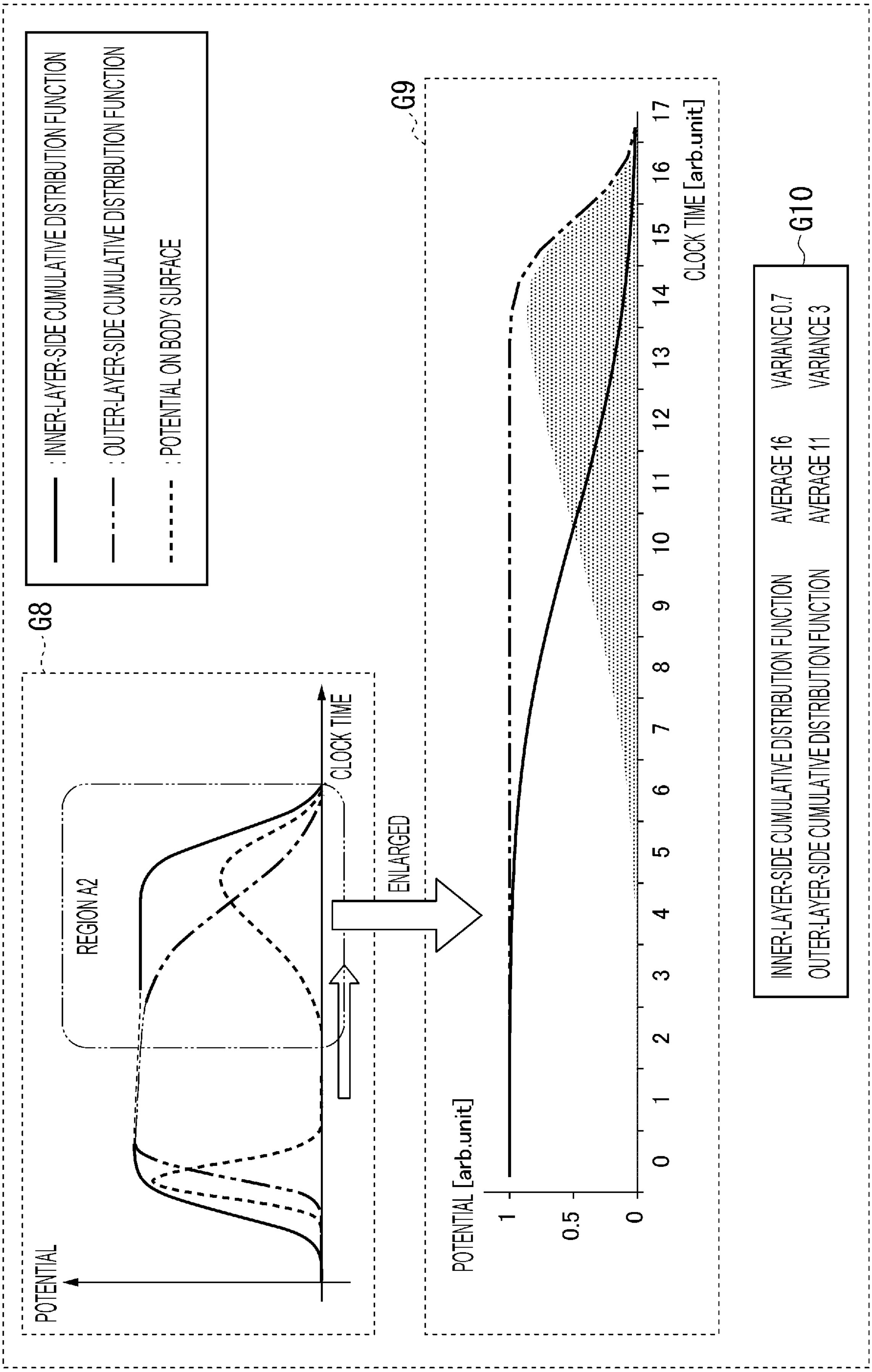
: INNER-LAYER-SIDE CUMULATIVE DISTRIBUTION FUNCTION
: OUTER-LAYER-SIDE CUMULATIVE DISTRIBUTION FUNCTION
: POTENTIAL ON BODY SURFACE
G8
POTENTIAL
CLOCK TIME
REGION A2
ENLARGED
G9
POTENTIAL [arb.unit]
1
0.5
0
0
1
2
3
4
5
6
7
8
9
10
11
12
13
14
15
16
17
CLOCK TIME [arb.unit]
G10
INNER-LAYER-SIDE CUMULATIVE DISTRIBUTION FUNCTION AVERAGE 16 VARIANCE 0.7
OUTER-LAYER-SIDE CUMULATIVE DISTRIBUTION FUNCTION AVERAGE 11 VARIANCE 3

FIG. 9

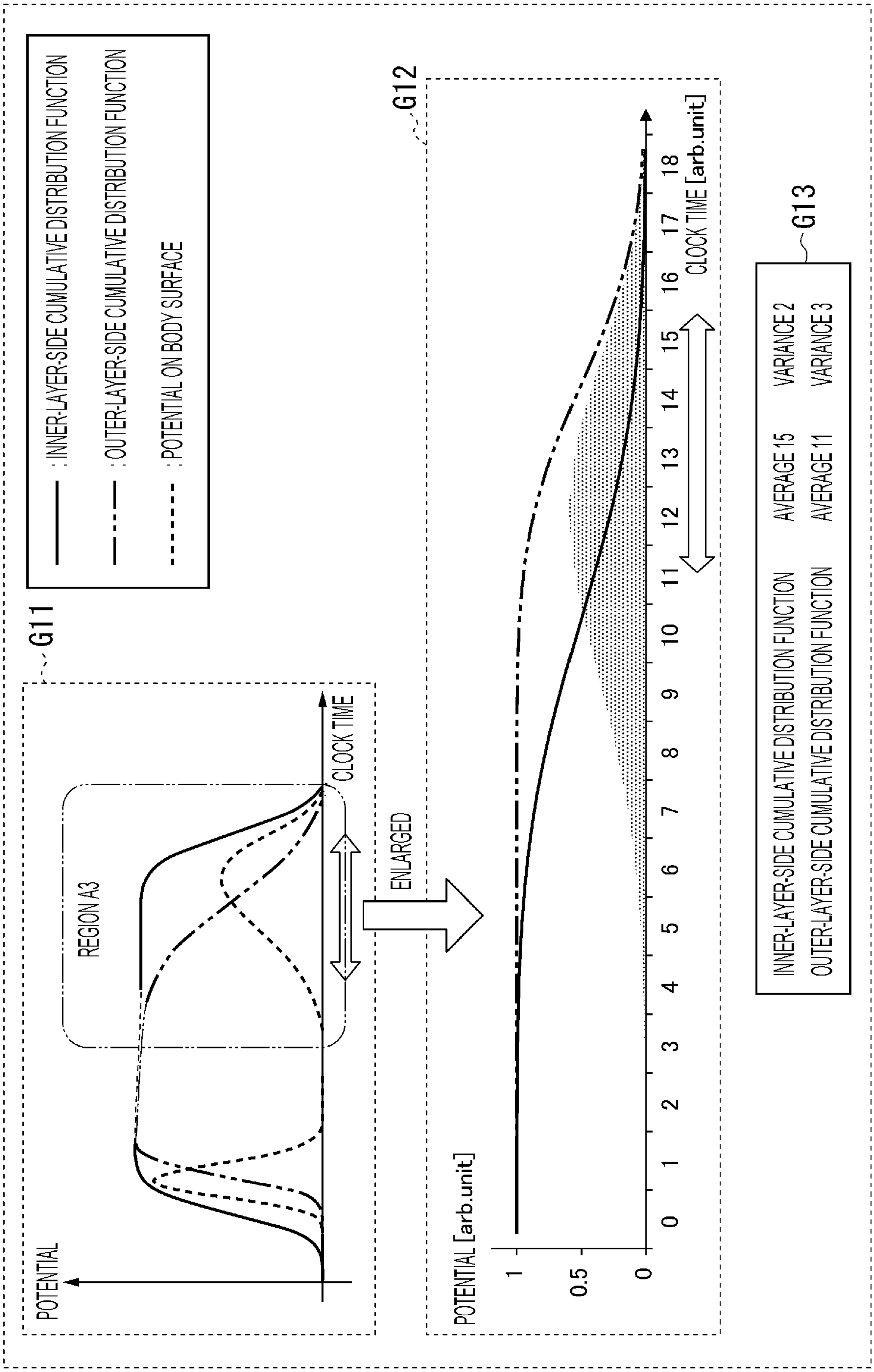
: INNER-LAYER-SIDE CUMULATIVE DISTRIBUTION FUNCTION
: OUTER-LAYER-SIDE CUMULATIVE DISTRIBUTION FUNCTION
: POTENTIAL ON BODY SURFACE
G11
POTENTIAL
CLOCK TIME
REGION A3
ENLARGED
G12
POTENTIAL [arb.unit]
1
0.5
0
0 1 2 3 4 5 6 7 8 9 10 11 12 13 14 15 16 17 18
CLOCK TIME [arb.unit]
G13
INNER-LAYER-SIDE CUMULATIVE DISTRIBUTION FUNCTION AVERAGE 15 VARIANCE 2
OUTER-LAYER-SIDE CUMULATIVE DISTRIBUTION FUNCTION AVERAGE 11 VARIANCE 3

FIG. 10

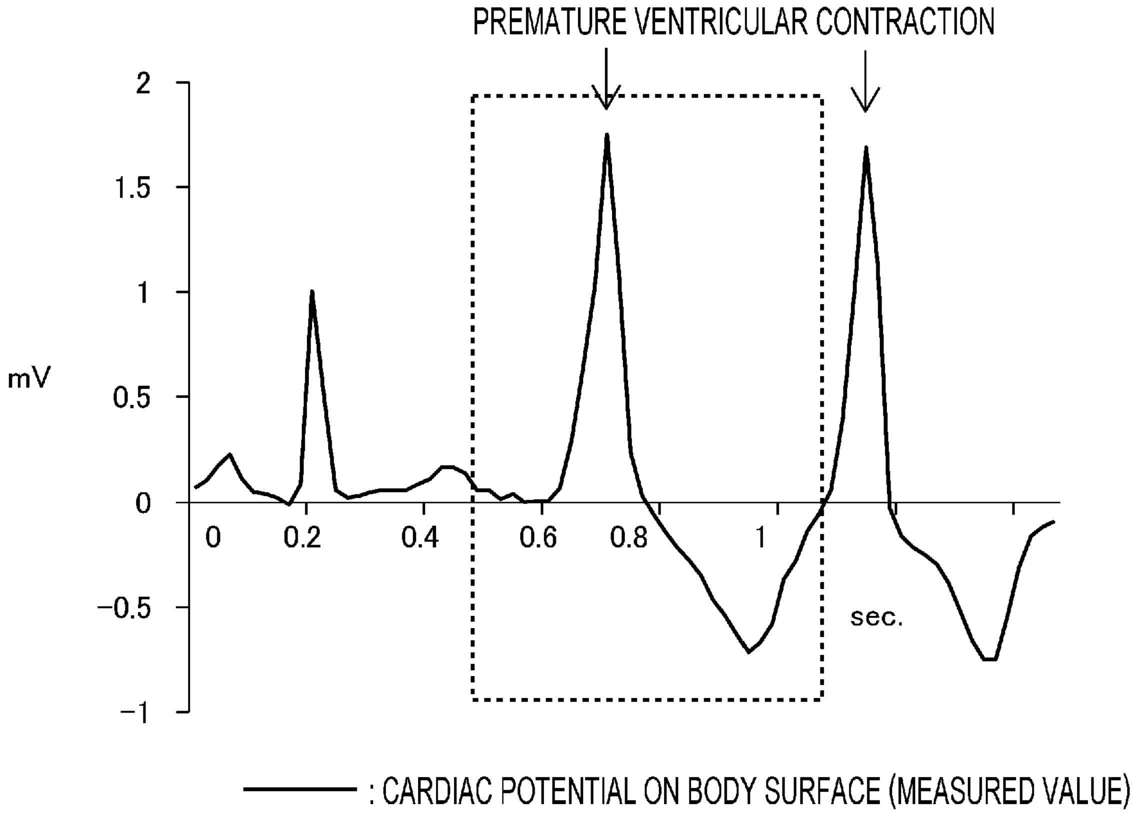
PREMATURE VENTRICULAR CONTRACTION
2
1.5
1
mV
0.5
0
−0.5
−1
0
0.2
0.4
0.6
0.8
1
sec.
: CARDIAC POTENTIAL ON BODY SURFACE (MEASURED VALUE)

FIG. 11

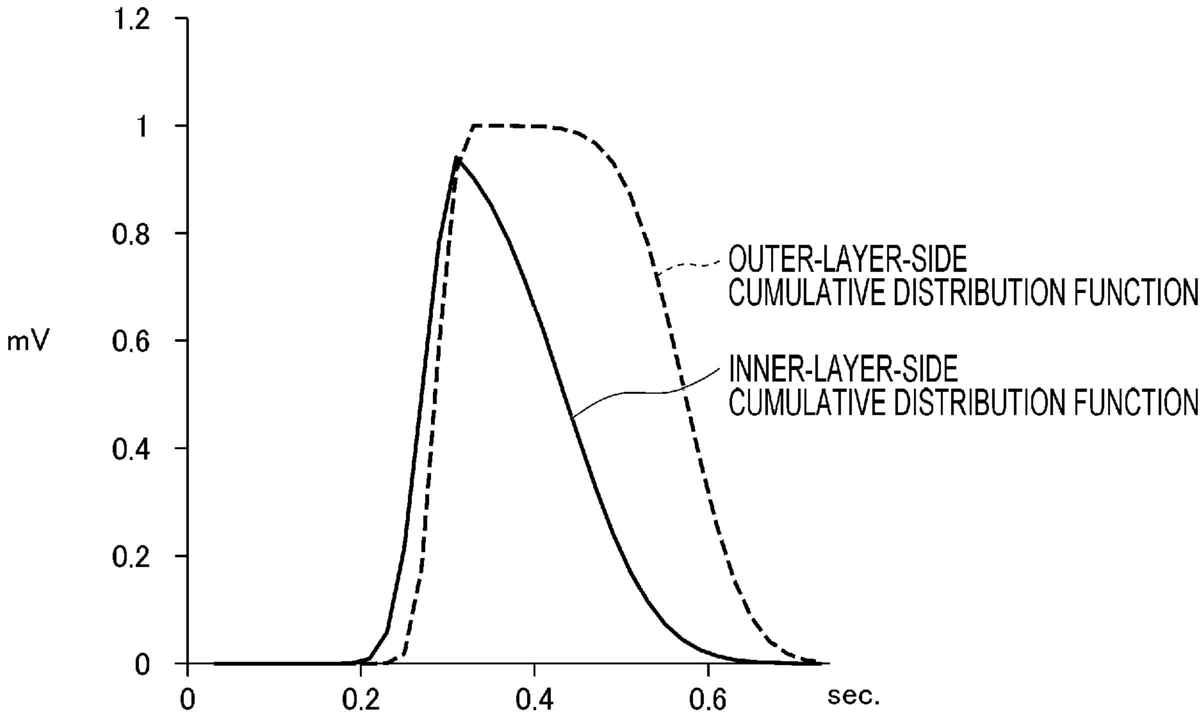
1.2
1
0.8
mV
0.6
0.4
0.2
0
0
0.2
0.4
0.6
sec.
OUTER-LAYER-SIDE
CUMULATIVE DISTRIBUTION FUNCTION
INNER-LAYER-SIDE
CUMULATIVE DISTRIBUTION FUNCTION

FIG. 12

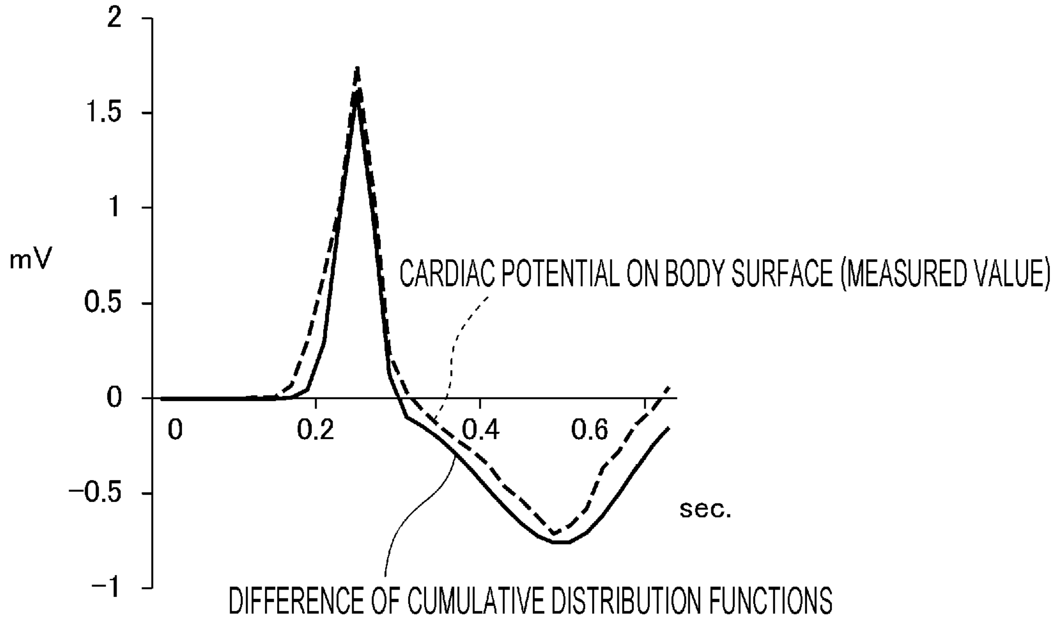
2
1.5
1
mV
0.5
0
−0.5
−1
0
0.2
0.4
0.6
sec.
CARDIAC POTENTIAL ON BODY SURFACE (MEASURED VALUE)
DIFFERENCE OF CUMULATIVE DISTRIBUTION FUNCTIONS

FIG. 13

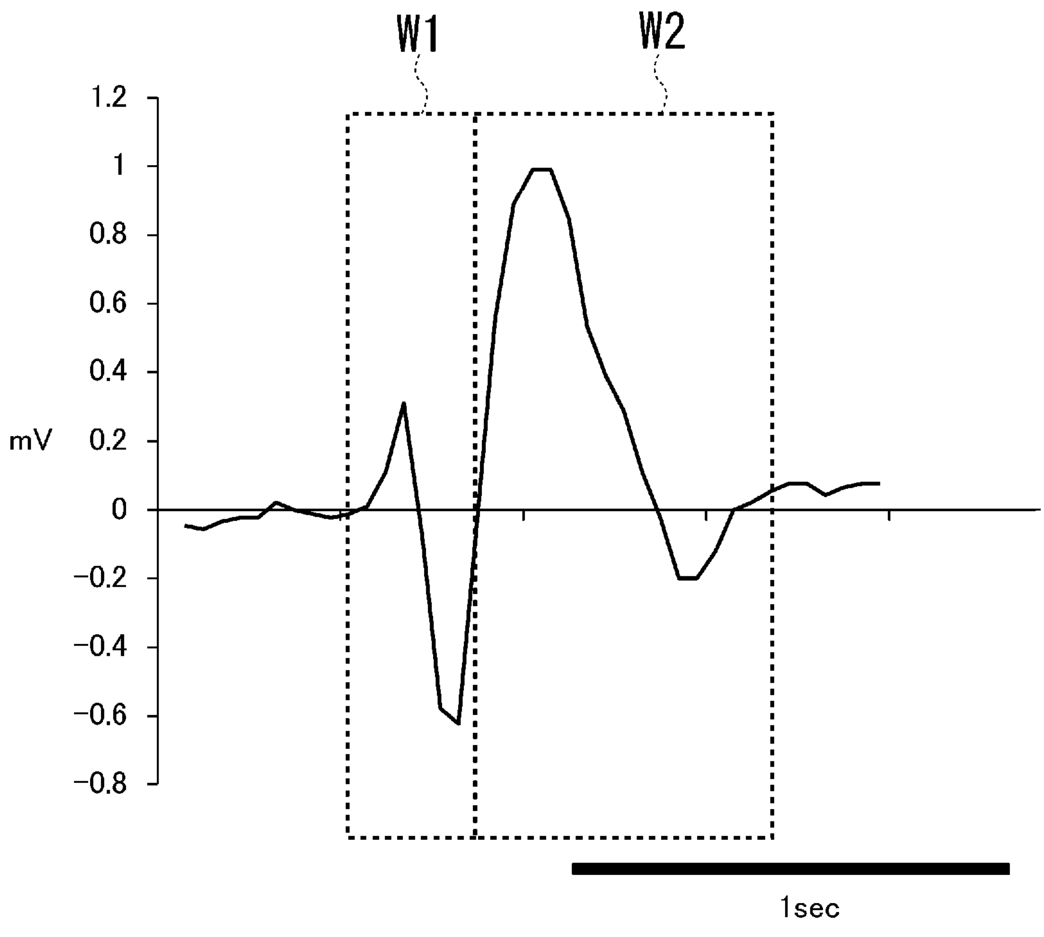

FIG. 14

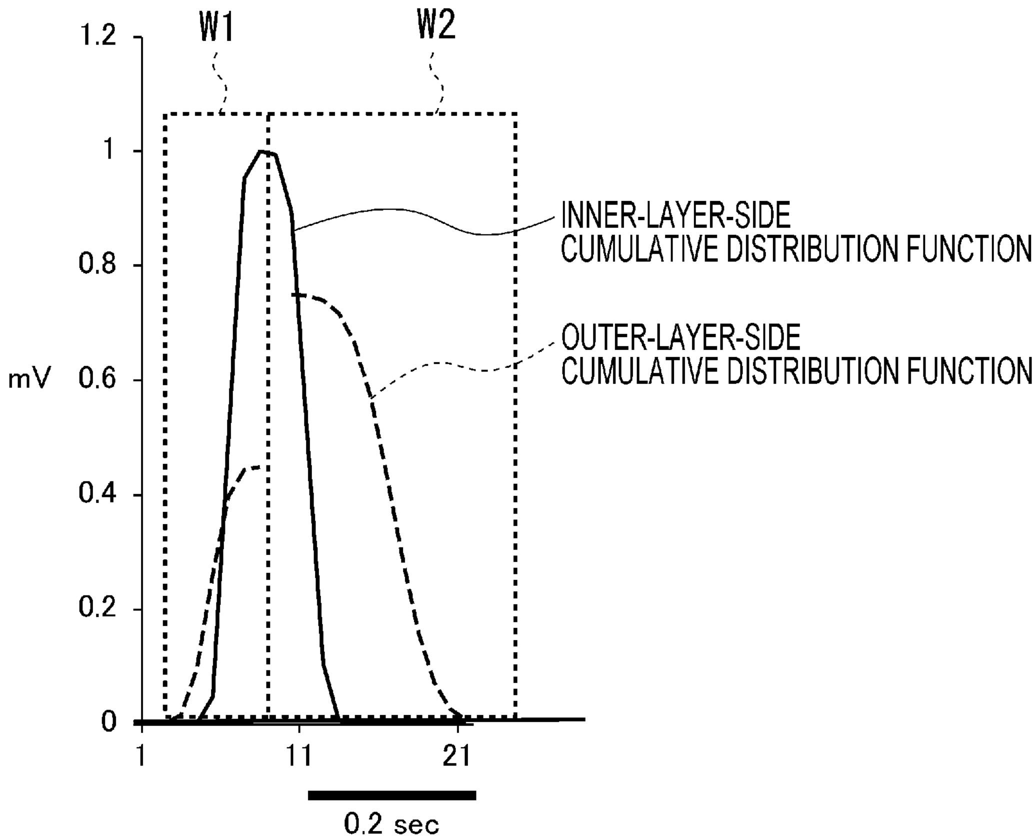
1.2
1
0.8
mV 0.6
0.4
0.2
0
W1
W2
INNER-LAYER-SIDE
CUMULATIVE DISTRIBUTION FUNCTION
OUTER-LAYER-SIDE
CUMULATIVE DISTRIBUTION FUNCTION
1
11
21
0.2 sec

FIG. 15

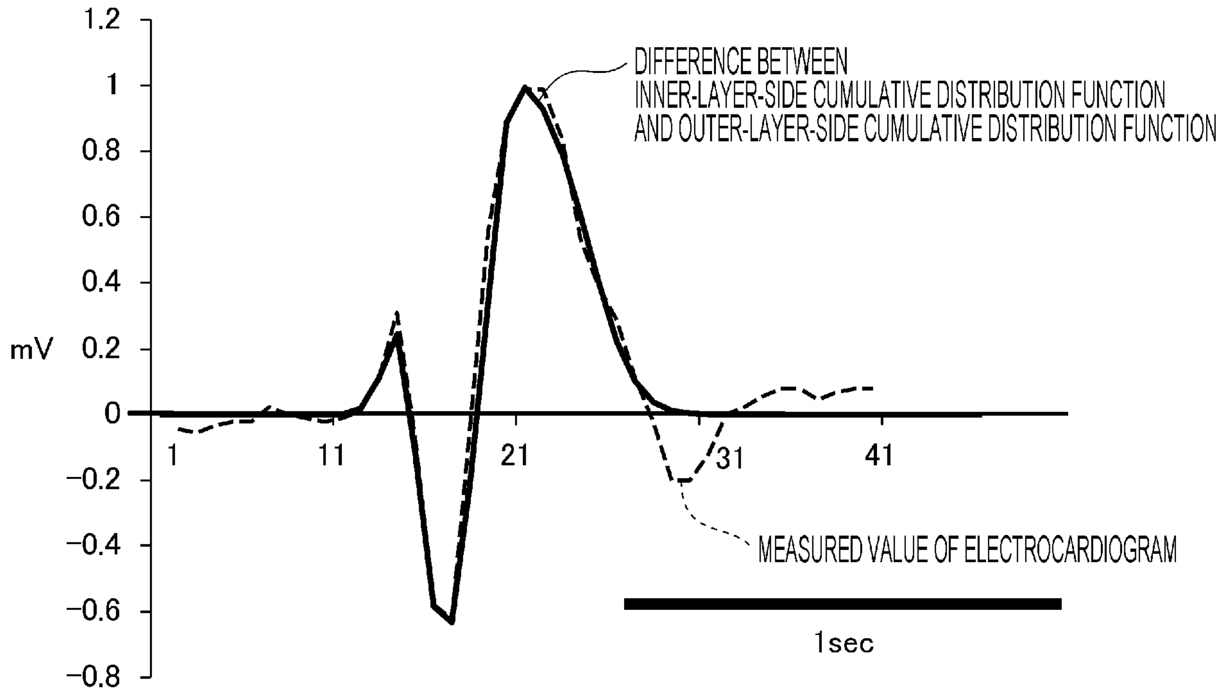
1.2
1
0.8
0.6
0.4
mV
0.2
0
−0.2
−0.4
−0.6
−0.8
1
11
21
31
41
DIFFERENCE BETWEEN
INNER-LAYER-SIDE CUMULATIVE DISTRIBUTION FUNCTION
AND OUTER-LAYER-SIDE CUMULATIVE DISTRIBUTION FUNCTION
MEASURED VALUE OF ELECTROCARDIOGRAM
1sec

FIG. 16

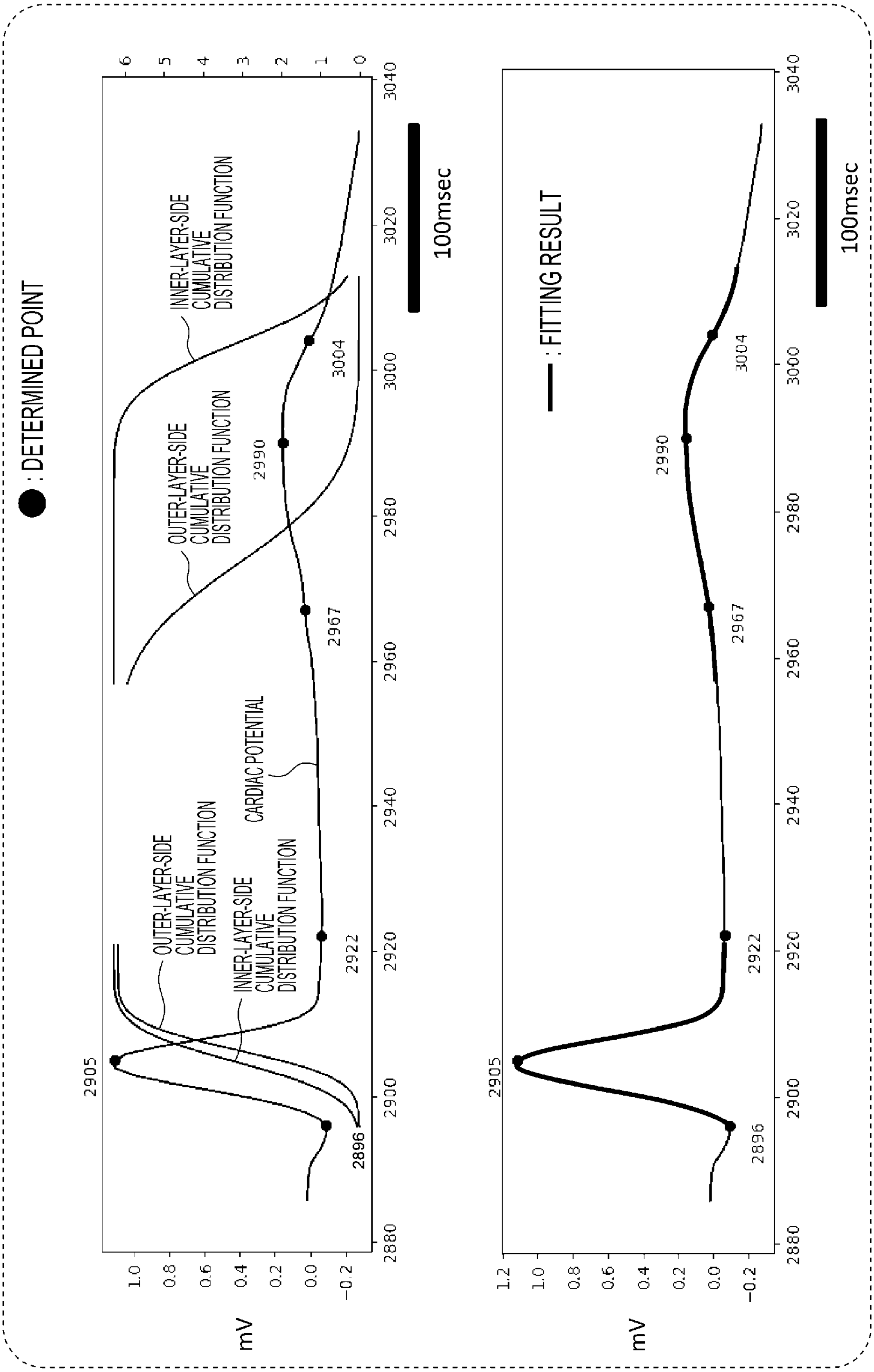
: DETERMINED POINT
OUTER-LAYER-SIDE CUMULATIVE DISTRIBUTION FUNCTION
INNER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
2896
2905
2922
2967
2990
3004
mV
100msec
: FITTING RESULT
2896
2905
2922
2967
2990
3004
mV
100msec

FIG. 17

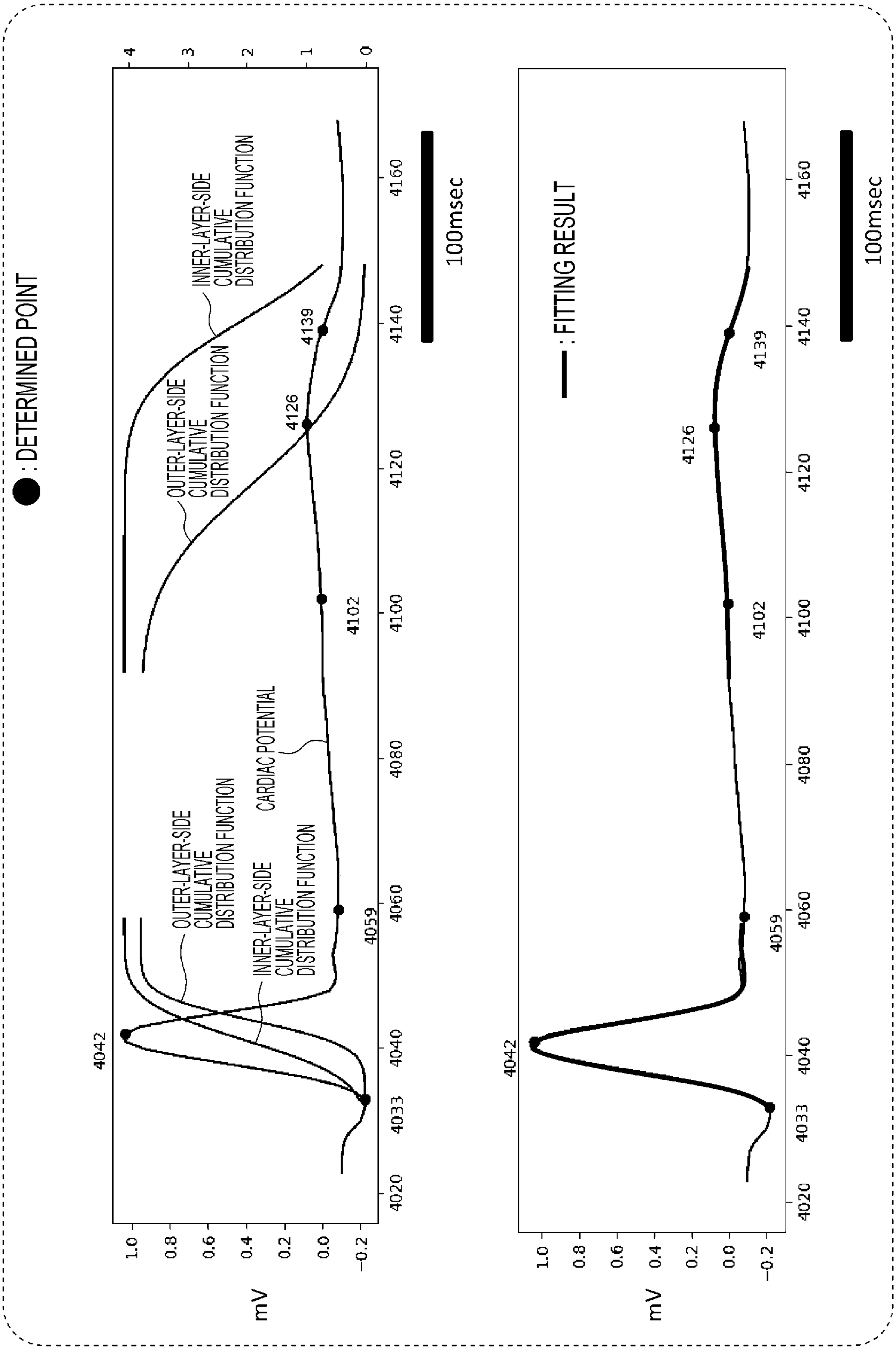
●: DETERMINED POINT
OUTER-LAYER-SIDE CUMULATIVE DISTRIBUTION FUNCTION
INNER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
4033
4042
4059
4102
4126
4139
mV
100msec
▬: FITTING RESULT
4033
4042
4059
4102
4126
4139
mV
100msec

FIG. 18

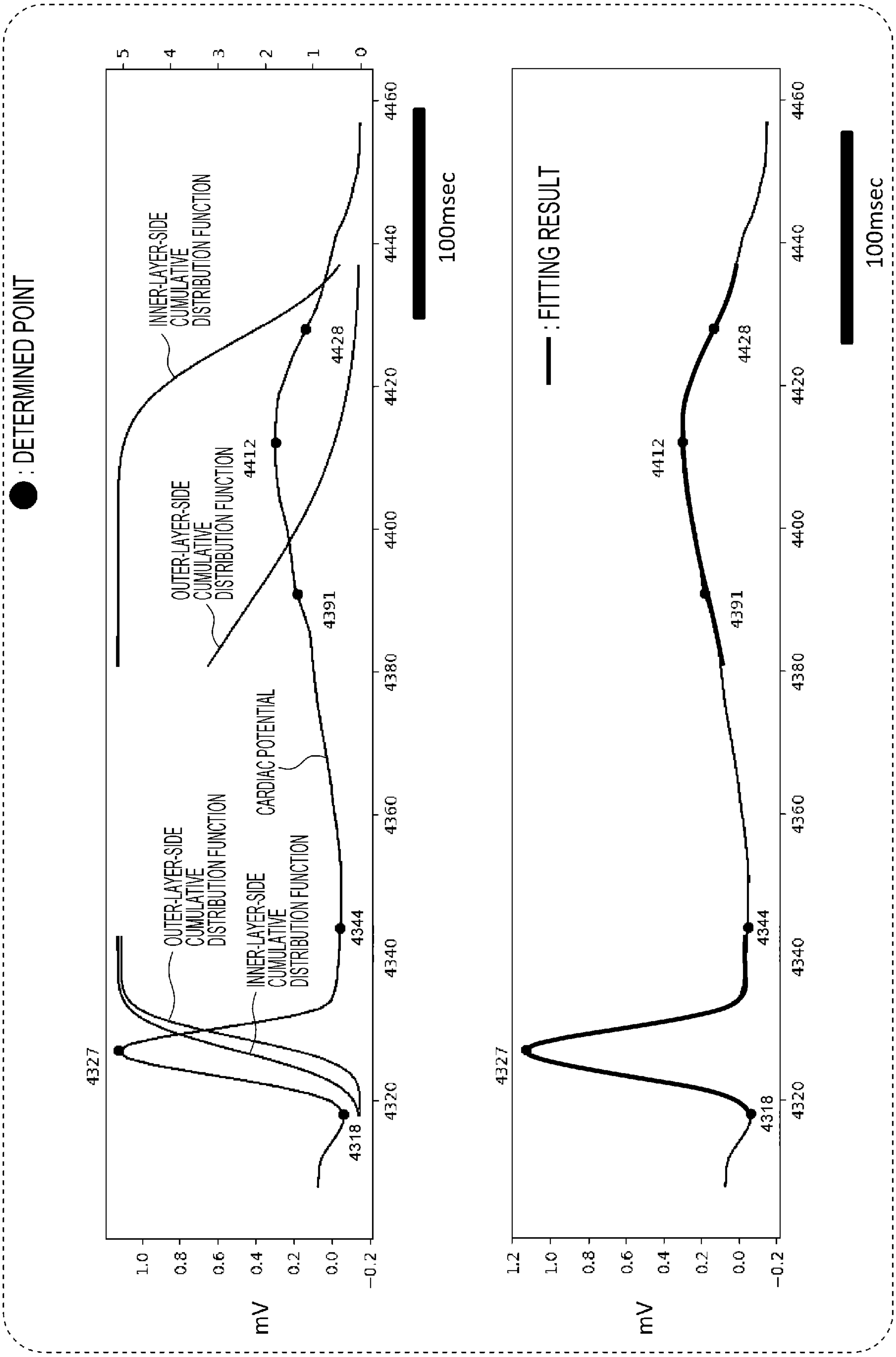
: DETERMINED POINT
mV
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
4318
4327
4344
4391
4412
4428
100msec
: FITTING RESULT

FIG. 19

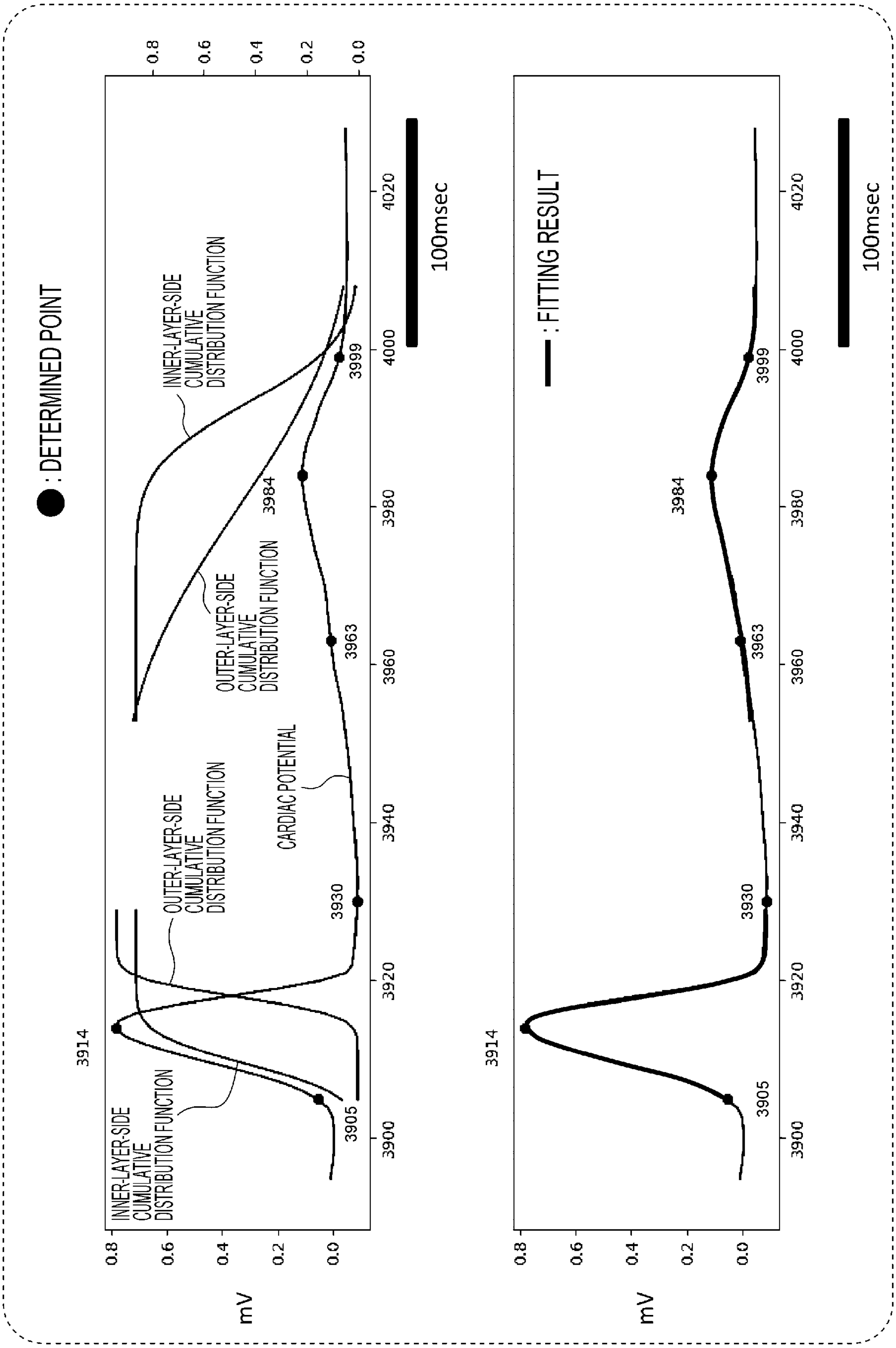
: DETERMINED POINT
mV
0.8
0.6
0.4
0.2
0.0
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
3905
3914
3930
3963
3984
3999
3900
3920
3940
3960
3980
4000
4020
100msec
: FITTING RESULT

FIG. 20

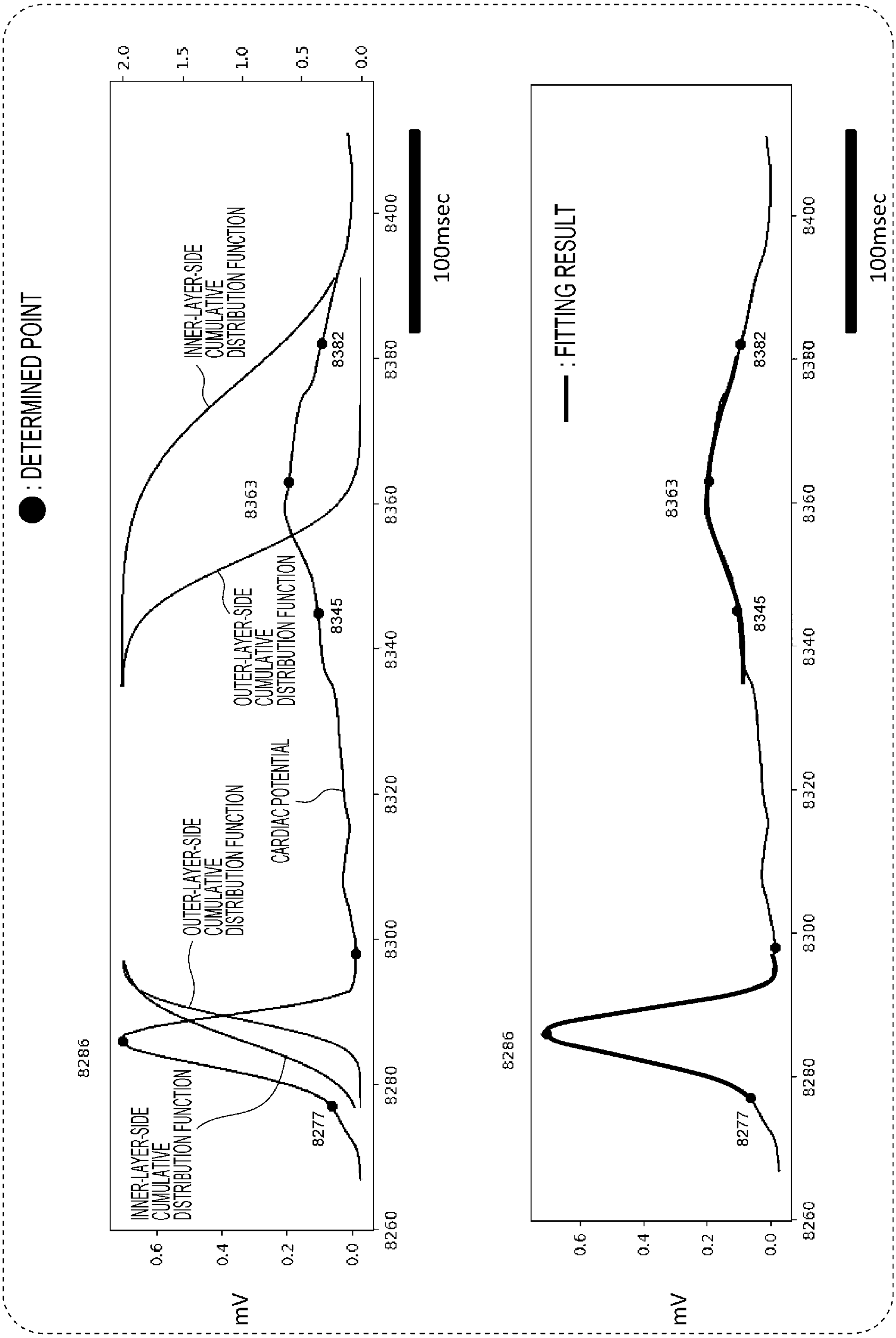
: DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
8277
8286
8345
8363
8382
mV
100msec
: FITTING RESULT
8277
8286
8345
8363
8382
mV
100msec

FIG. 21

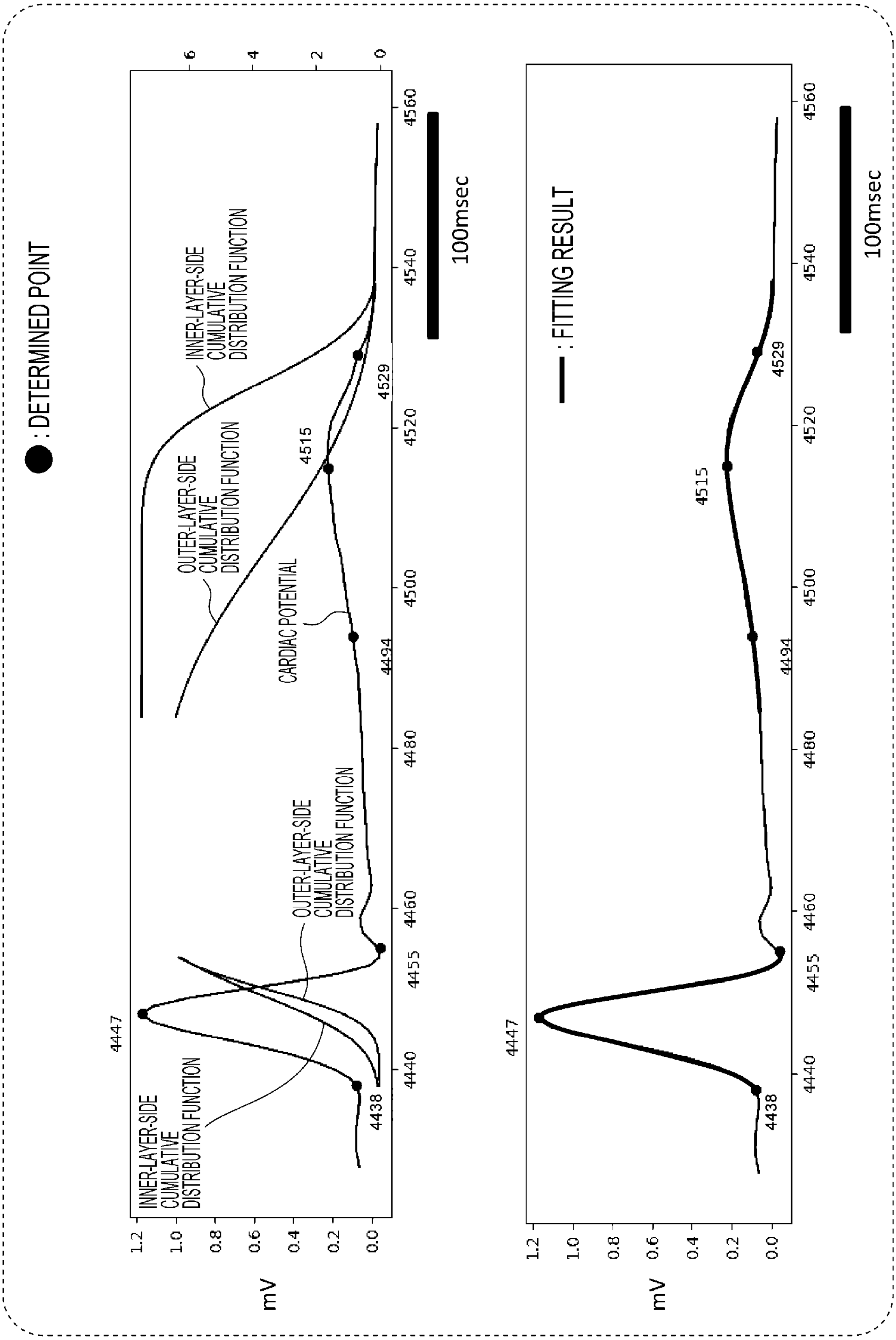

: DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
4438
4447
4455
4494
4515
4529
100msec
mV
: FITTING RESULT

FIG. 22

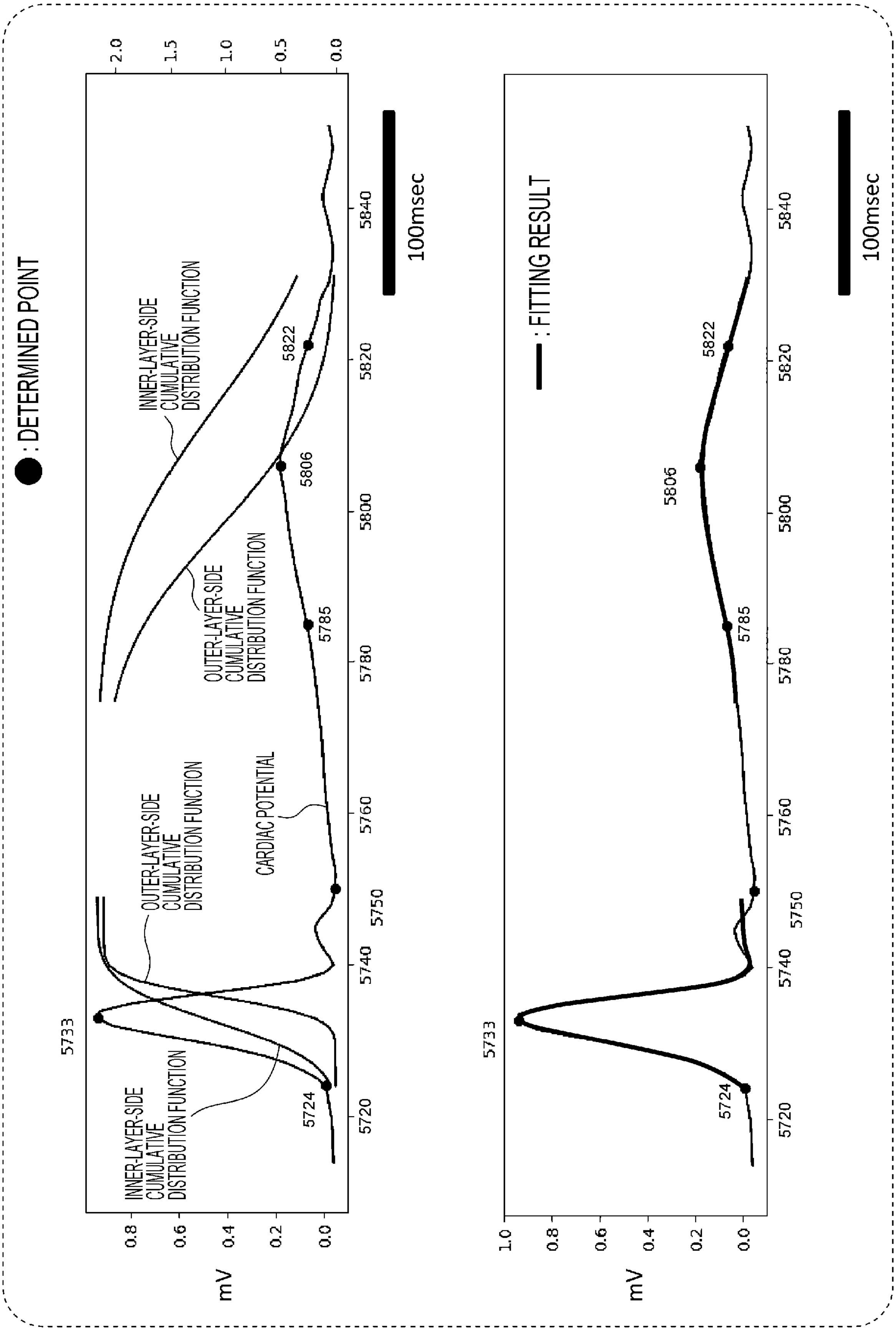

● : DETERMINED POINT
mV
0.8
0.6
0.4
0.2
0.0
2.0
1.5
1.0
0.5
0.0
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
5724
5733
5750
5785
5806
5822
5720
5740
5760
5780
5800
5820
5840
100msec
— : FITTING RESULT
1.0

FIG. 23

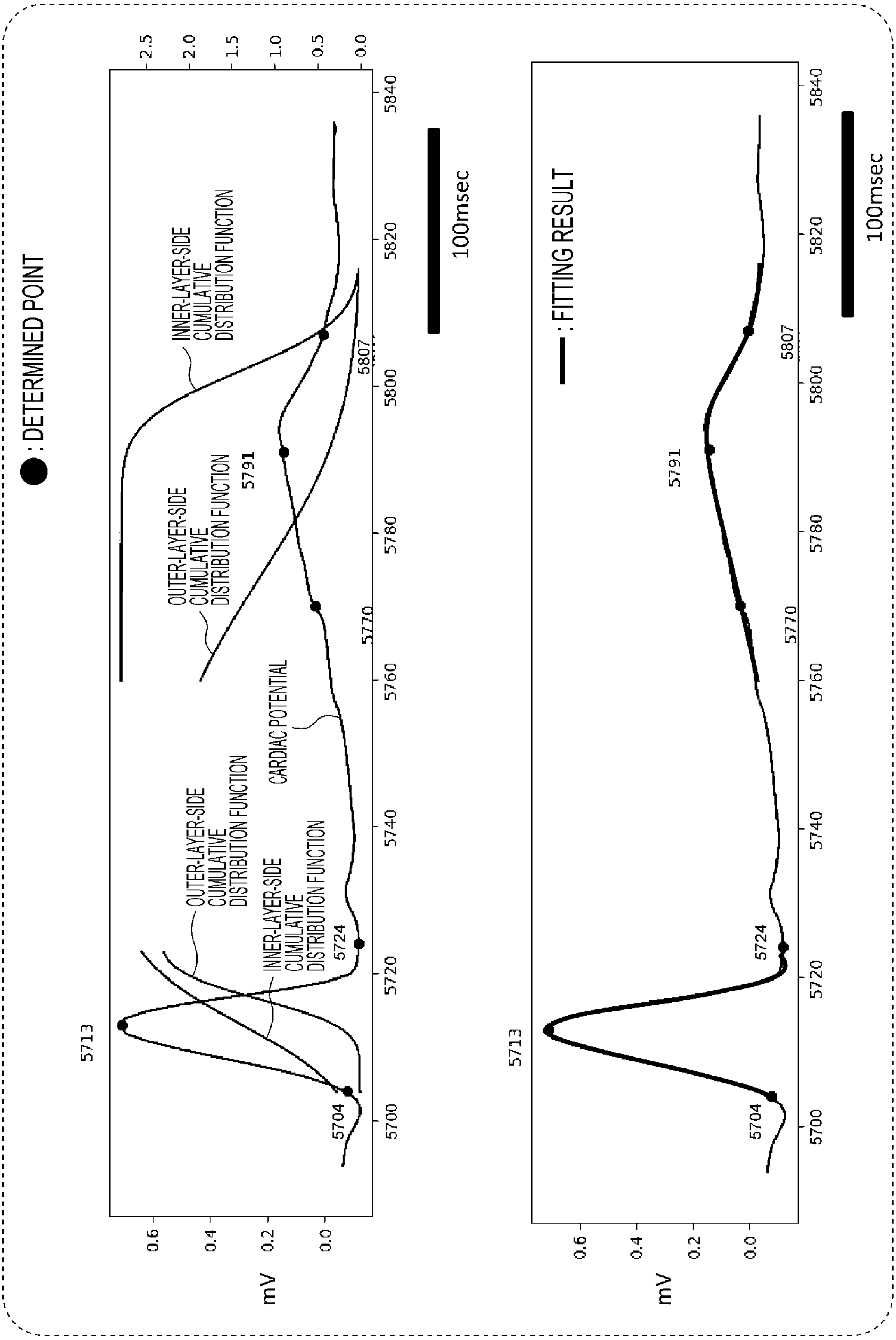
●: DETERMINED POINT
mV
0.6
0.4
0.2
0.0
2.5
2.0
1.5
1.0
0.5
0.0
5700
5720
5740
5760
5780
5800
5820
5840
5704
5713
5724
5770
5791
5807
OUTER-LAYER-SIDE CUMULATIVE DISTRIBUTION FUNCTION
INNER-LAYER-SIDE CUMULATIVE DISTRIBUTION FUNCTION
CARDIAC POTENTIAL
100msec
—: FITTING RESULT

FIG. 24

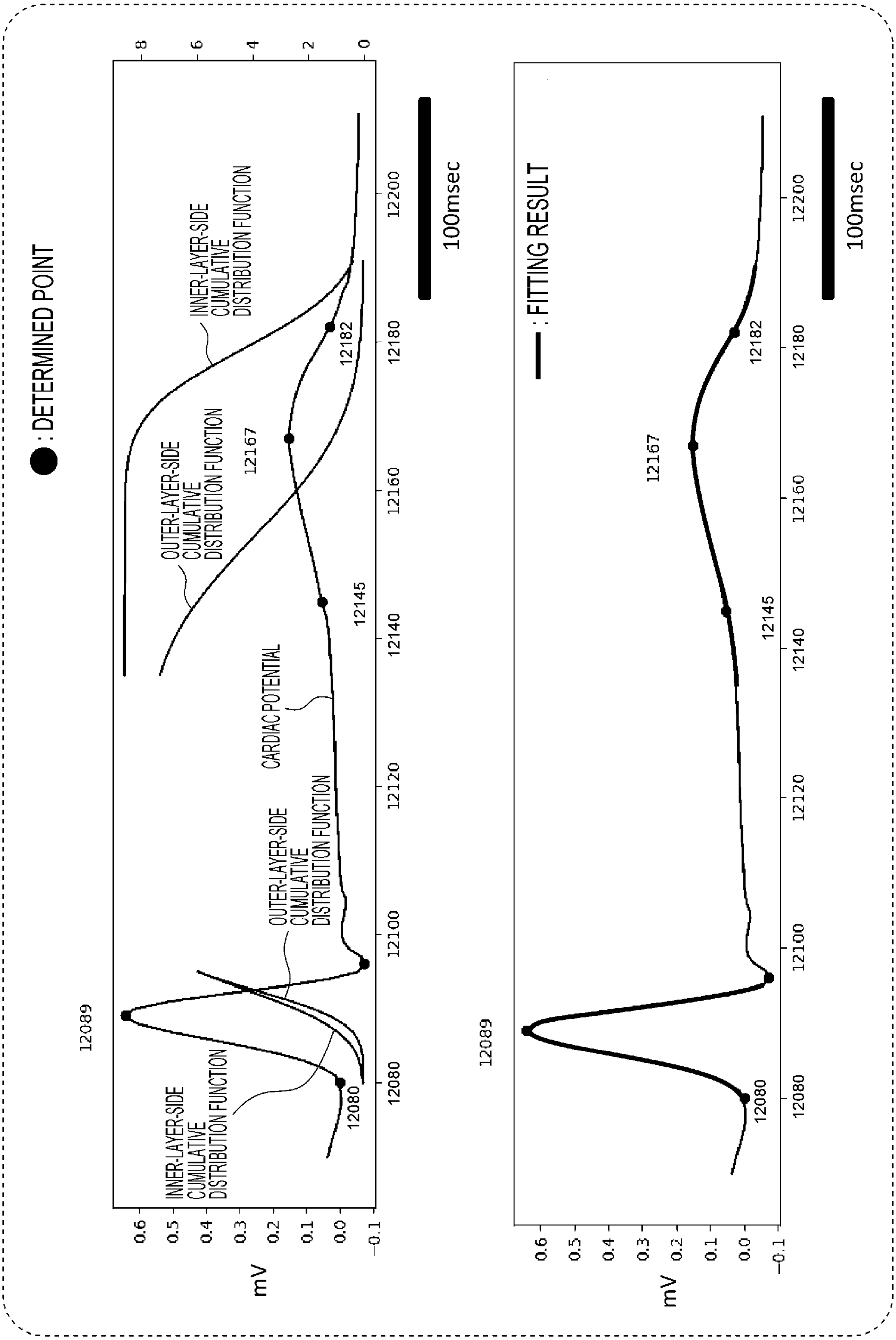
: DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
12080
12089
12145
12167
12182
mV
0.6
0.5
0.4
0.3
0.2
0.1
0.0
−0.1
12080
12100
12120
12140
12160
12180
12200
8
6
4
2
0
100msec
: FITTING RESULT
12080
12089
12145
12167
12182
mV
0.6
0.5
0.4
0.3
0.2
0.1
0.0
−0.1
12080
12100
12120
12140
12160
12180
12200
100msec

FIG. 25

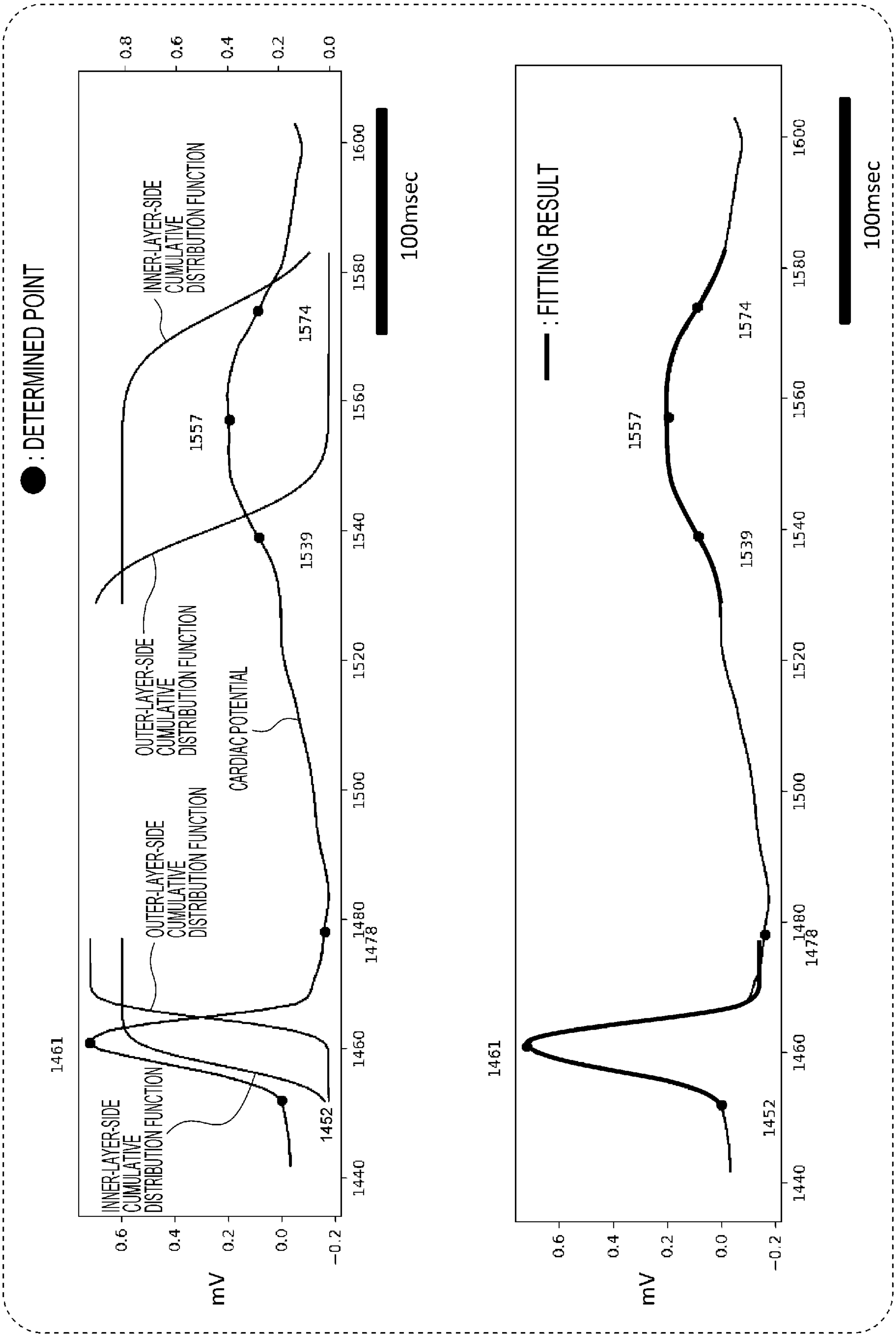
● : DETERMINED POINT
INNER-LAYER-SIDE CUMULATIVE DISTRIBUTION FUNCTION
OUTER-LAYER-SIDE CUMULATIVE DISTRIBUTION FUNCTION
OUTER-LAYER-SIDE CUMULATIVE DISTRIBUTION FUNCTION
INNER-LAYER-SIDE CUMULATIVE DISTRIBUTION FUNCTION
CARDIAC POTENTIAL
1452
1461
1478
1539
1557
1574
mV
100msec
: FITTING RESULT

FIG. 26

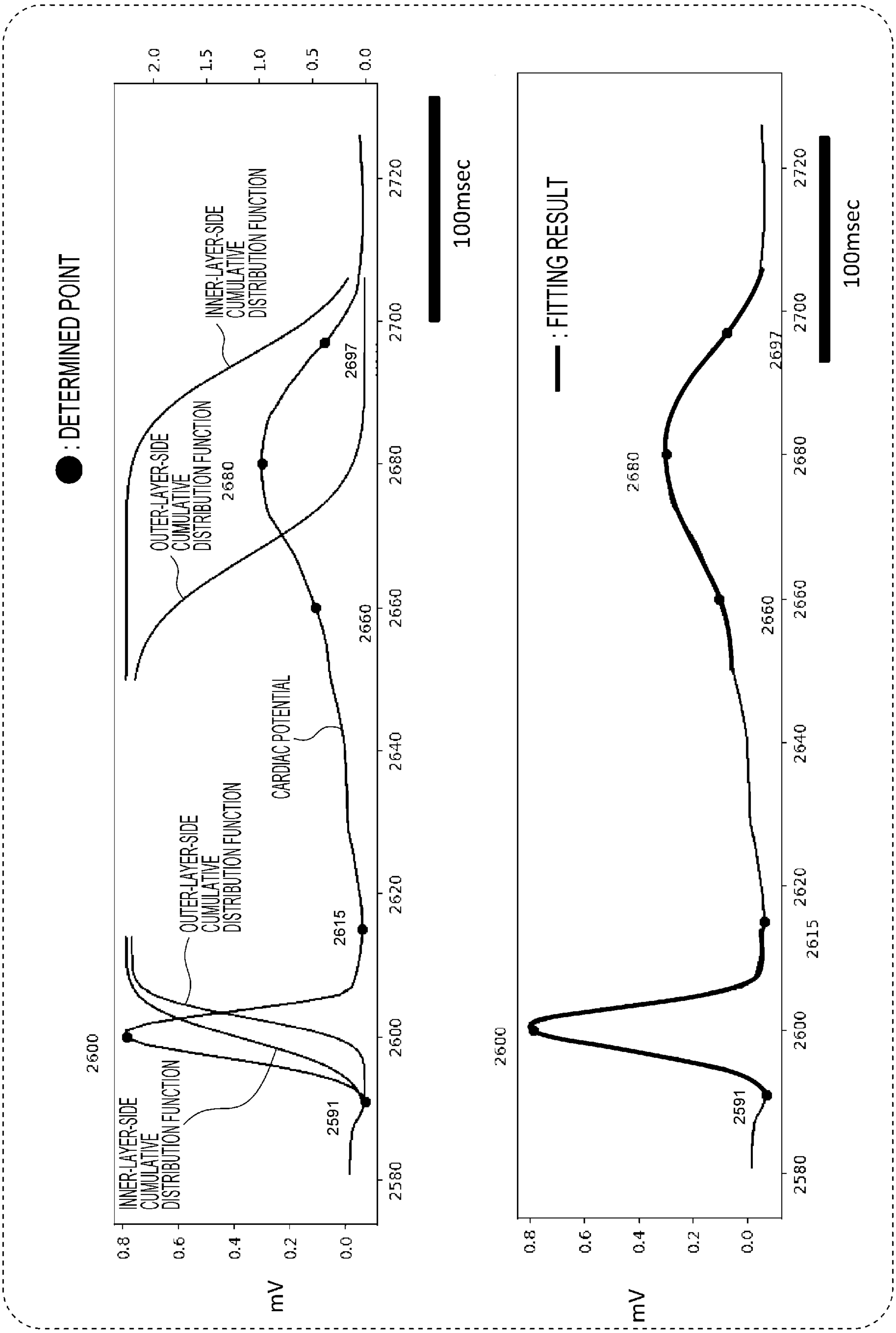
: DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
2591
2600
2615
2660
2680
2697
100msec
mV
0.8
0.6
0.4
0.2
0.0
2.0
1.5
1.0
0.5
0.0
2580
2600
2620
2640
2660
2680
2700
2720
: FITTING RESULT
2591
2600
2615
2660
2680
2697
100msec
mV
0.8
0.6
0.4
0.2
0.0
2580
2600
2620
2640
2660
2680
2700
2720

FIG. 27

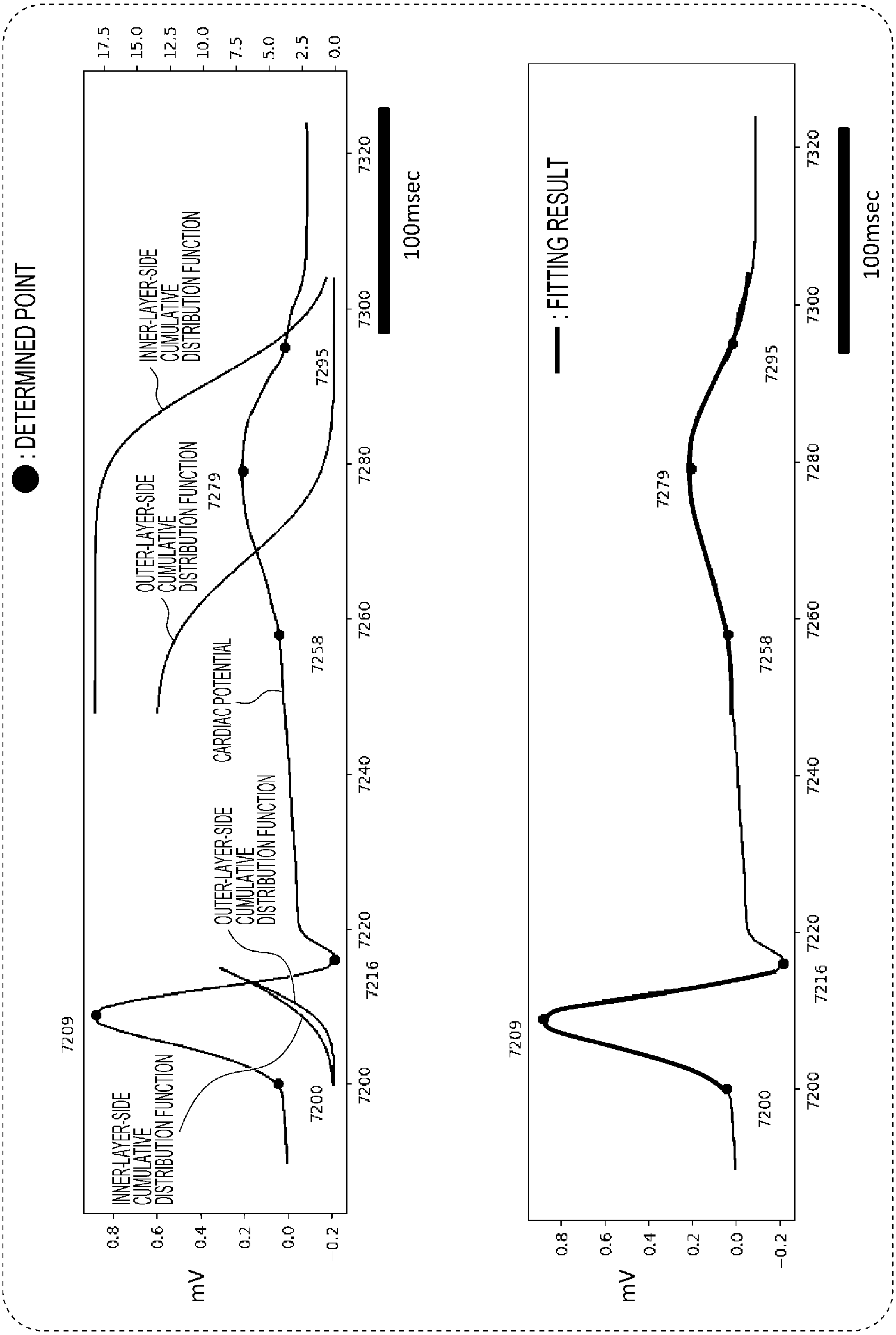
● : DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
7200
7209
7216
7258
7279
7295
mV
0.8
0.6
0.4
0.2
0.0
−0.2
7200
7220
7240
7260
7280
7300
7320
17.5
15.0
12.5
10.0
7.5
5.0
2.5
0.0
100msec
— : FITTING RESULT
7200
7209
7216
7258
7279
7295
mV
100msec

FIG. 28

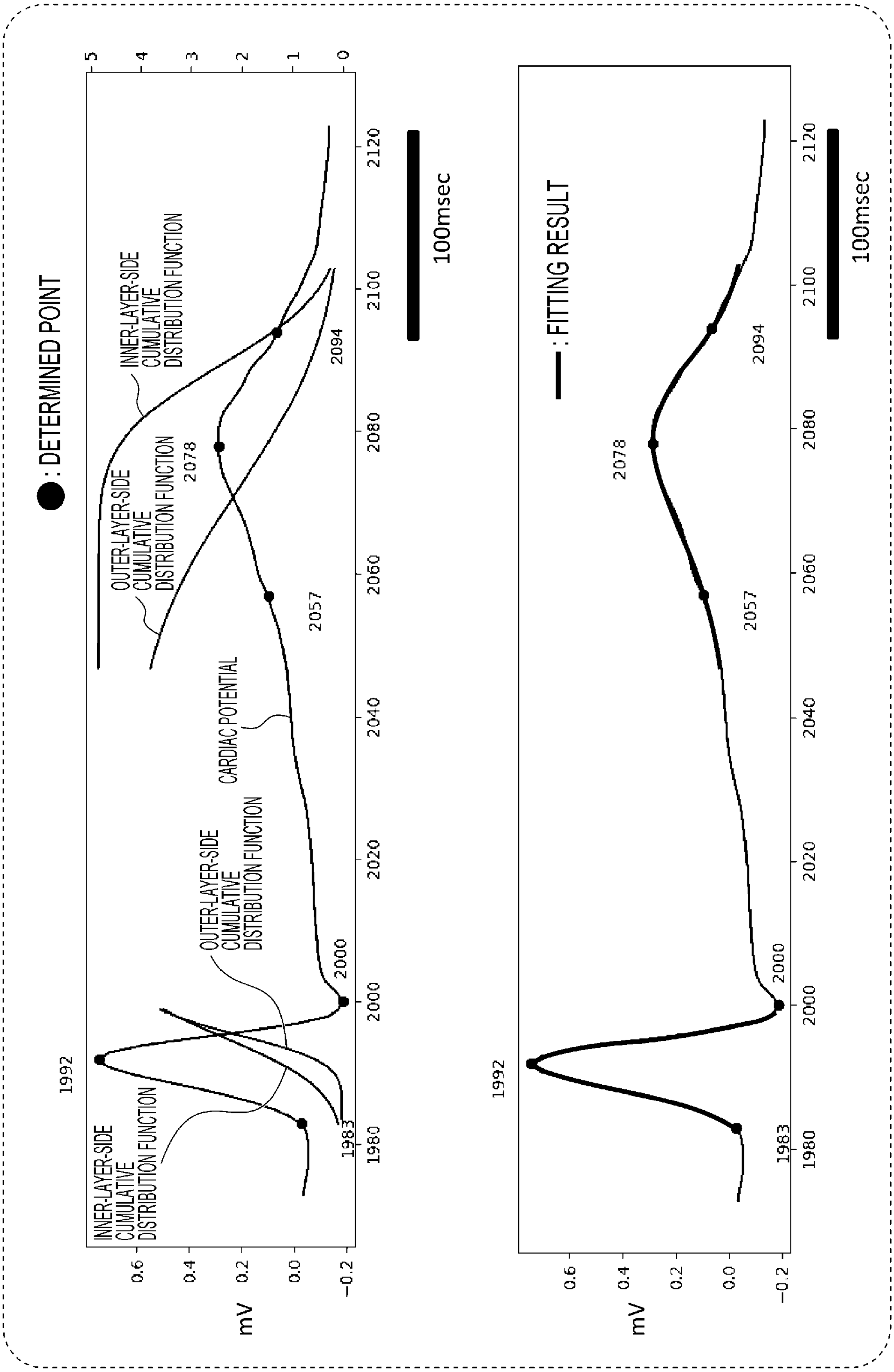
: DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
1983
1992
2000
2057
2078
2094
mV
100msec
: FITTING RESULT

FIG. 29

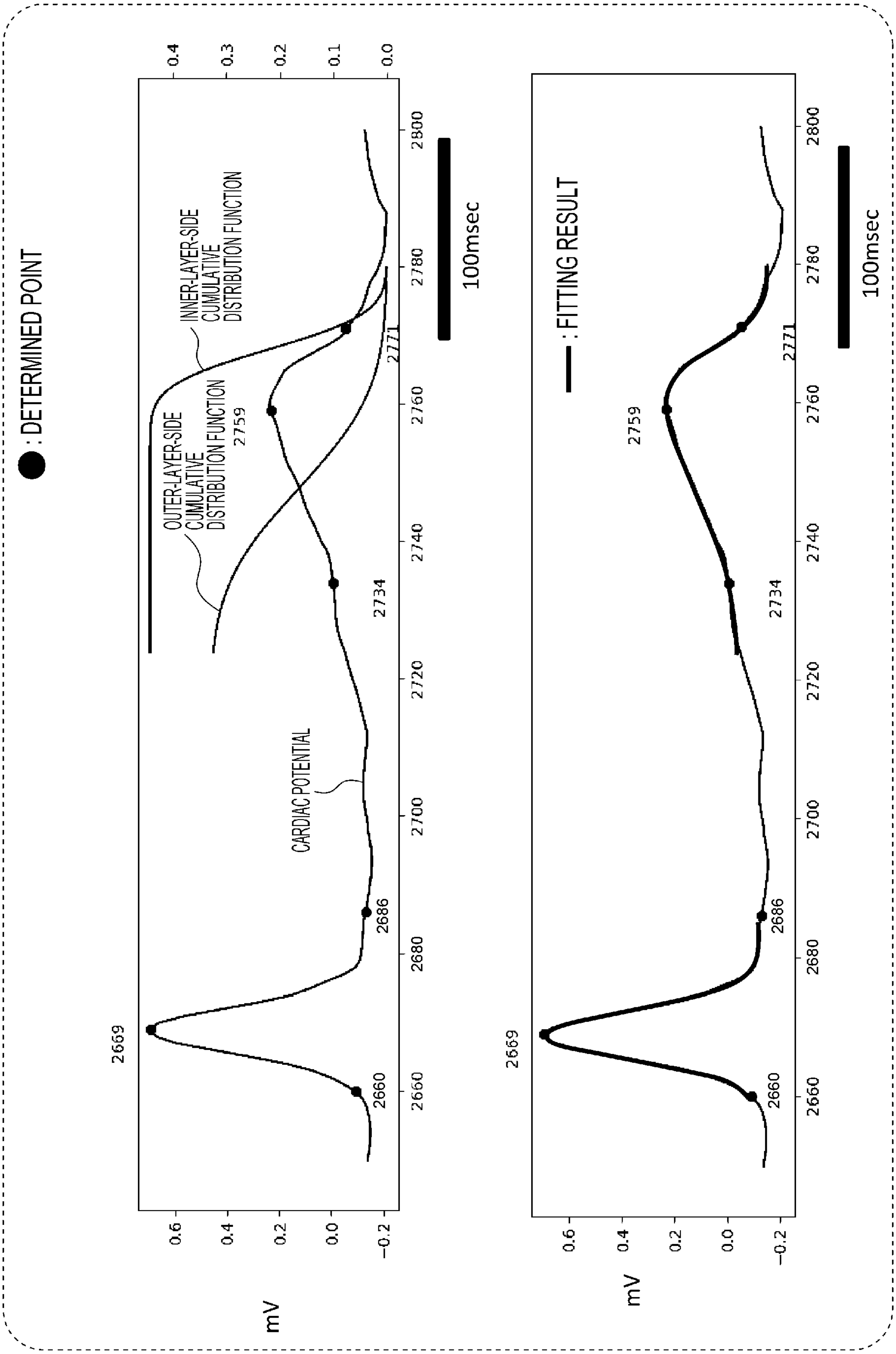
● : DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
: FITTING RESULT
100msec
mV
2660
2669
2686
2734
2759
2771
2660
2680
2700
2720
2740
2760
2780
2800
0.6
0.4
0.2
0.0
−0.2
0.4
0.3
0.2
0.1
0.0

FIG. 30

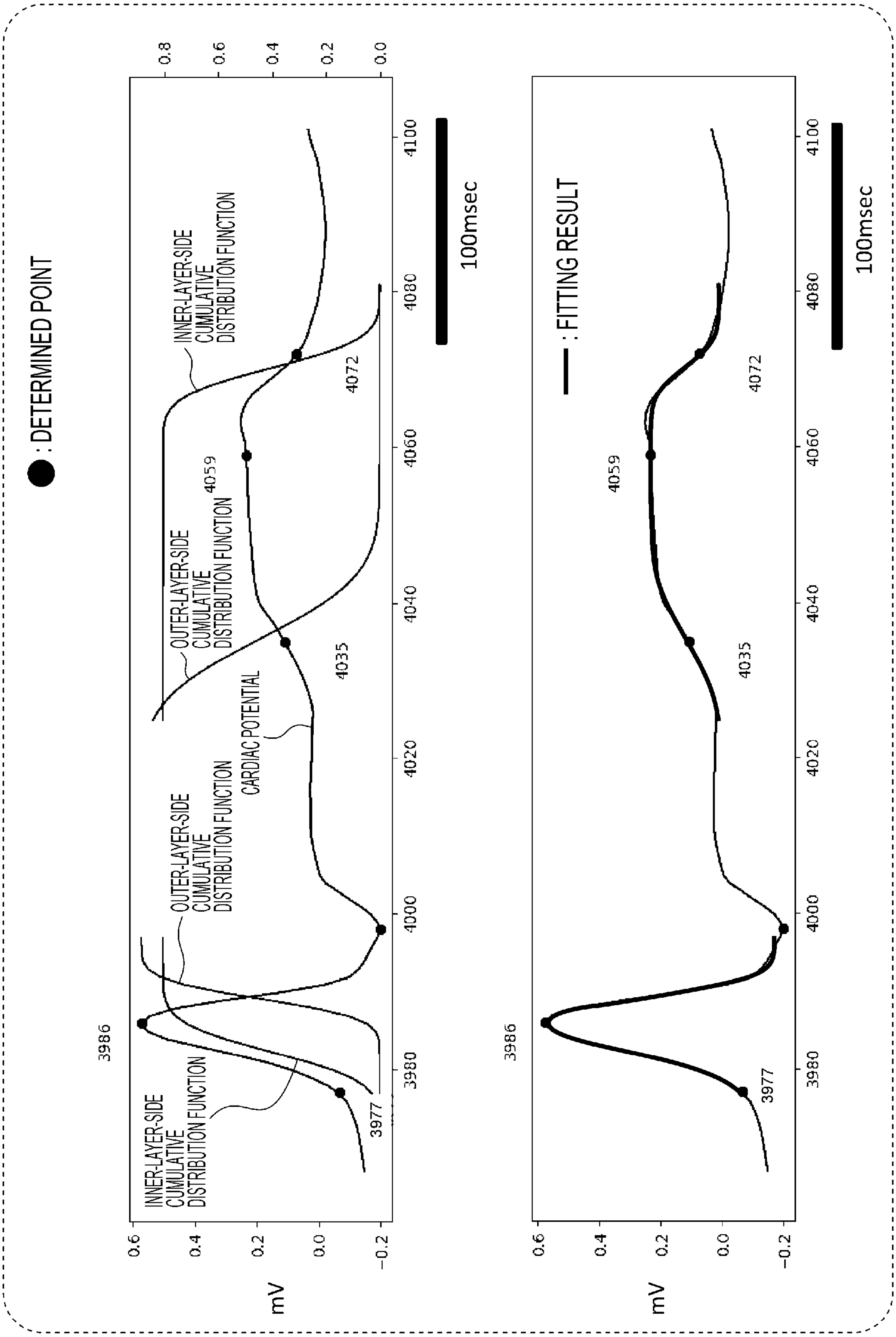
: DETERMINED POINT
INNER-LAYER-SIDE CUMULATIVE DISTRIBUTION FUNCTION
OUTER-LAYER-SIDE CUMULATIVE DISTRIBUTION FUNCTION
OUTER-LAYER-SIDE CUMULATIVE DISTRIBUTION FUNCTION
CARDIAC POTENTIAL
INNER-LAYER-SIDE CUMULATIVE DISTRIBUTION FUNCTION
3977
3986
4035
4059
4072
mV
0.6
0.4
0.2
0.0
−0.2
0.8
3980
4000
4020
4040
4060
4080
4100
100msec
: FITTING RESULT

FIG. 31

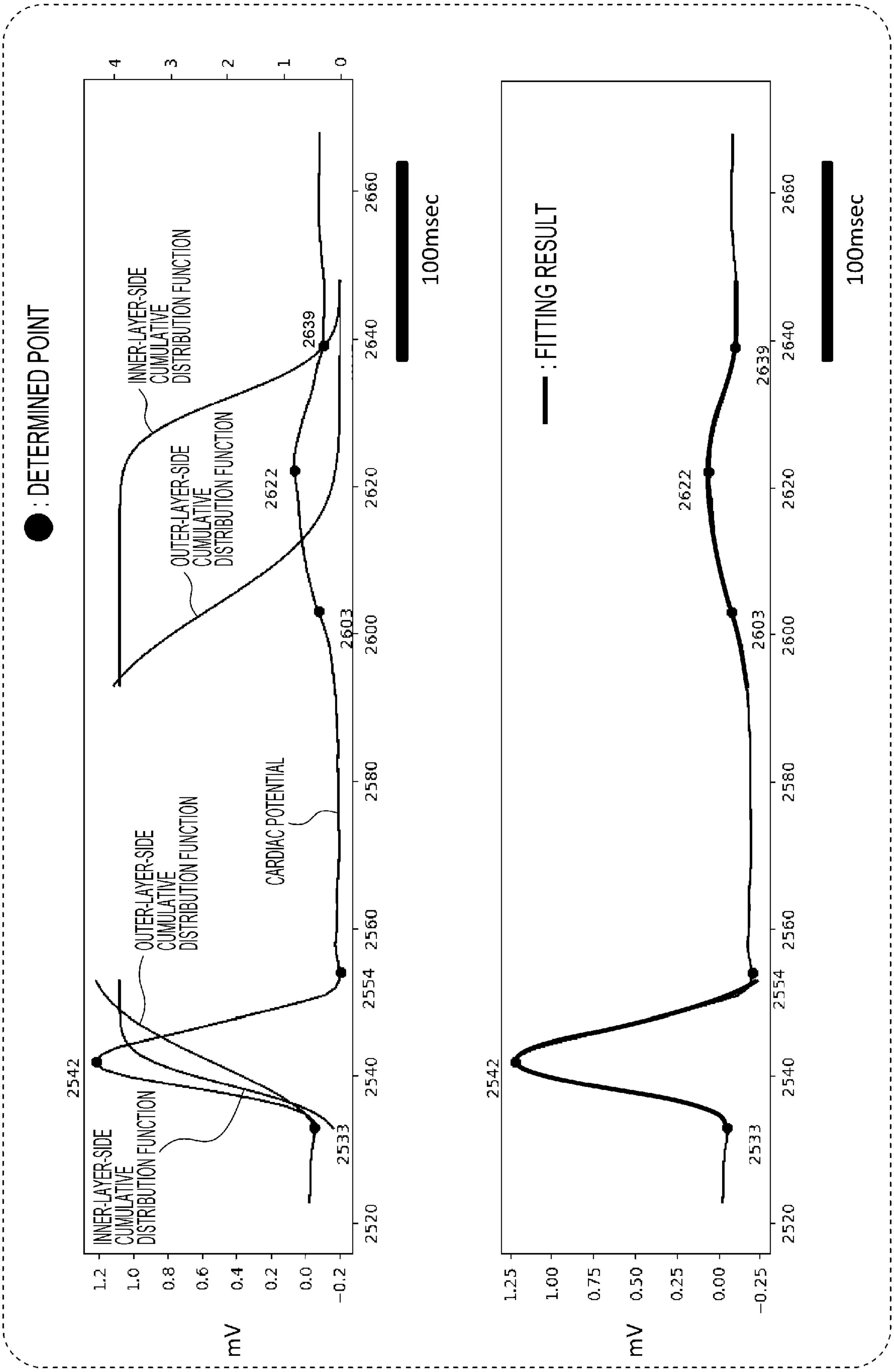
● : DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
INNER-LAYER-SIDE CUMULATIVE DISTRIBUTION FUNCTION
OUTER-LAYER-SIDE CUMULATIVE DISTRIBUTION FUNCTION
2533
2542
2554
2603
2622
2639
mV
1.2
1.0
0.8
0.6
0.4
0.2
0.0
−0.2
0
1
2
3
4
2520
2540
2560
2580
2600
2620
2640
2660
100msec
: FITTING RESULT
mV
1.25
1.00
0.75
0.50
0.25
0.00
−0.25

FIG. 32

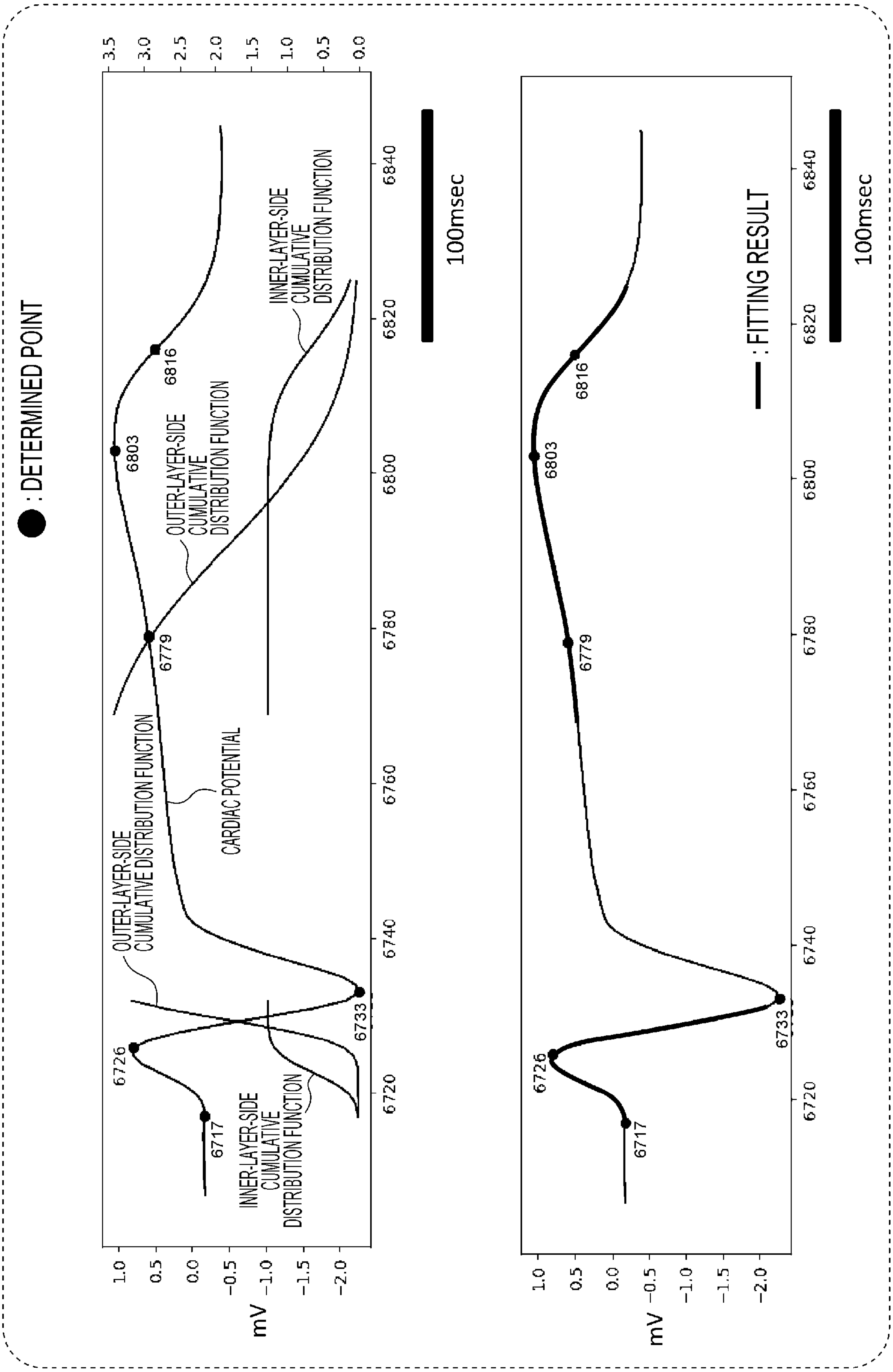
: DETERMINED POINT
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
INNER-LAYER-SIDE CUMULATIVE DISTRIBUTION FUNCTION
6717
6726
6733
6779
6803
6816
6720
6740
6760
6780
6800
6820
6840
mV
1.0
0.5
0.0
-0.5
-1.0
-1.5
-2.0
3.5
3.0
2.5
2.0
1.5
1.0
0.5
0.0
100msec
: FITTING RESULT
6717
6726
6733
6779
6803
6816
mV
100msec

FIG. 33

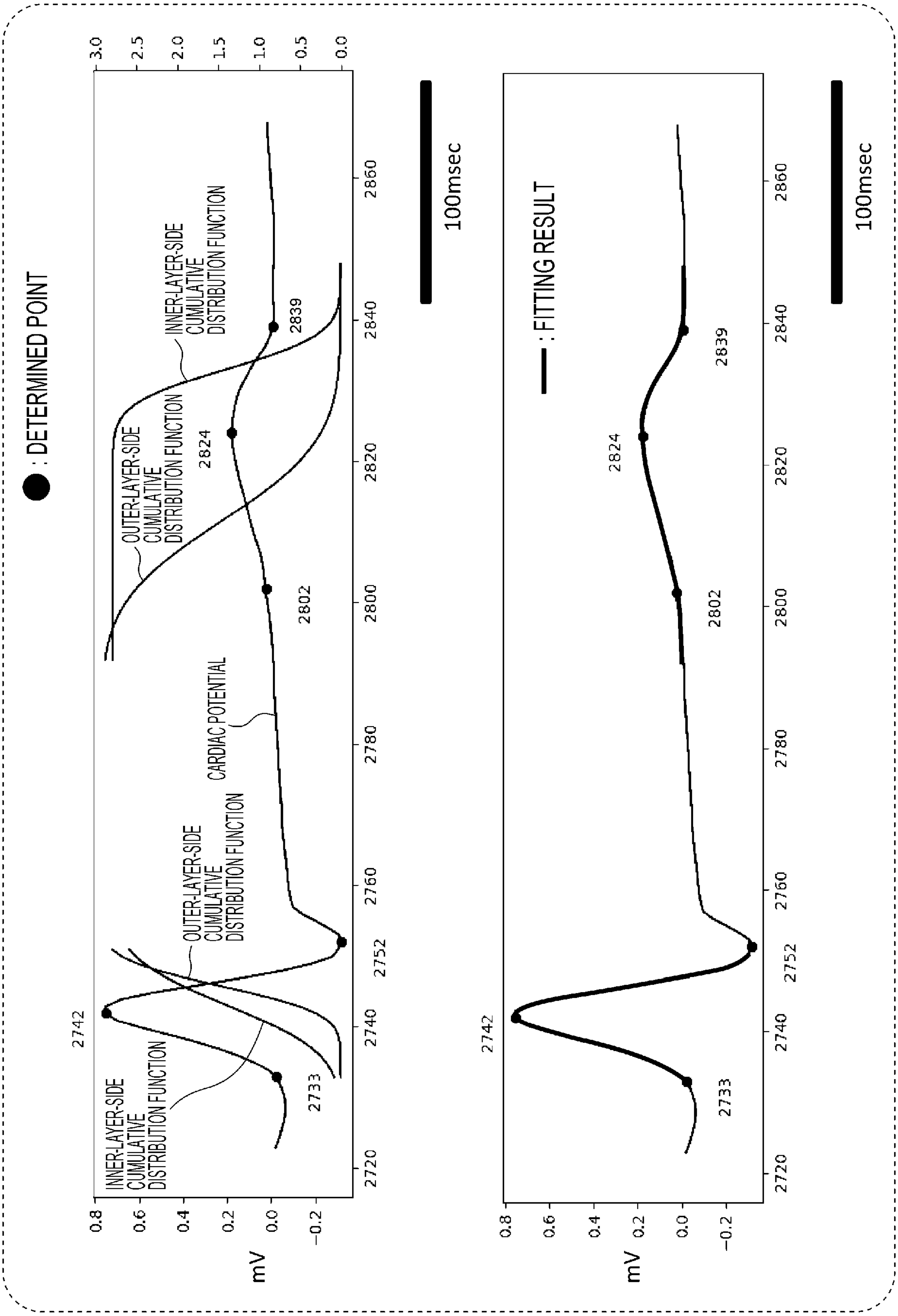
: DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
2733
2742
2752
2802
2824
2839
mV
0.8
0.6
0.4
0.2
0.0
−0.2
3.0
2.5
2.0
1.5
1.0
0.5
0.0
2720
2740
2760
2780
2800
2820
2840
2860
100msec
: FITTING RESULT

FIG. 34

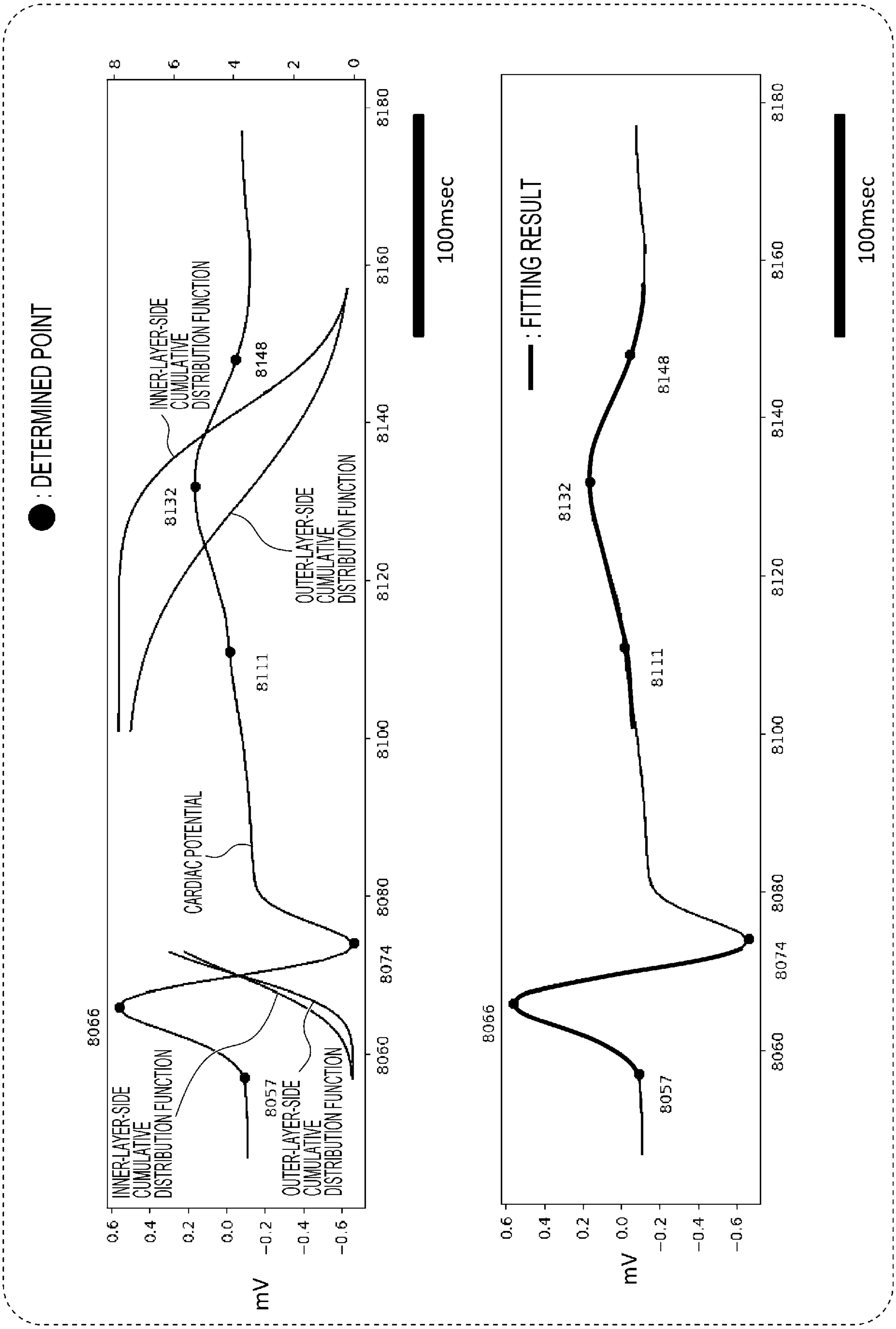
: DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
8057
8066
8074
8111
8132
8148
mV
100msec
: FITTING RESULT
8057
8066
8074
8111
8132
8148
mV
100msec

FIG. 35

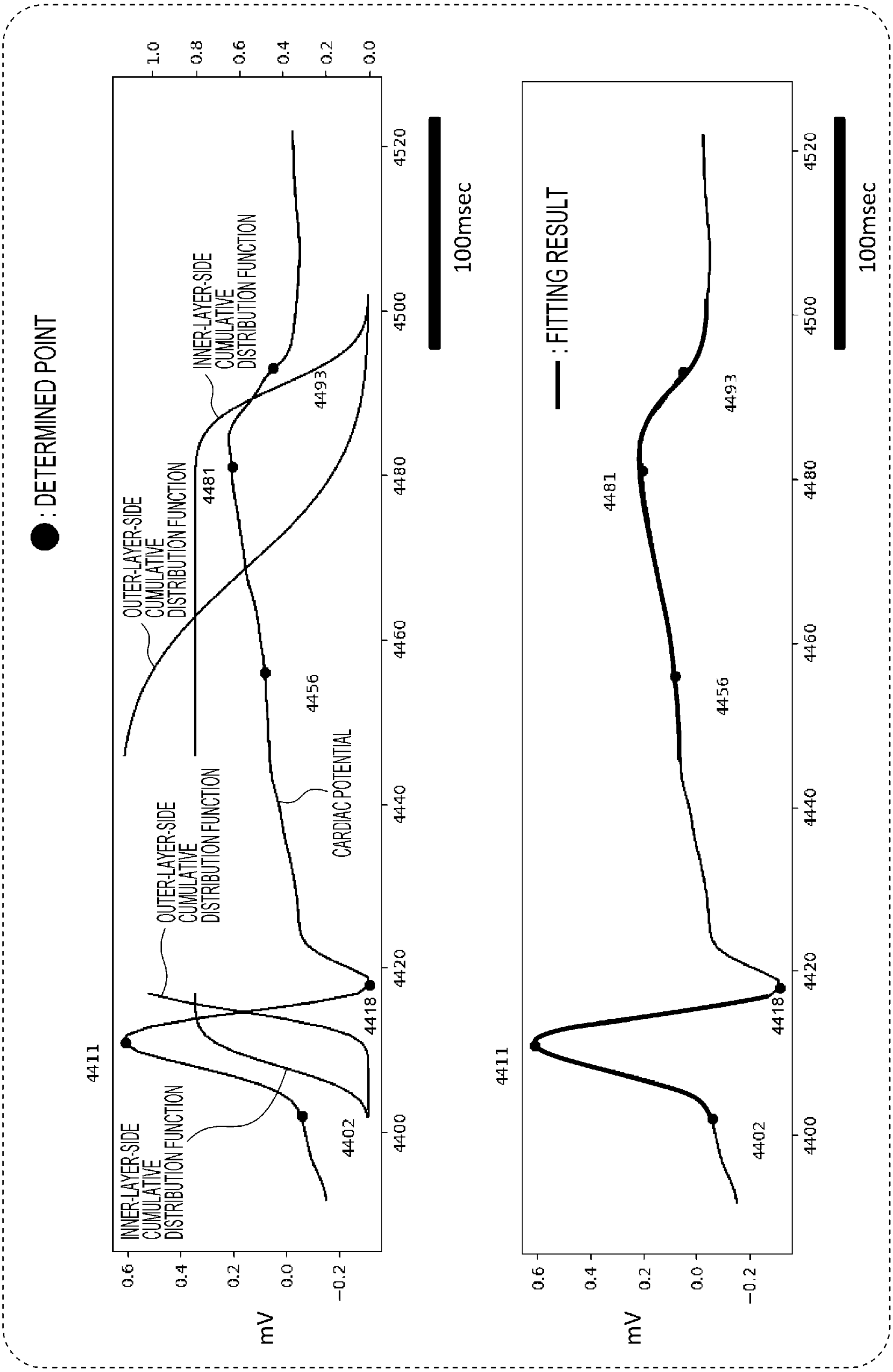
: DETERMINED POINT
INNER-LAYER-SIDE CUMULATIVE DISTRIBUTION FUNCTION
OUTER-LAYER-SIDE CUMULATIVE DISTRIBUTION FUNCTION
OUTER-LAYER-SIDE CUMULATIVE DISTRIBUTION FUNCTION
INNER-LAYER-SIDE CUMULATIVE DISTRIBUTION FUNCTION
CARDIAC POTENTIAL
4402
4411
4418
4456
4481
4493
4400
4420
4440
4460
4480
4500
4520
mV
0.6
0.4
0.2
0.0
−0.2
1.0
0.8
0.6
0.4
0.2
0.0
100msec
: FITTING RESULT
4402
4411
4418
4456
4481
4493
4400
4420
4440
4460
4480
4500
4520
mV
0.6
0.4
0.2
0.0
−0.2
100msec

FIG. 36

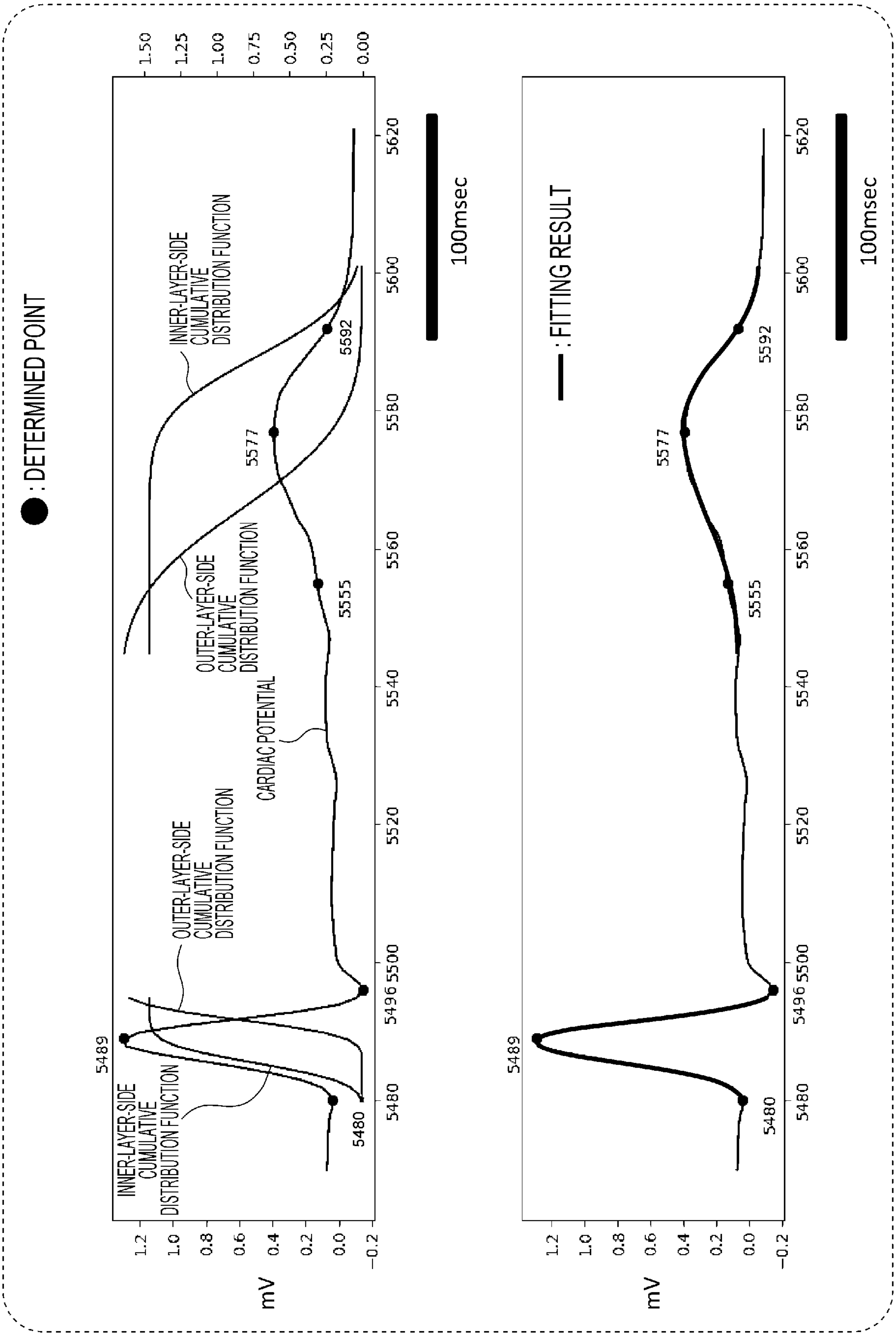
● : DETERMINED POINT
mV
1.2
1.0
0.8
0.6
0.4
0.2
0.0
−0.2
1.50
1.25
1.00
0.75
0.50
0.25
0.00
5480
5500
5520
5540
5560
5580
5600
5620
5480
5489
5496
5555
5577
5592
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
100msec
: FITTING RESULT
mV
1.2
1.0
0.8
0.6
0.4
0.2
0.0
−0.2
5480
5500
5520
5540
5560
5580
5600
5620
5480
5489
5496
5555
5577
5592
100msec

FIG. 37

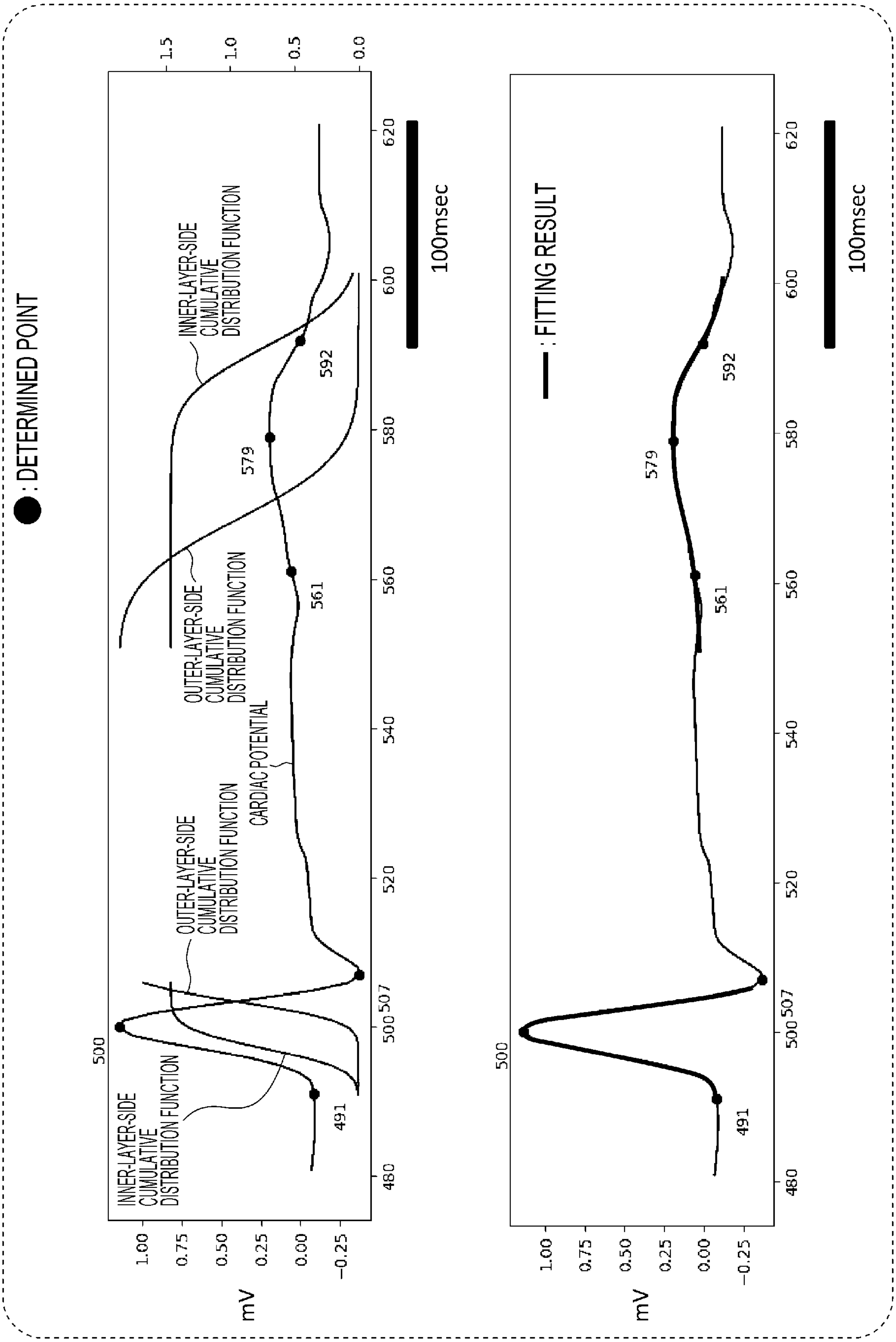
: DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
491
500
507
561
579
592
mV
1.00
0.75
0.50
0.25
0.00
−0.25
480
500
520
540
560
580
600
620
1.5
1.0
0.5
0.0
100msec
: FITTING RESULT
491
500
507
561
579
592
mV
1.00
0.75
0.50
0.25
0.00
−0.25
480
500
520
540
560
580
600
620
100msec

FIG. 38

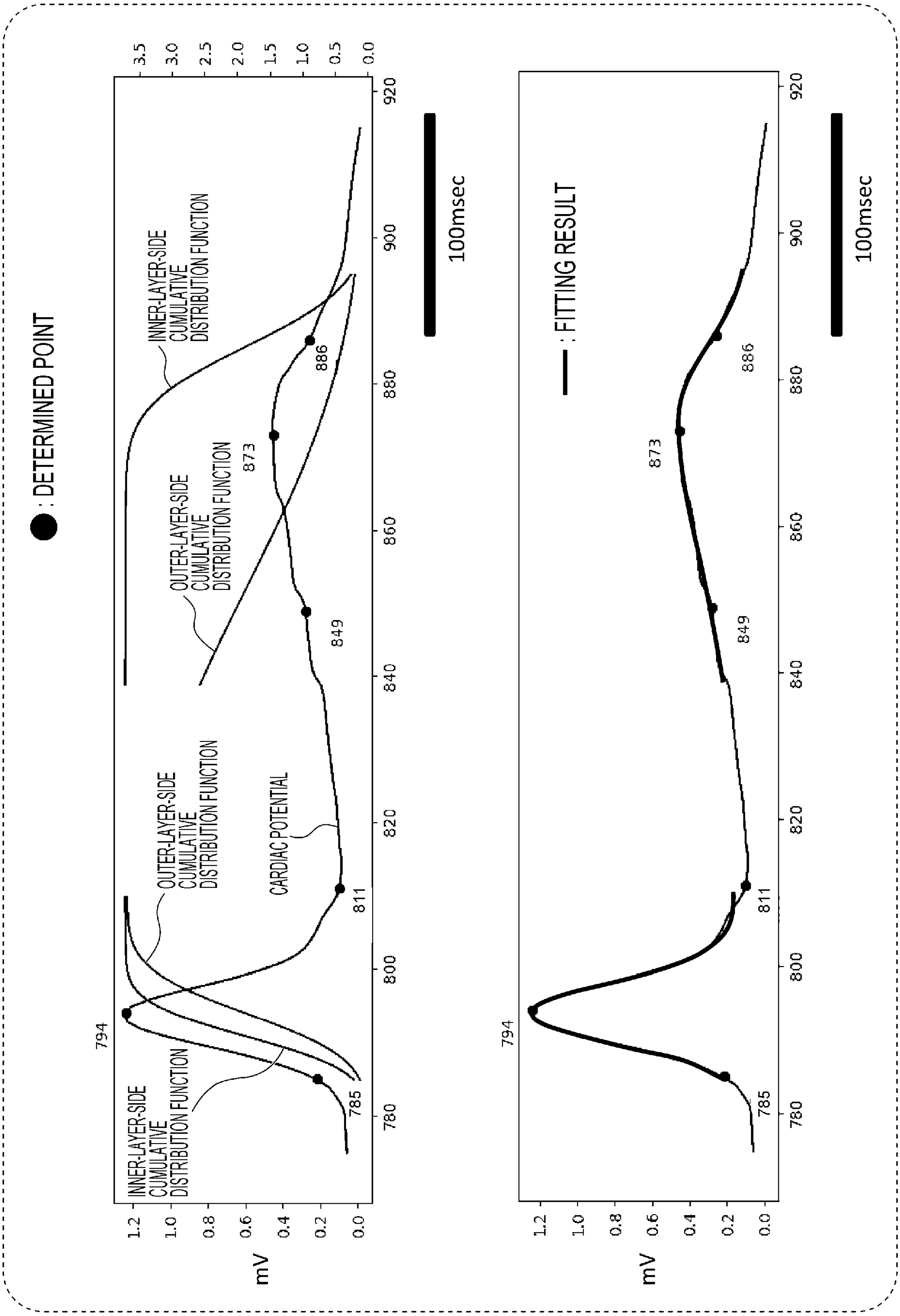
: DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
785
794
811
849
873
886
mV
1.2
1.0
0.8
0.6
0.4
0.2
0.0
3.5
3.0
2.5
2.0
1.5
1.0
0.5
0.0
780
800
820
840
860
880
900
920
100msec
: FITTING RESULT
785
794
811
849
873
886
mV
1.2
1.0
0.8
0.6
0.4
0.2
0.0
780
800
820
840
860
880
900
920
100msec

FIG. 39

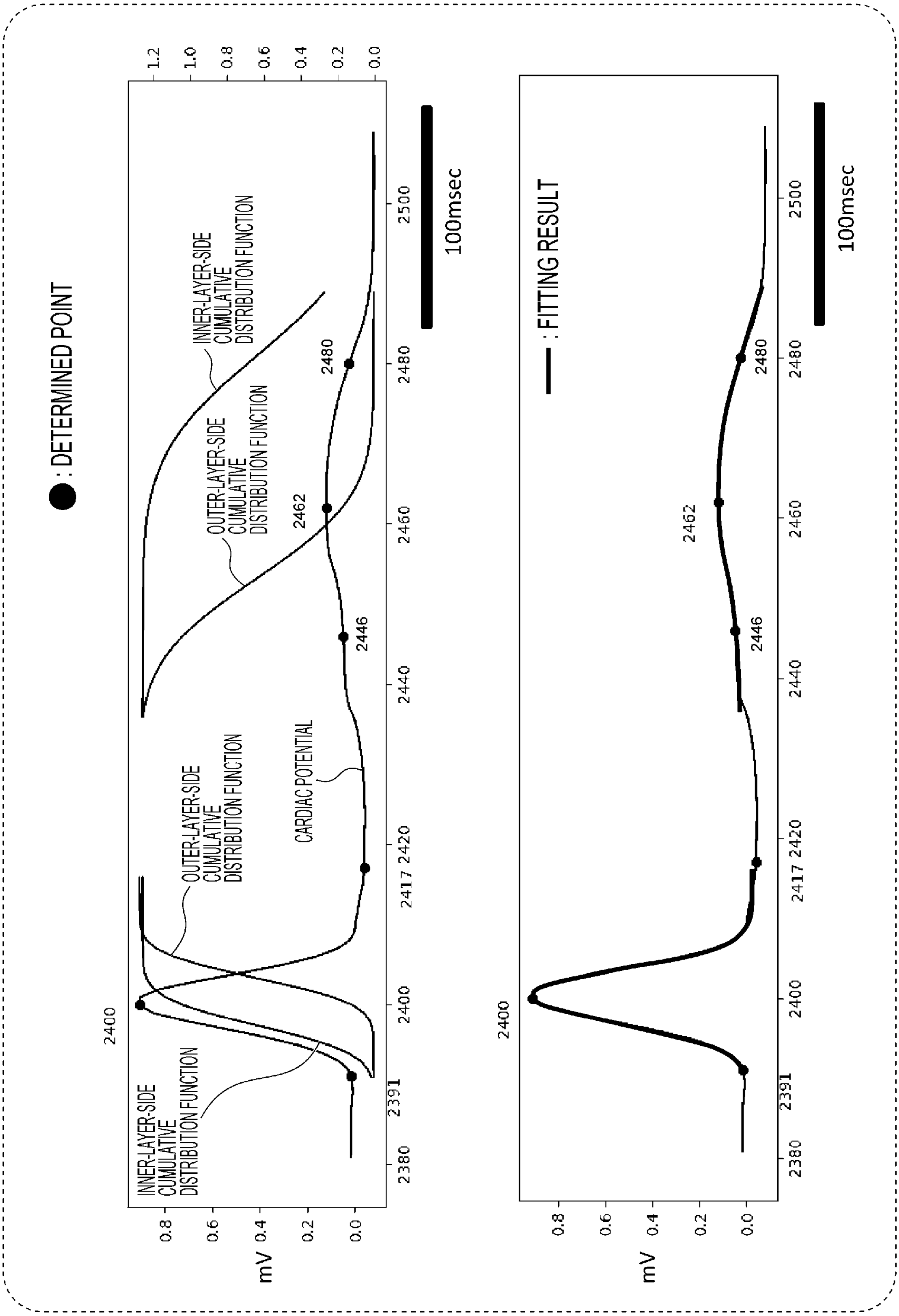

● : DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
mV
100msec
: FITTING RESULT
2380 2391 2400 2417 2420 2440 2446 2460 2462 2480 2500
0.0 0.2 0.4 0.6 0.8 1.0 1.2

FIG. 40

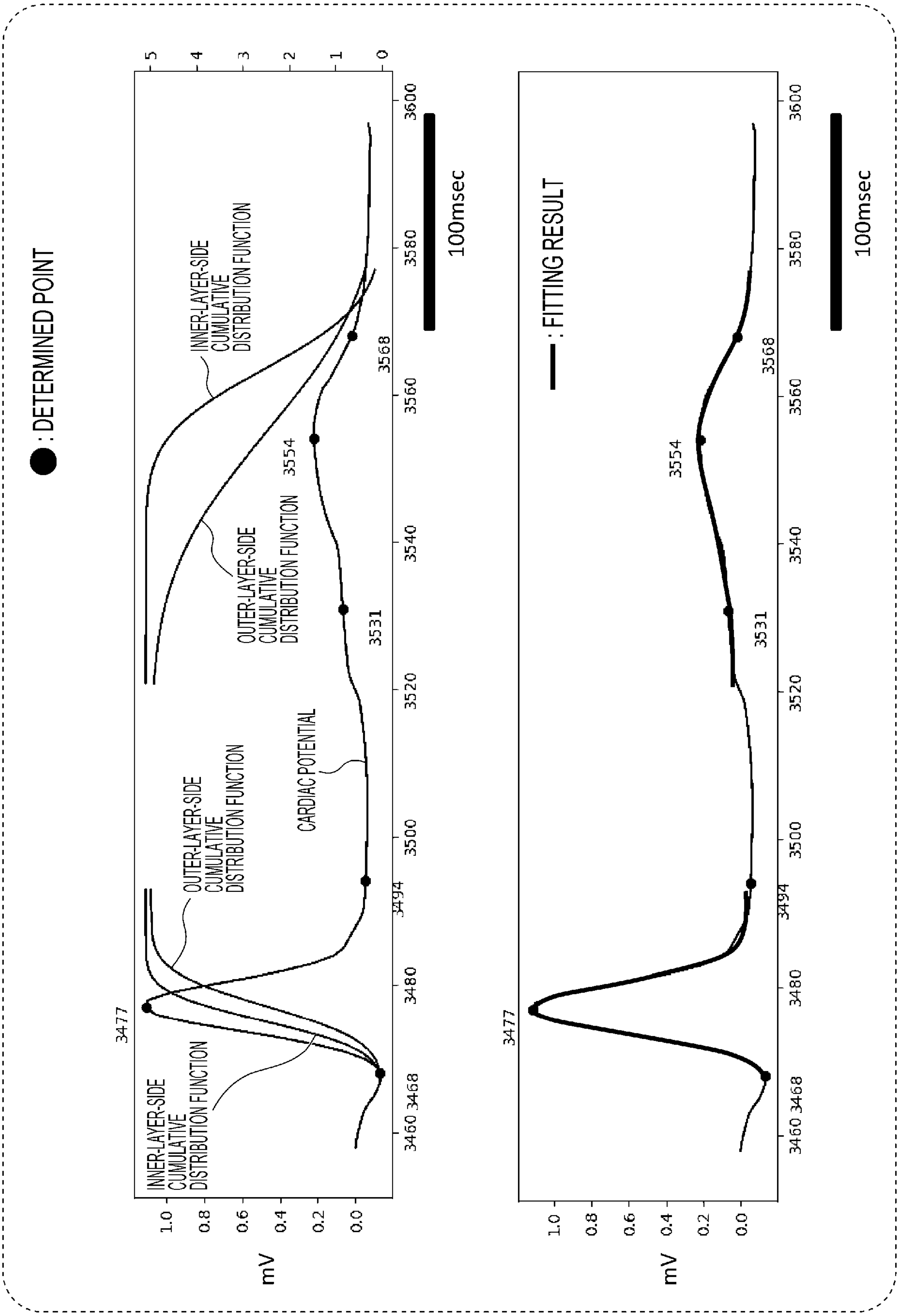
: DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
3477
3468
3494
3531
3554
3568
mV
1.0
0.8
0.6
0.4
0.2
0.0
3460
3480
3500
3520
3540
3560
3580
3600
0
1
2
3
4
5
100msec
: FITTING RESULT
3477
3468
3494
3531
3554
3568
mV
1.0
0.8
0.6
0.4
0.2
0.0
3460
3480
3500
3520
3540
3560
3580
3600
100msec

FIG. 41

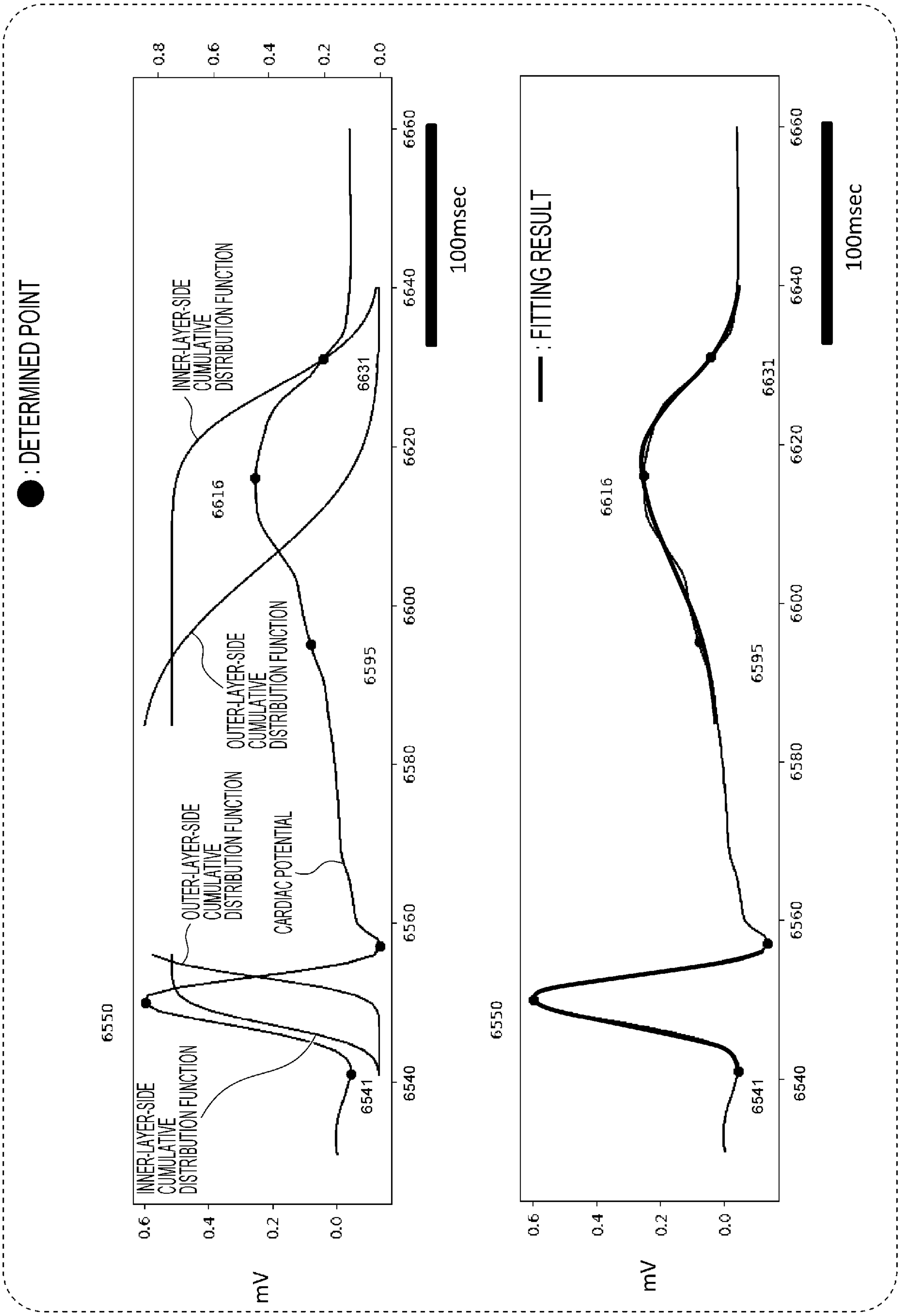
: DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
6541
6550
6595
6616
6631
mV
0.6
0.4
0.2
0.0
0.8
6540
6560
6580
6600
6620
6640
6660
100msec
: FITTING RESULT

FIG. 42

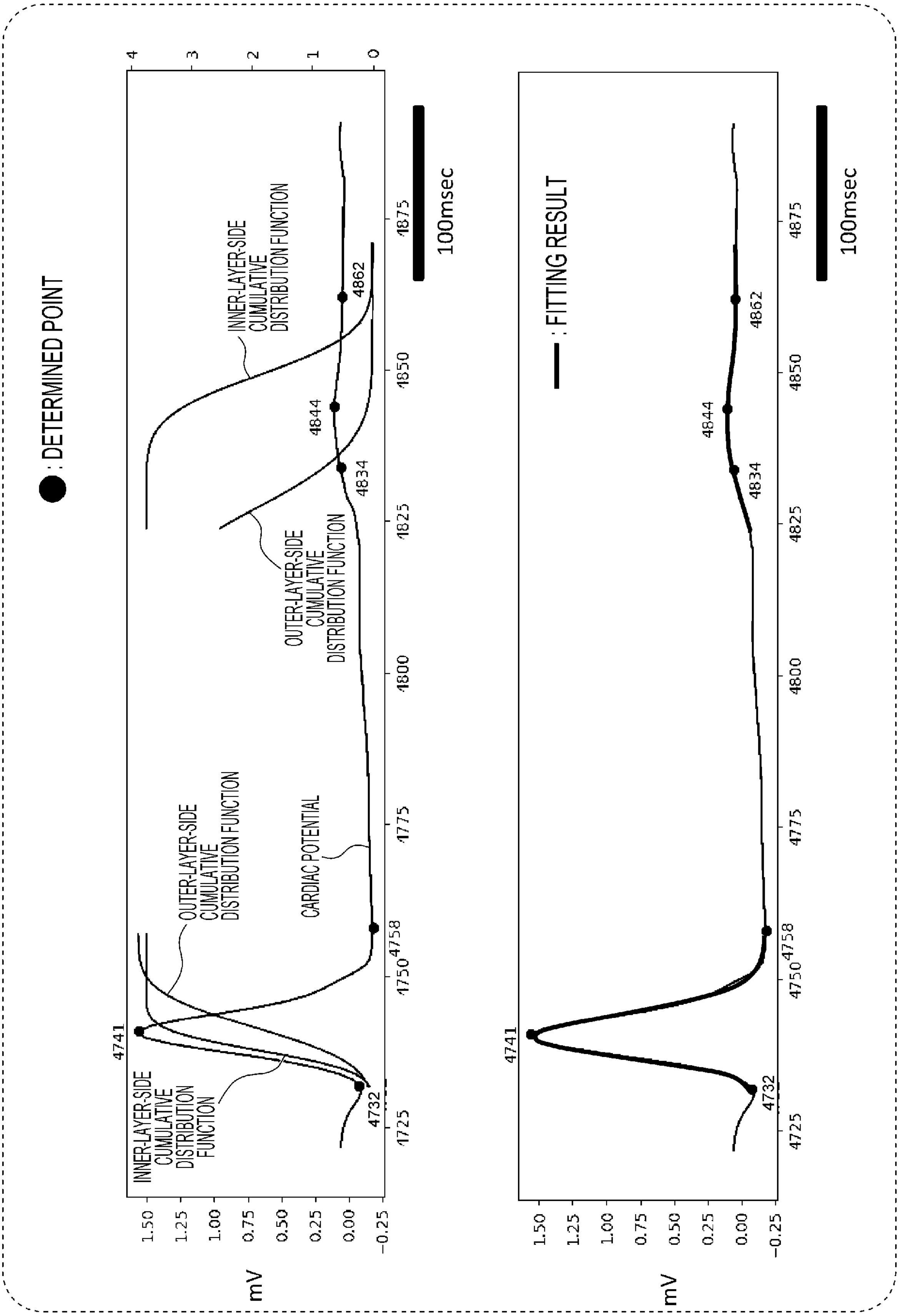
● : DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
4732
4741
4758
4834
4844
4862
mV
1.50
1.25
1.00
0.75
0.50
0.25
0.00
−0.25
4725
4750
4775
4800
4825
4850
4875
0
1
2
3
4
100msec
: FITTING RESULT
4732
4741
4758
4834
4844
4862
mV
1.50
1.25
1.00
0.75
0.50
0.25
0.00
−0.25
4725
4750
4775
4800
4825
4850
4875
100msec

FIG. 43

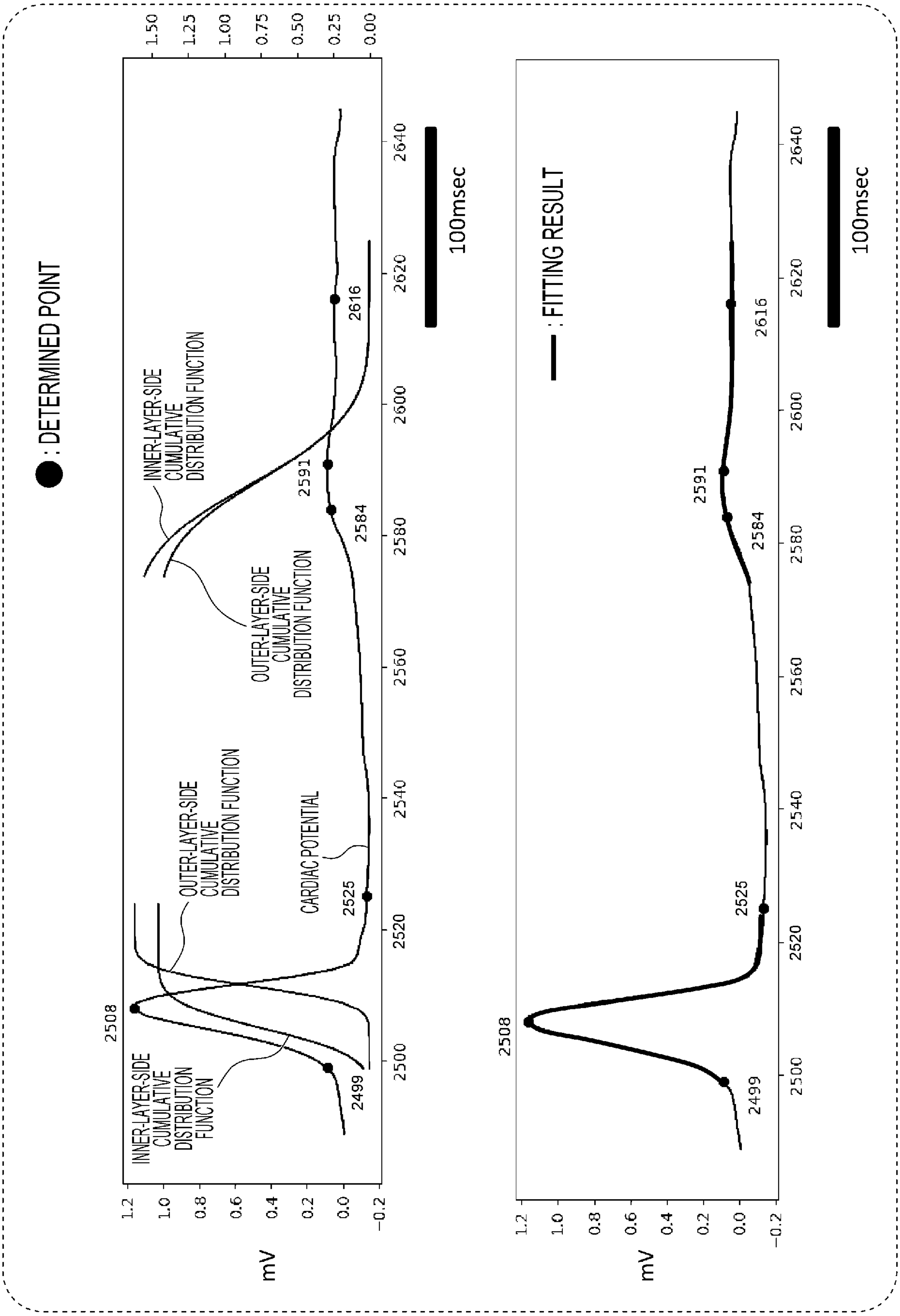
: DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
2499
2508
2525
2584
2591
2616
mV
100msec
: FITTING RESULT

FIG. 44

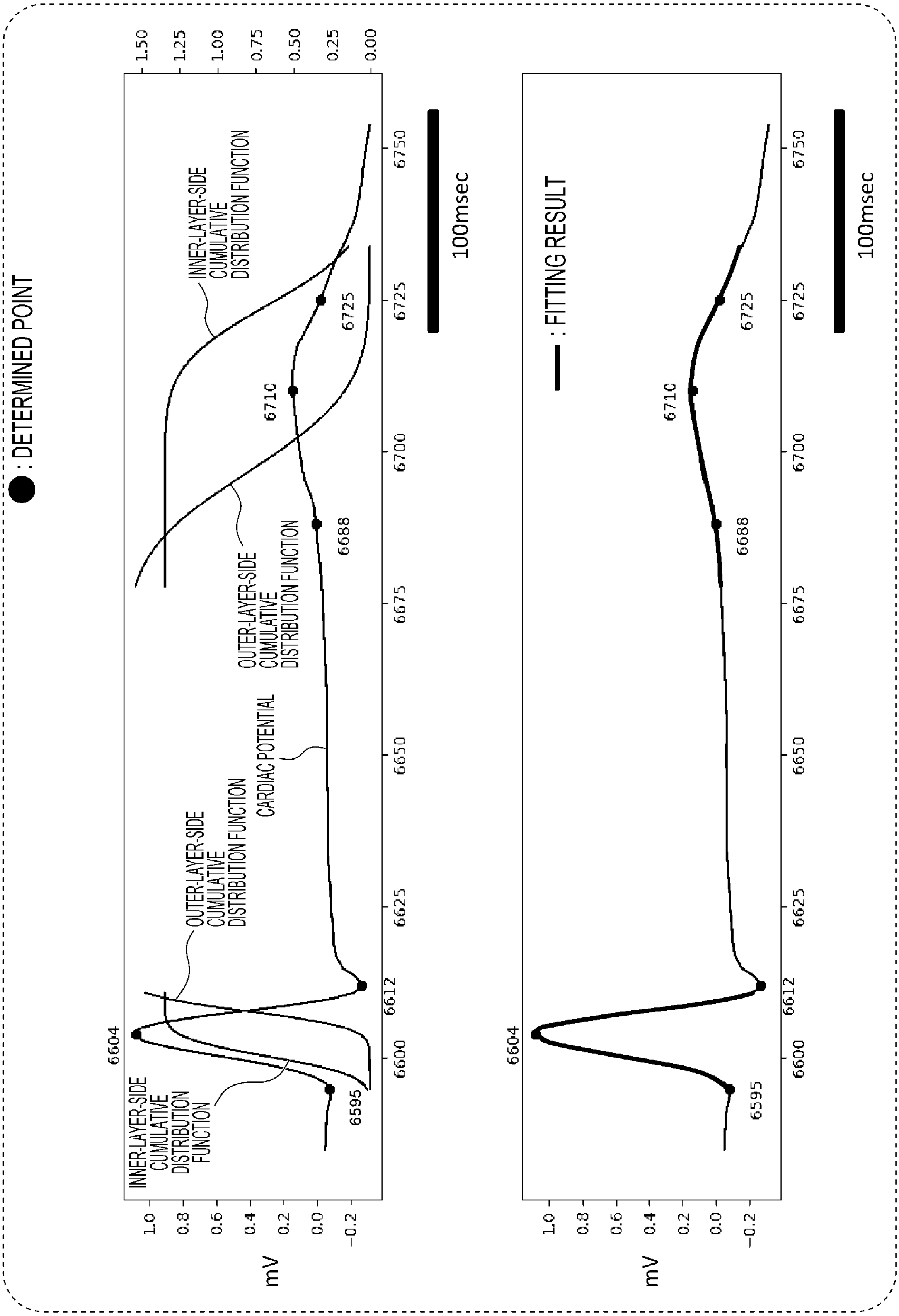
: DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
6595
6604
6612
6688
6710
6725
mV
1.0
0.8
0.6
0.4
0.2
0.0
−0.2
6600
6625
6650
6675
6700
6725
6750
1.50
1.25
1.00
0.75
0.50
0.25
0.00
100msec
: FITTING RESULT

FIG. 45

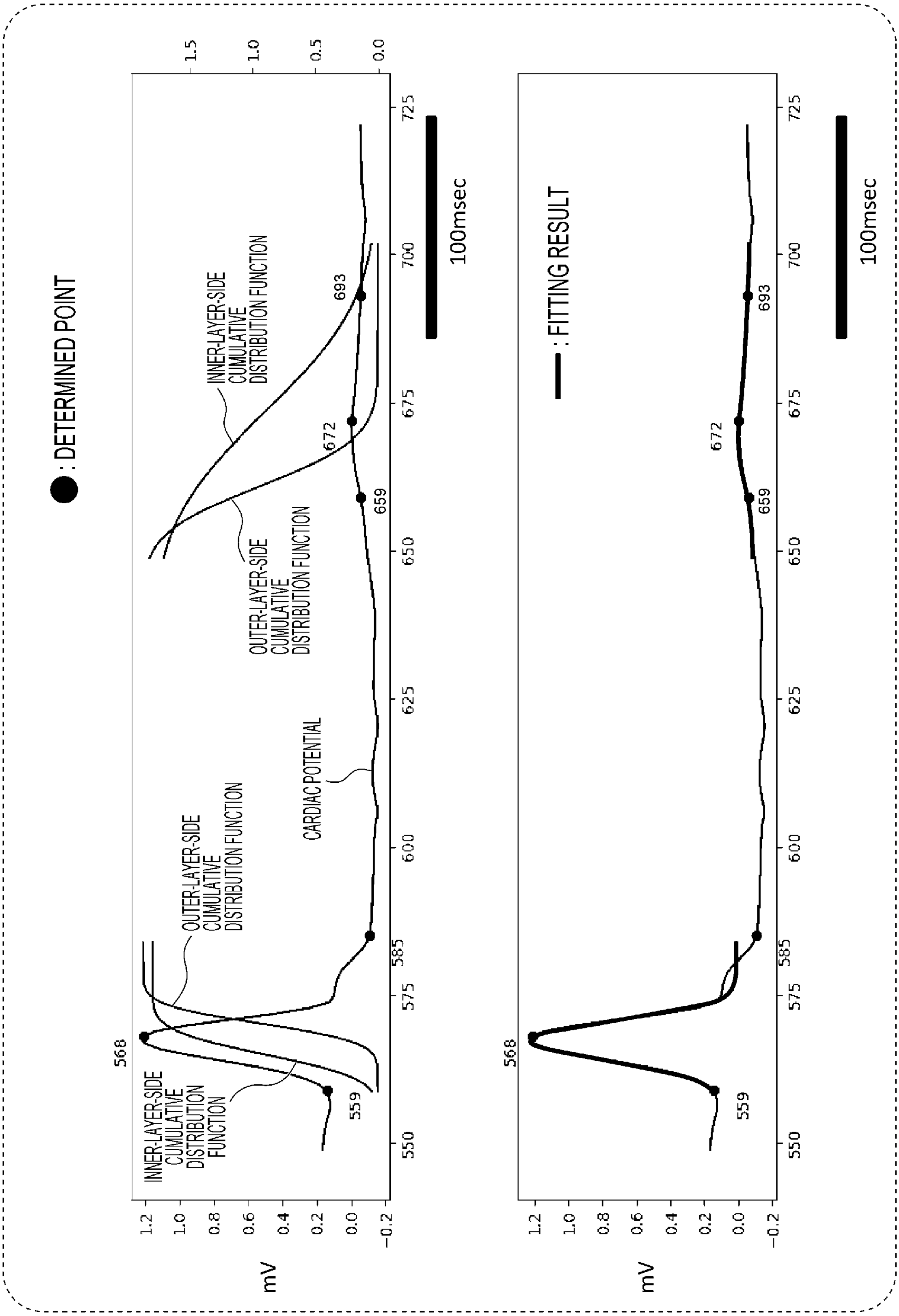

: DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
559
568
585
659
672
693
mV
1.2
1.0
0.8
0.6
0.4
0.2
0.0
−0.2
1.5
1.0
0.5
0.0
550
575
600
625
650
675
700
725
100msec
: FITTING RESULT
559
568
585
659
672
693
mV
1.2
1.0
0.8
0.6
0.4
0.2
0.0
−0.2
550
575
600
625
650
675
700
725
100msec

FIG. 46

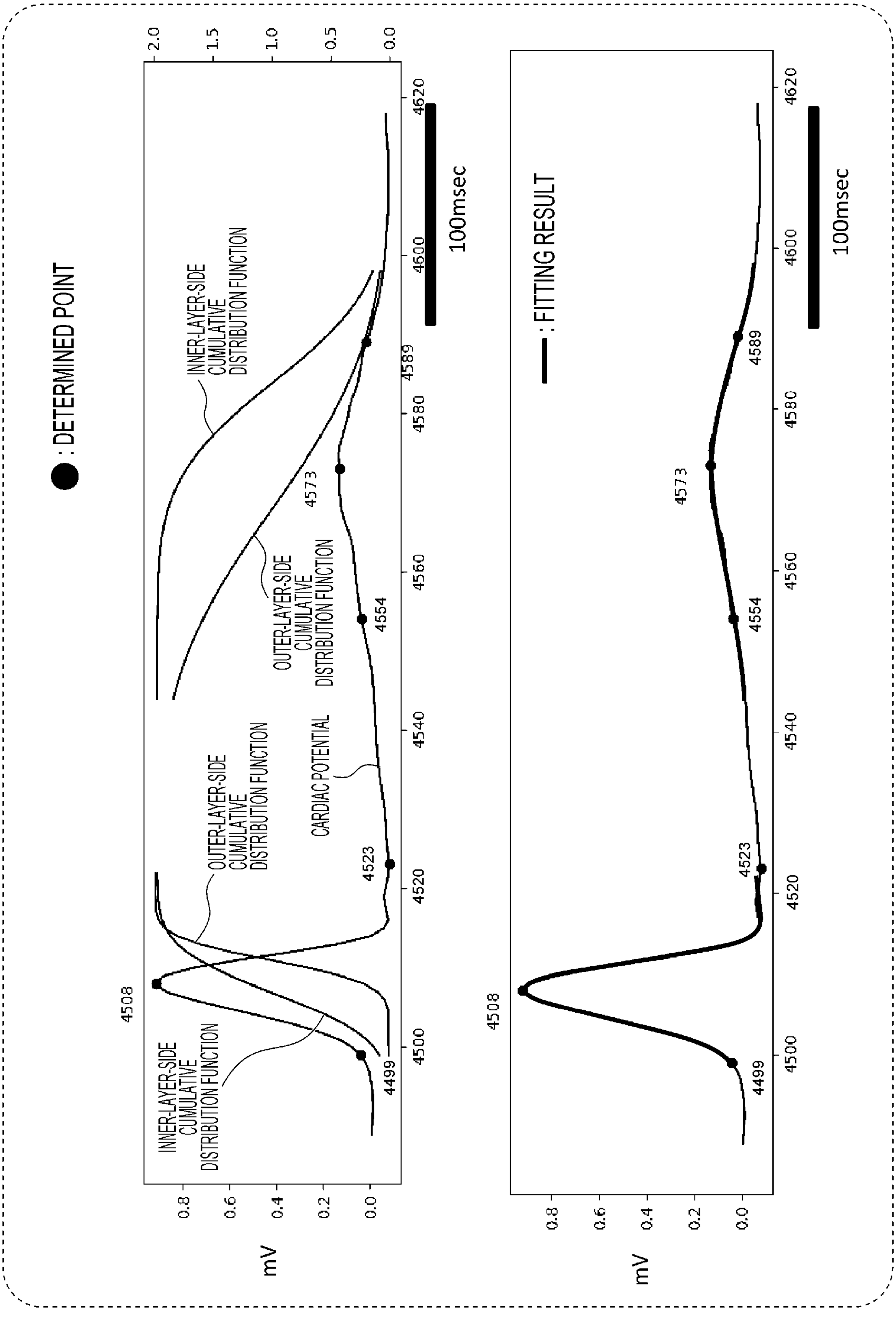
: DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
4499
4508
4523
4554
4573
4589
mV
0.0
0.2
0.4
0.6
0.8
2.0
1.5
1.0
0.5
0.0
4500
4520
4540
4560
4580
4600
4620
100msec
: FITTING RESULT
4499
4508
4523
4554
4573
4589
mV
0.0
0.2
0.4
0.6
0.8
4500
4520
4540
4560
4580
4600
4620
100msec

FIG. 47

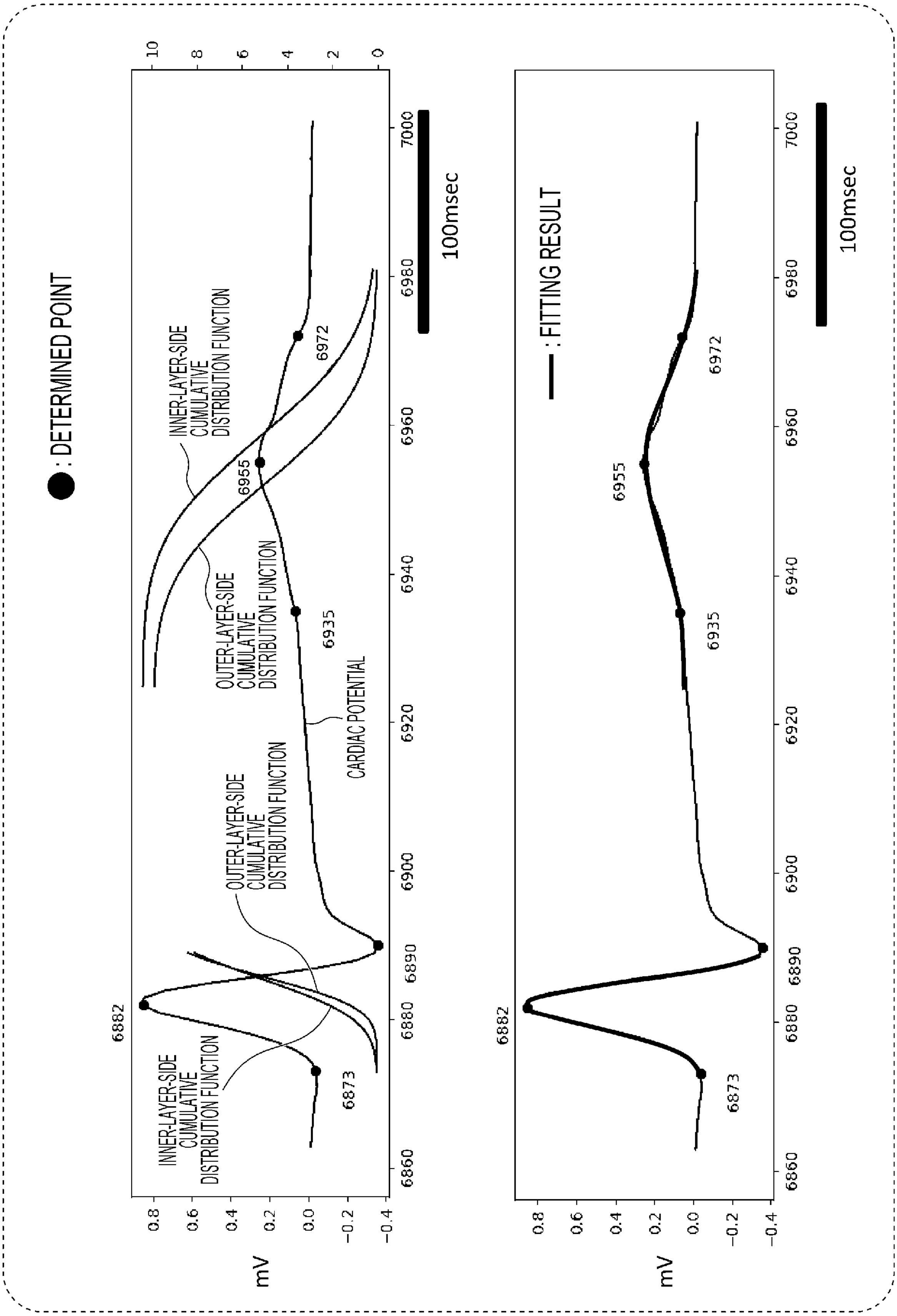
: DETERMINED POINT
INNER-LAYER-SIDE CUMULATIVE DISTRIBUTION FUNCTION
OUTER-LAYER-SIDE CUMULATIVE DISTRIBUTION FUNCTION
OUTER-LAYER-SIDE CUMULATIVE DISTRIBUTION FUNCTION
INNER-LAYER-SIDE CUMULATIVE DISTRIBUTION FUNCTION
CARDIAC POTENTIAL
6873
6882
6935
6955
6972
mV
0.8
0.6
0.4
0.2
0.0
−0.2
−0.4
6860
6880
6890
6900
6920
6940
6960
6980
7000
10
8
6
4
2
0
100msec
: FITTING RESULT
6873
6882
6935
6955
6972
mV
100msec

FIG. 48

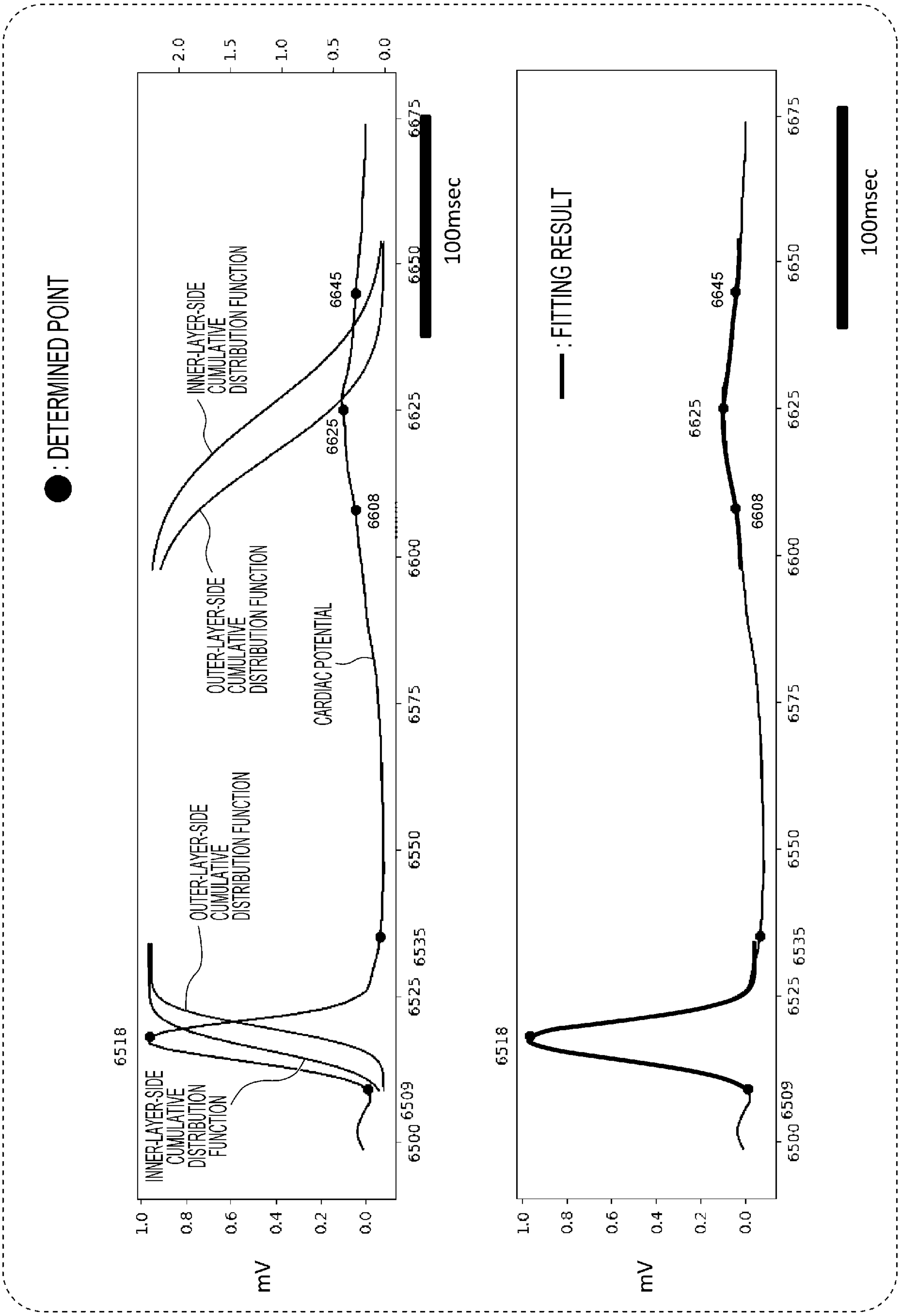
: DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
6509
6518
6535
6608
6625
6645
6500
6525
6550
6575
6600
6650
6675
100msec
mV
1.0
0.8
0.6
0.4
0.2
0.0
2.0
1.5
1.0
0.5
0.0
: FITTING RESULT

FIG. 49

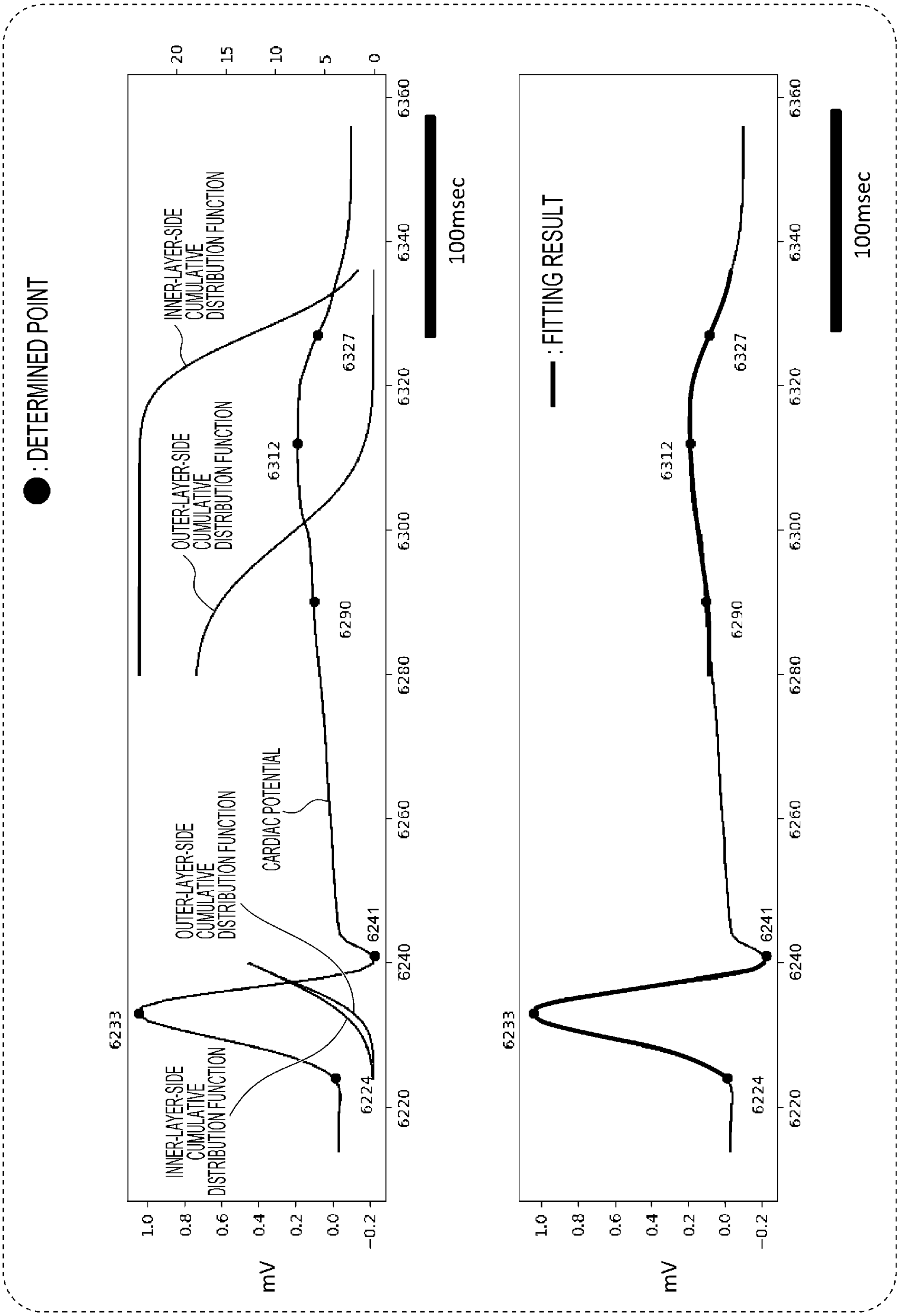
: DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
6224
6233
6241
6290
6312
6327
100msec
mV
: FITTING RESULT

FIG. 50

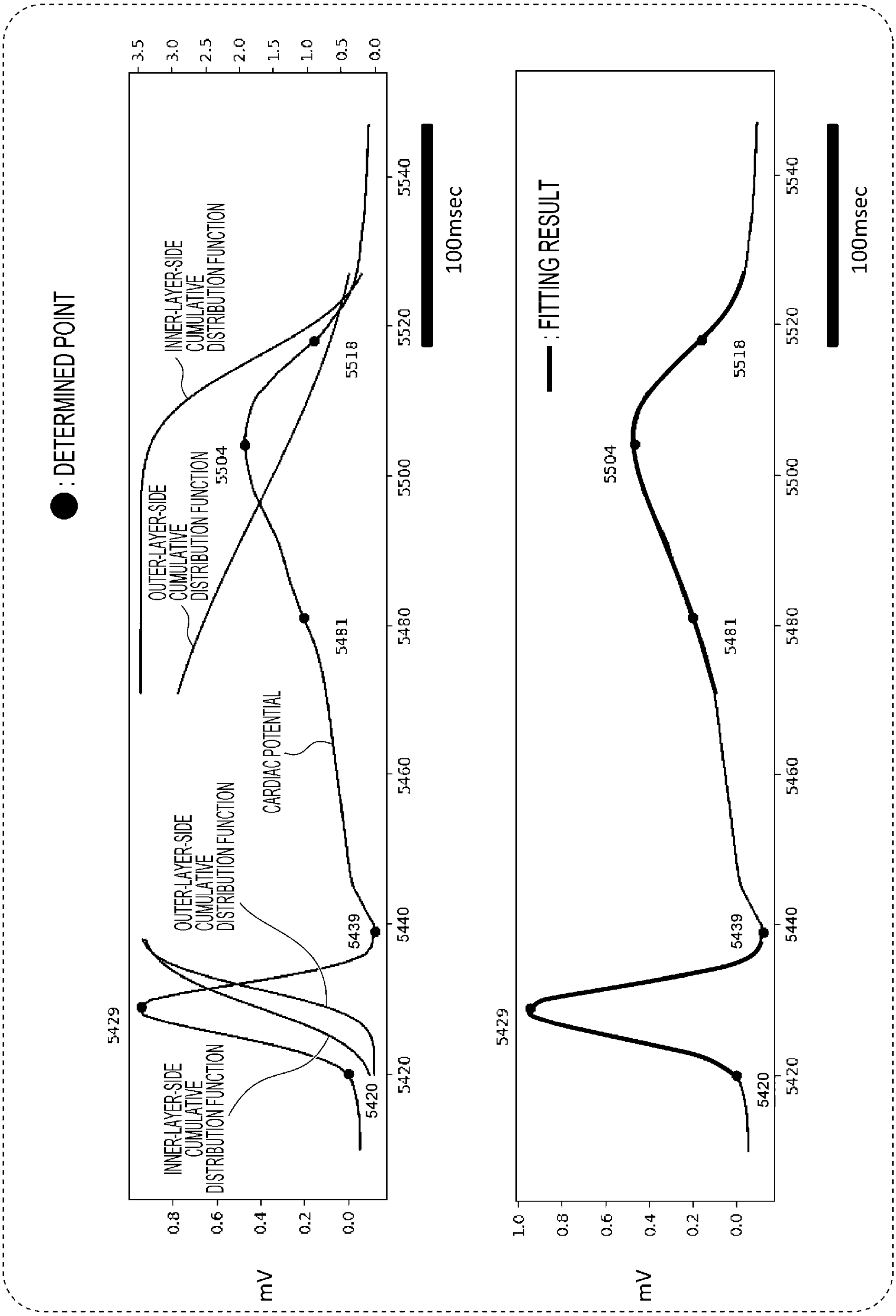
● : DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
5420
5429
5439
5481
5504
5518
mV
100msec
: FITTING RESULT
5420
5429
5439
5481
5504
5518
mV
100msec

FIG. 51

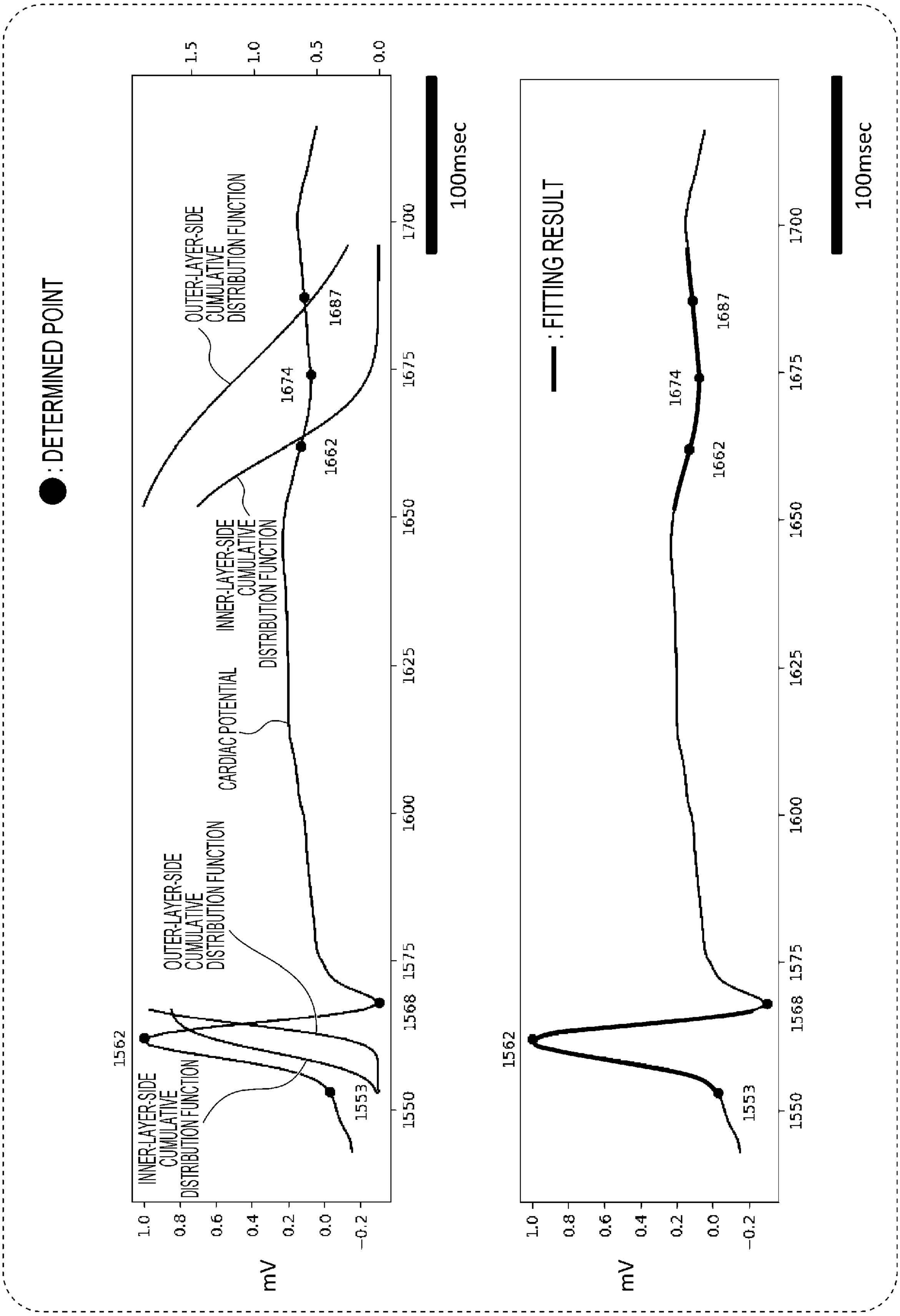
● : DETERMINED POINT
mV
1.0
0.8
0.6
0.4
0.2
0.0
−0.2
1.5
1.0
0.5
0.0
1550
1575
1600
1625
1650
1675
1700
1553
1562
1568
1662
1674
1687
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
100msec
: FITTING RESULT

FIG. 52

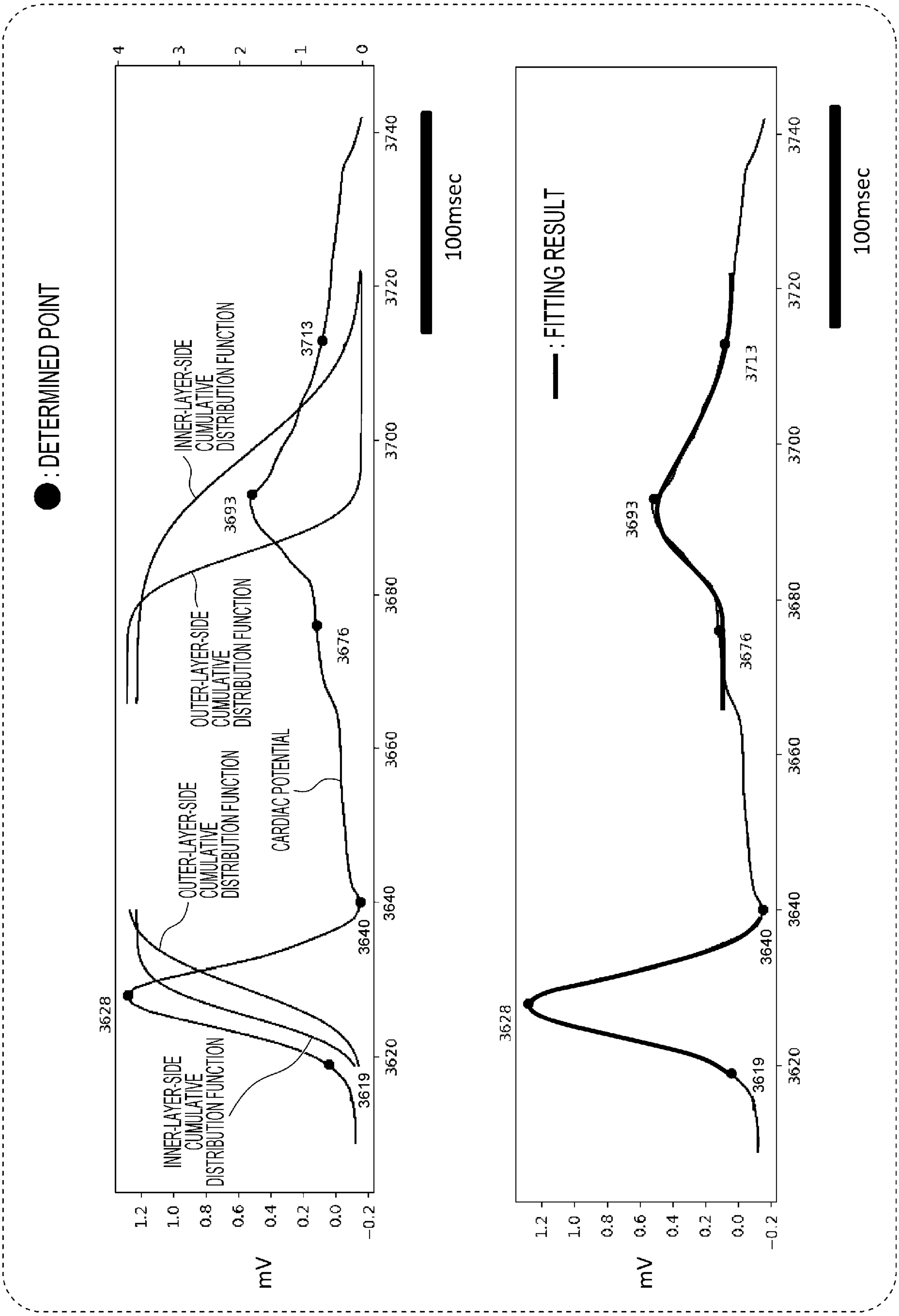
● : DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
3619
3628
3640
3676
3693
3713
mV
100msec
: FITTING RESULT

FIG. 53

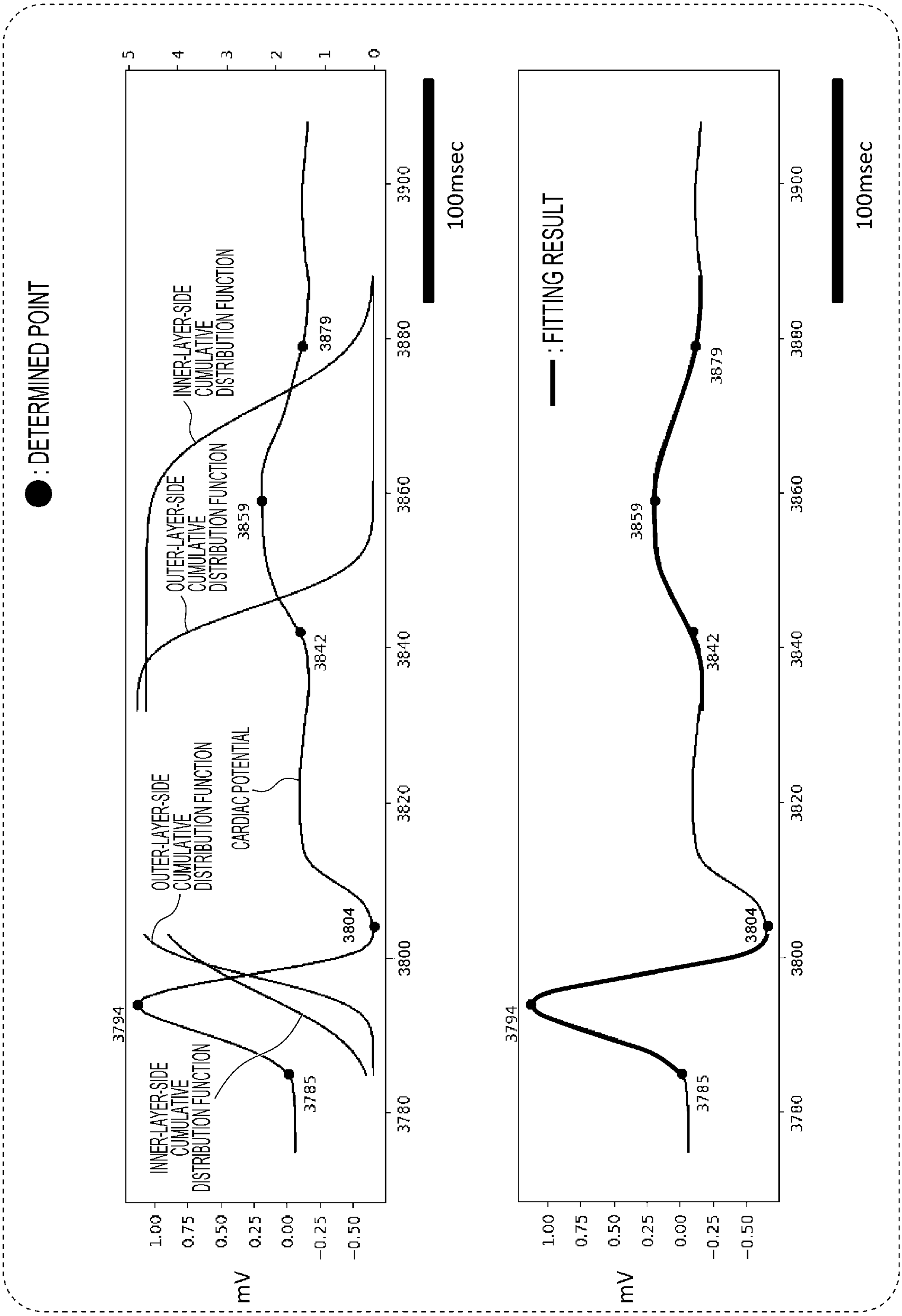
: DETERMINED POINT
mV
1.00
0.75
0.50
0.25
0.00
−0.25
−0.50
0
1
2
3
4
5
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
3785
3794
3804
3842
3859
3879
3780
3800
3820
3840
3860
3880
3900
100msec
: FITTING RESULT
mV
1.00
0.75
0.50
0.25
0.00
−0.25
−0.50
3785
3794
3804
3842
3859
3879
3780
3800
3820
3840
3860
3880
3900
100msec

FIG. 54

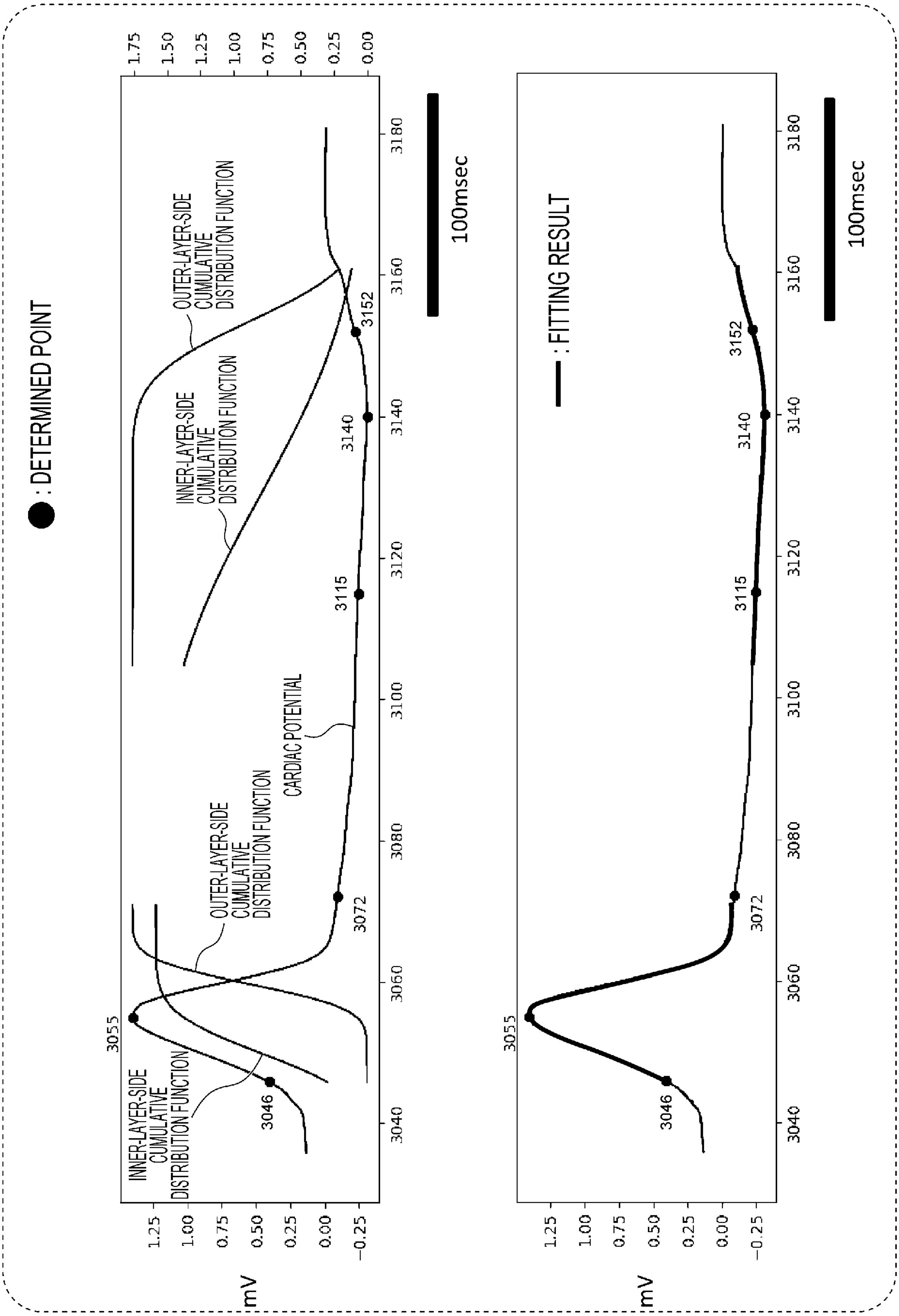
: DETERMINED POINT
INNER-LAYER-SIDE CUMULATIVE DISTRIBUTION FUNCTION
OUTER-LAYER-SIDE CUMULATIVE DISTRIBUTION FUNCTION
CARDIAC POTENTIAL
OUTER-LAYER-SIDE CUMULATIVE DISTRIBUTION FUNCTION
INNER-LAYER-SIDE CUMULATIVE DISTRIBUTION FUNCTION
3046
3055
3072
3115
3140
3152
mV
1.25
1.00
0.75
0.50
0.25
0.00
−0.25
1.75
1.50
3040
3060
3080
3100
3120
3140
3160
3180
100msec
: FITTING RESULT

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HIROSHI TANAKA**. Methodology in Electrocardiographic Inverse Problems. *Medical Electronics and Biotechnology*, 1985, vol. 23 (3), 147-158
- **WATARU SHIMIZU**. The role of M cells in the origin of T waves. *JPN. J. ELECTROCARDIOLOGY*, 2001, vol. 21 (2), 101-108
- **YOSHIFUMI TANAKA** ; **SHINJI NODO** ; **YASUO YAMAZAKI** ; **ARISA NAKASONE**. Program for constructing body surface electrocardiogram from cardiac action potential waveform. *Anaesthesia/intensive care and technology 2011*, 2011, vol. 1, 91-95
- **KHEIRATI ROONIZI EBADOLLAH**. A New Algorithm for Fitting a Gaussian Function Riding on the Polynomial Background. *IEEE SIGNAL PROCESSING LETTERS*, 01 November 2013, vol. 20 (11), ISSN 1070-9908, 1062-1065
- **MAHSA AKHBARI et al.** ECG denoising and fiducial point extraction using an extended Kalman filtering framework with linear and nonlinear phase observations. *PHYSIOLOGICAL MEASUREMENT, INSTITUTE OF PHYSICS PUBLISHING*, 15 January 2016, vol. 37, ISSN 0967-3334, 203-226
- **YOSHIFUMI TANAKA**. Electrocardiogram waveform understood from the constitution, Reading action potentials of myocardium. *Gakken Medical Shujunsha Co., Ltd.*, 2012